# EUROPEAN PATENT SPECIFICATION

(11) **EP 0 816 380 B1**
(45) Date of publication and mention of the grant of the patent: **25.08.2004**
(21) Application number: 96902484.3
(22) Date of filing: 20.02.1996
(51) Int. Cl.: C12N 15/12, C12N 5/10, C07K 14/705, C07K 16/28, G01N 33/577

(54) **OCIF PROTEIN AND METHODS FOR THE PRODUCTION OF THE SAME**
OCIF PROTEIN UND METHODEN ZU DESSEN GEWINNUNG
PROTEINE OCIF ET SES PROCEDES DE PRODUCTION

(30) Priority: 20.02.1995 JP 5497795; 21.07.1995 JP 20750895
(43) Date of publication of application: 07.01.1998
(60) Divisional application: 04076464.9
(73) Proprietor: Sankyo Co., Ltd., Tokyo 103-8426 (JP)
(72) Inventor: GOTO, Masaaki, Shimotsuga-gun, Tochigi 329-05 (JP); TSUDA, Eisuke, Shimotsuga-gun, Tochigi 329-05 (JP); MOCHIZUKI, Shin'ichi, Kawachi-gun, Tochigi 329-04 (JP); YANO, Kazuki, Shimotsuga-gun, Tochigi 329-05 (JP); KOBAYASHI, Fumie, Kawachi-gun, Tochigi 329-11 (JP); SHIMA, Nobuyuki, Kawachi-gun, Tochigi 329-04 (JP); YASUDA, Hisataka, Kawachi-gun, Tochigi 329-04 (JP); NAKAGAWA, Nobuaki, Shimotsuga-gun, Tochigi 329-05 (JP); MORINAGA, Tomonori, Shimotsuga-gun, Tochigi 321-02 (JP); UEDA, Masatsugu, Kawagoe-shi, Saitama 350-11 (JP); HIGASHIO, Kanji, Kawagoe-shi, Saitama 350 (JP)
(74) Representative: Wakerley, Helen Rachael
(86) International application number: PCT/JP1996/000374
(87) International publication number: WO 1996/026217

(56) References cited:
- WO-A-96/28546
- WO-A-97/23614
- DATABASE EMROD EMBL DATABASES Accession Number: M59378, 28 June 1991 GOODWIN R ET AL: "Molecular cloning and expression of the type 1 and type 2 receptors for tumor necrosis factor" XP002077049
- CHAMBERS T ET AL: "Generation of osteoclast-inductive and osteoclastogenic cell lines from the H-2btsA58 transgenic mouse" PROCEEDINGS OF THE NATIONAL ACADEMY OF SCIENCES OF USA., vol. 90, June 1993, pages 5578-5582, XP002077046
- SMITH C A ET AL: "THE TNF RECEPTOR SUPERFAMILY OF CELLULAR AND VIRAL PROTEINS: ACTIVATION, COSTIMULATION, AND DEATH" CELL, vol. 76, 25 March 1994, pages 959-962, XP002029050
- TSUDA E ET AL: "Isolation of a novel cytokine from human fibroblasts that specifically inhibits osteoclastogenesis" BIOCHEMICAL AND BIOPHYSICAL RESEARCH COMMUNICATIONS., vol. 234, 8 May 1997, pages 137-142, XP002076438
- REDDI A: "Bone morphogenesis and modelling: soluble signals sculpt osteosomes in the solid state" CELL, vol. 89, 18 April 1997, pages 159-161, XP002076439
- SIMONET W ET AL: "Osteoprotegerin: a novel secreted protein involved in the regulation of bone density" CELL, vol. 89, 18 April 1997, pages 309-319, XP002077048
- J. BONE. MINER. RES., (1992), Vol. 7, No. 12, FAWTHROP F.W. et al., "The Effect of Transforming Growth Factor Beta on the Plasminogen Activator Activity of Normal Human Osteoblast-Like Cells and a Human Osteosacroma Cell Line MG-63", pages 1363-1371.
- J. CELL PHYSIOL., (1993), Vol. 155, No. 1, FENTON A.J. et al., "Long-Term Culture of Disaggregated Rat Osteoclasts Inhibition of Bone Resorption and Reduction of Osteoclast-Like Cell Number by Calcitonin and PTHrP107-139", pages 1-7.

## Description

### Field of the invention

This invention relates to a novel protein, osteoclastogenesis inhibitory factor (0CIF), and methods for producing the protein.

### Background of the invention

Human bones are always remodelling by the repeated process of resorption and reconstitution. In the process, osteoblasts and osteoclasts are considered to be the cells mainly responsible for bone formation and bone resorption, respectively. A typical example of disease caused by the progression of abnormal bone metabolism is osteoporosis. The disease is known to be provoked by the condition in which bone resorption by osteoclasts exceeds bone formation by osteoblasts, but the mechanism of osteoporosis has not yet been completely elucidated. Osteoporosis causes pain in the bone and makes the bone fragile, leading to fracture. Since osteoporosis increases the number of bedridden old people, it has become a social issue with the increasing number of old people. Therefore, efficacious drugs for the treatment of the disease are expected to be developed. Bone mass reduction caused by the abnormal bone metabolism is thought to be prevented by inhibiting bone resorption, improving bone formation, or improving the balanced metabolism.

Bone formation is expected to be promoted by stimulating growth, differentiation, or activation of osteoblasts. Many cytokines are reported to stimulate growth or differentation of osteoblasts , i.e. fibroblast growth factor (FGF) (Rodan S.B. et al., Endocrinology vol. 121, p1917, 1987), insulin-like growth factor-I (IGF-I) (Hock J. M. et al., Endocrinology vol. 122, p254, 1988), insulin-like growth factor-II (IGF-II) (McCarthy T. et al., Endocrinology vol. 124, p301, 1989), Activin A (Centrella M. et al., Mol, Cell, Biol. vol. 11, p250, 1991), Vasculotropin (Varonique M et al., Biochem. Biophys. Res. Commun. vol. 199, p380, 1994), and bone morphogenetic protein (BMP) (Yamaguchi, A et al., J. Cell Biol. vol. 113, p682, 1991, Sampath T.K. et al., J. Biol Chem. vol.267, p20532, 1992, and Knutsen R. et al., Biochem. Biophys. Res. Commun. vol.194, p1352, 1993.

On the other hand, cytokines which inhibits differentiation and/or maturation of osteoclasts have been paid attention and have been intensively studied. Transforming growth factor-β (Chenu C. et al., Proc. Natl. Acad. Sci. USA, vol. 85, p5683, 1988) and interleukin-4 (Kasano K. et al., Bone-Miner., vol. 21, p179, 1993) are found to inhibit the differentiation of osteoclasts. Calcitonin (Bone-Miner., vol. 17, p347, 1992), Macrophage colony-stimulating factor (Hattersley G. et al. J. Cell. Physiol. vol.137, p199, 1988), interleukin-4 (Watanabe, K. et al., Biochem. Biophys. Res. Commun. vol. 172, p1035, 1990), and interferon-y (Gowen M. et al., J. Bone Miner. Res., vol.1, p469, 1986) are found to inhibit bone resorption by osteoclasts.

These cytokines are expected to be efficacious drugs for improving bone mass reduction by stimulating bone formation and/or by inhibiting bone resorption. The cytokines such as insulin like growth factor-I and bone morphogenetic proteins are now investigated in clinical trials for their effects in treatment of patients with bone diseases. Calcitonin is already used as a drug to care osteoporosis and to diminish pain in osteoporosis.

Examples of drugs now clinically utilized for the treatment of bone diseases and for shortening the treatment period are dihydroxyvitamine D₃, vitamin K₂, calcitonin and its derivatives, hormones such as estradiol, ipriflavon, and calcium preparations. However, these drugs do not provide satisfactory therapeutic effects, and novel drug substances have been expected to be developed. As mentioned, bone metabolism is controlled in the balance between bone resorption and bone formation. Therefore, cytokines which inhibit osteoclast differentiation and/or maturation are expected to be developed as drugs for the treatment of bone diseases such as osteoporosis.

### Disclosure of Invention

This invention was initiated from the view point described above. The purpose of this invention is to offer both a novel factor termed osteoclastogenesis inhibitory factor (OCIF) and a procedure to produce the factor efficiently.

The inventors have intensively searched for osteoclastogenesis inhibitory factors in human embryonic fibloblast IMR-90 (ATCC CCL186) conditioned medium and have found a novel osteoclastogenesis inhibitory factor (0CIF) which inhibits differentiation and/or maturation of osteoclasts.

The inventors have established a method for accumulating the protein to a high concentration by culturing IMR-90 cells using alumina ceramic pieces as the cell adherence matrices.

The inventors have also established an efficient method for isolating the protein, OCIF, from the IMR-90 conditioned medium using the following sequential column chromatography, ion-exchange, heparin affinity, cibacron-blue affinity, and reverse phase.

The inventors, based on the amino acid sequence of the purified natural OCIF, successfully cloned a cDNA encoding this protein. The inventors established also a procedure to produce this protein which inhibits differentiation of osteoclasts. This invention concerns a protein which is produced by human lung fibroblast cells, has molecular weights in SDS-PAGE of 60KD under reducing conditions and 60 KD and 120 KD under non-reducing conditions, has affinity for both cation-exchange resins and heparin, reduces its activity to inhibit differentiation and maturation of osteoclasts if treated for 10 minutes at 70°C or for 30 minutes at 56°C, and lose its activity to inhibit differentiation and maturation of osteoclasts by the treatment for 10 minutes at 90°C. The amino acid sequence of the protein OCIF which is described in the present invention is provided by SEQ In Nos 1, 2 and 3, and is clearly different from any known factors inhibiting formation of osteoclasts.

A method to purify OCIF protein, comprises: (1) culturing human fibroblasts, (2) applying the conditioned medium to a heparin column to obtain the adsorbed fraction, (3) purifying the OCIF protein using a cation-exchange column, (4) purifying the OCIF protein using a heparin affinity column, (5) purifying the OCIF protein using a cibacron blue affinity column, (6) isolating the OCIF protein using reverse-phase column chromatography. Cibacron blue F3GA coupled to a carrier made of synthetic hydrophilic polymers is an example of materials used to prepare Cibacron blue columns. These columns are conventionally called "blue colomns".

The invention includes a method for accumulating the OCIF protein to a high concentration by culturing human fibroblasts using alumina ceramic pieces as the cell-adherence matrices.

Moreover, the inventors determined the amino acid sequences of the peptides derived from OCIF, designed the primers based on these amino acid sequences, and obtained cDNA fragments encoding OCIF from a cDNA library of IMR-90 cells.

### Detailed description of the invention

The OCIF protein of the present invention can be isolated from human fibroblast conditioned medium with high yield. The procedure to isolate OCIF is based on ordinary techniques for purifying proteins from biomaterials, in accordance with the physical and chemical properties of OCIF protein. For example, concentrating procedure includes ordinary biochemical techniques such as ultrafiltration, lyophylization, and dialysis. Purifying procedure includes combinations of several chromatographic techniques for purifying proteins such as ion-exchange column chromatography, affinity column chromatography, gel filtration column chromatography, hydrophobic column chromatography, reverse phase column chromatography, and preparative gel electrophoresis. The human fibroblast used for production of the OCIF protein is preferably IMR-90. A method for producing the IMR-90 conditioned medium is preferably a process comprising, adhering human embryonic fibroblast IMR-90 cells to alumina ceramic pieces in roller-bottles, using DMEM medium supplemented with 5 % new born calf serum for the cell culture, and cultivating the cells in roller-bottles for 7 to 10 days by stand cultivation. CHAPS (3-[(3-cholamid opropyl)-dimethylammonio]-1-propanesulfonate) is prefarably added to the buffer as a detergent in the purification steps of OCIF protein.

OCIF protein of the instant invention can be initially obtained as a heparin binding basic OCIF fraction by applying the culture medium to a heparin column (Heparin-Sepharose CL-6B, Pharmacia), eluting with 10 mM Tris-HCl buffer, pH 7.5, containing 2 M NaCl, and then by applying the OCIF fraction to a Q • anion-exchange column (HiLoad-Q/FF, Pharmacia), and collecting non-adsorbed fraction. OCIF protein can be purified by subjecting the obtained OCIF fraction to purification on a S · cation-exchange column (HiLoad-S/FF, Pharmacia). a heparin column (Heparin-5PW, TOSOH), Cibacrone Blue column (Blue-5PW, TOSOH), and a reverse-phase column (BU-300 C4, Perkin Elmer) and the material is defined by the previously described properties.

The present invention relates to a method of cloning cDNA encoding the OCIF protein based on the amino acid sequence of natural OCIF and a method of obtaining recombinant OCIF protein that inhibits differentiation and/or maturation of osteoclasts. The OCIF protein is purified according to the method described in the present invention and is treated with endopeptidase (for example, lysylendopeptidase). The amino acid sequences of the peptides produced by the digestion are determined and the mixture of oligonucleotides that can encode each internal amino acid sequence was systhesized. The OCIF cBNA fragment. is obtained by PCR (preferably RT-PCR, reverse transcriptase PCR) using the oligonucleotide mixtures described above as primers. The full length OCIF cDNA encoding the OCIF protein is cloned from a cDNA library using the obtained OCIF DNA fragment as a probe. The OCIF cDNA containing the entire coding region is inserted into an expression vector. The recombinant OCIF can be produced by expressing the OCIF cDNA containing the entire coding region in mammalian cells or bacteria.

The novel proteins OCIF2, OCIF3, OCIF4, and OCIF5 are variants of OCIF and have the activity described above.

These OCIF variants are obtained from the cDNA library constructed with IMR-90 poly(A) + RNA by hybridization using the OCIF cDNA fragment as a probe. Each of the OCIF variant cDNAs containing the entire coding region is inserted into an expression vector. Each recombinant OCIF variant can be produced by expressing each of the OCIF variant cDNAs containing the entire coding region in the conventional hosts. Each recombinant OCIF variant can be purified according to the method described in this invention. Each recombinant OCIF variant has an ability to inhibit osteoclastogenesis.

OCIF mutants are substitution mutants comprising replacement of one cysteine residue possibly involved in dimer formation with serine residue, and various deletion mutants of OCTF. Substitutions or deletions are introduced into the OCIF cDNA using polymerase chain reaction (PCR) or by restriction enzyme digestion. Each of these mutated OCIF cDNAs is inserted into a vector containing an appropriate promoter for gene expression. The resultant expression vector for each of the OCIF mutants is transfected into eukaryotic cells such as mammalian cells. Each OCIF mutant can be obtained and purified from the conditioned media of the transfected cells.

The present invention provides OCIF variants, mutants or truncated cDNA, as defined in the accompanying claims.

The present invention provides polyclonal antibodies and a method to quantitatively determine OCIF concentration using these polyclonal antibodies.

As antigens (immunogens), natural OCIF obtained from IMR-90 conditioned medium, recombinant OCIF produced by such hosts as microorganisms and eukaryotes using OCIF cDNA, synthetic peptides designed based on the amino acid sequence of OCIF, or peptides obtained from OCIF by partial digestion can be used. Anti-OCIF polyclonal antibodies are obtained by immunizing appropriate mammals with the antigens in combination with adjuvants for immunization if necessary, purifying from the serum by the ordinary purification methods. The anti-OCIF polyclonal antibodies which are labelled with radioisotopes or enzyme can be used in radio-immunoassay (RIA) system or immunoassay (EIA) system. By using these assay systems, the concentrations of OCIF in biological materials such as blood and ascites and cells-culture medium can be easily determined.

The antibodies of the present invention can be used in radio immunoassay (RIA) or enzyme immunoassay (EIA). By using these assay systems, the concentration of OCIF in biological materials such as blood and ascites can be easily determined.

The present invention provides novel monoclonal antibodies and a method to quantitatively determine OCIF concentration using these monoclonal antibodies.

Anti-OCIF monoclonal antibodies can be produced by the conventional method using OCIF as an antigen. Native OCIF obtained from the culture medium of IMR-90 cells and recombinant OCIF produced by such hosts as microorganisms and eukaryotes using OCIF cDNA can be used as antigens. Alternatively, synthesized peptides designed based on the amino acid sequence of OCIF and peptides obtained from OCIF by partial digestion can be also used as antigens. Immunized lymphocytes obtained by immunization of mammals with the antigen or by an in vitro immunization method were fused with myeloma of mammals to obtain hybridoma. The hybridoma clones secreting antibody which recognizes 0CIF were selected from the hybridomas obtained by the cell fusion. The desired antibodies can be obtained by cell culture of the selected hybridoma clones. In preparation of hybridoma, small animals such as mice or rats are generally used for immunization. To immunize, OCIF is suitably diluted with a saline solution (0.15 M NaCl), and is intravenously or intraperitoneally administered with an adjuvant to animals for 2 -5 times every 2 -20 days. The immunized animal was killed three days after final immunization, the spleen was taken out and the splenocytes were used as immunized B lymphocytes.

Mouse myeloma cell lines for cell fusion with the immunized B lymphocytes include, for example, p3/x63-Ag8, p3-U1, NS-1, MFC-11, SP-2/0, F0, p3x63 Ag8.653, and S194. Rat R-210 cell line may also be used. Human B lymphocytes are immunized by an in vitro immunization method and are fused with human myeloma cell line or EB virus transformed human B lymphocytes which are used as a parent cell line for cell fusion, to produce human type antibody.

Cell fusion of the immunized B lymphocytes and myeloma cell line is carried out principally by the conventional methods. For example, the method of Koehler G. et al. (Nature 256, 495-497, 1975) is generally used, and also an electric pulse method can be applied to cell fusion. The immunized B lymphocytes and transformed B cells are mixed at conventional ratios and a cell culture medium without FBS containing polyethylene glycol is generally used for cell fusion. The B lymphocytes fused with myeloma cell lines are cultured in HAT selection medium containing FBS to select hybridoma.

For screening of hybridoma producing anti-OCIF antibody, EIA, plaque assay, Ouchterlony, or agglutination assay can be principally used. Among them, EIA is simple and easy to operate with sufficient accuracy and is generally used. By EIA using purified OCIF, the desired antibody can be selected easily and accurately. Thus obtained hybridoma can be cultured by the conventional method of cell culture and frozen for stock if necessary. The antibody can be produced by culturing hybridoma using the ordinary cell culture method or by transplanting hybridoma intraperitoneally to animals. The antibody can be purified by the ordinary purification methods such as salt precipitation, gel filtration, and affinity chromatography. The obtained antibody specifically reacts with OCIF and can be used for determination of OCIF concentration and for purification of OCIF. The antibodies of the present invention recognize epitopes of OCIF and have high affinity to 0CIF. Therefore, they can be used for the construction of EIA. By (using) this assay system, the concentration of OCIF in biological materials such as blood and ascites can be easily determined.

OCIF of the present invention may be used as an agent used for treating bone diseases. The present invention provides the use of the proteins of the present invention in the preparation of medicaments as set out in the accompanying claims.

Ratswere subjected to denervation of left forelimb.Test compounds vere administered daily after surgery for 14 days. After 2 weeks treatment, the animals were sacrificed and their forelimbs were dissected. Thereafter bones were tested for mechanical strength by three point bending method. OCIF improved mechanical strength of bone in a dose dependent manner.

The OCIF protein of the invention is useful as a pharmaceutical ingredients for treating or improving decreased bone mass in such as osteoporosis, bone diseases such as rheumatism, osteoarthritis, and abnormal bone metabolism in multiple myeloma. The OCIF protein is also useful as an antigen to establish immunological diagnosis of the diseases. Pharmaceutical preparations containing the OCIF protein as an active ingredients are formulated and can be orally or parenterally administered. The preparation contains the OCIF protein of the present invention as an efficacious ingredient and is safely administered to human and animals. Examples of the pharmaceutical preparations include compositions for injection or intravenous drip, suppositories, nasal preparations, sublingual preparations, and tapes for percutaneous absorption. The pharmaceutical preparation for injection can be prepared by mixing the pharmacologically efficacious amount of OCIF protein and pharmaceutically acceptable carriers. The carriers are vehicles and/or activators, e.g. amino acids, saccharides, cellulose derivatives, and other organic and inorganic compounds which are generally added to active ingredients. When the OCIF protein is mixed with the vehicles and/or activators to prepare injections, pH adjuster, buffer, stabilizer, solubilizing agent, etc. can be added, if necessary.

### Brief description of the figures

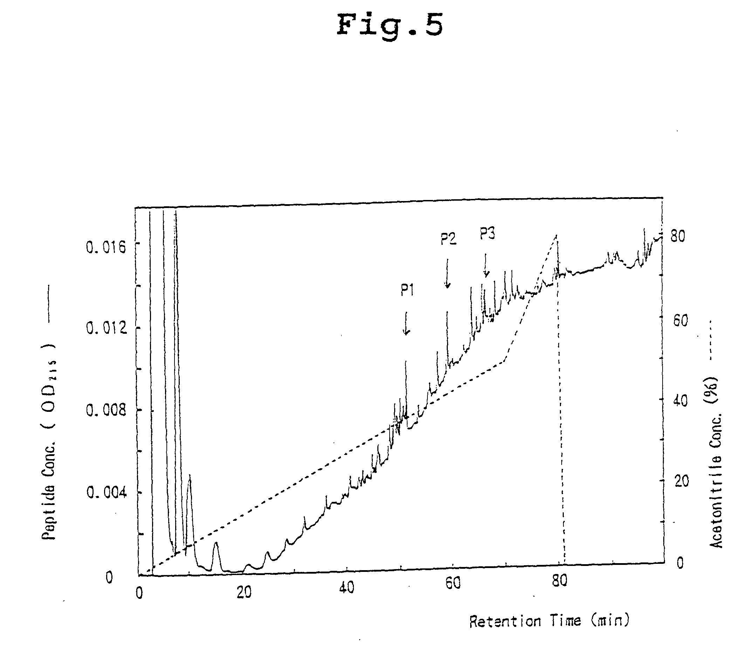

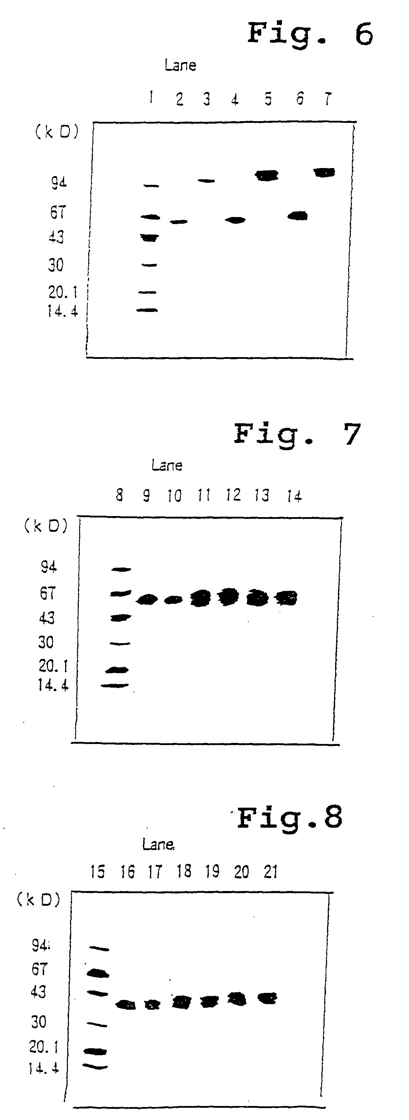

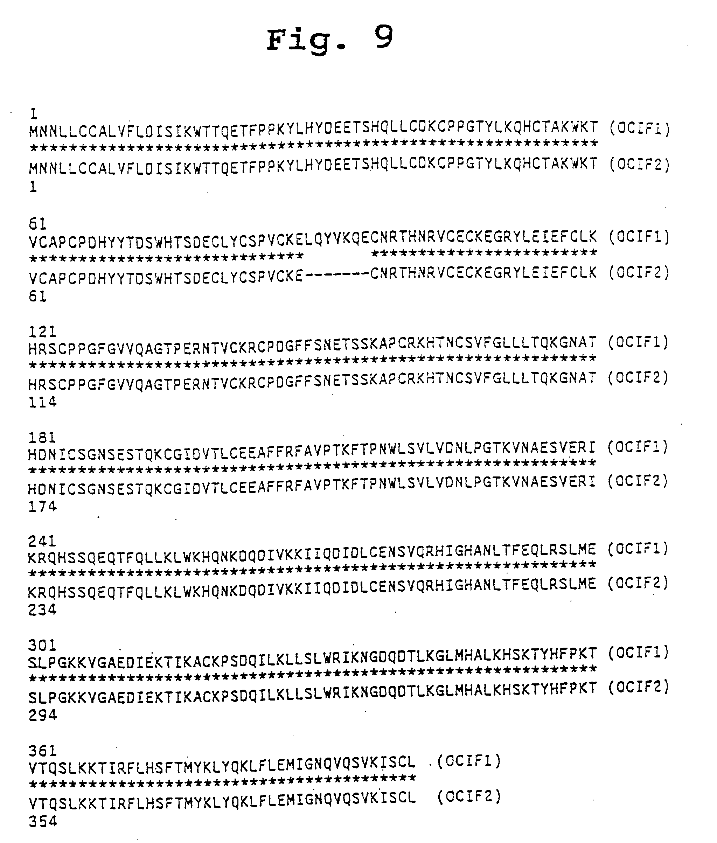

Figure 1 shows the elution pattern of crude OCIF protein (Hiload-Q/FF pass-through fraction ; sample 3) from a Hiload-S/HP column.
Figure 2 shows the elution pattern of crude OCIF protein (heparin-5PW fraction ; sample 5) from a blue-5PW column.
Figure 3 shows the elution pattern of OCIF protein (blue-5PW fraction 49 to 50) from a reverse-phase column.
Figure 4 shows the SDS-PAGE of isolated OCIF proteins under reducing conditions or non-reducing conditions.
   Description of the lanes,
   lane 1,4 ; molecular weight marker proteins
   lane 2,5 ; OCIF protein of peak 6 in figure 3
   lane 3,6 ; OCIF protein of peak 7 in figure 3
Figure 5 shows the elution pattern of peptides obtained by the digestion of pyridyl ethylated OCIF protein digested with lysylendopeptidase, on a reverse-phase column.
Figure 6 shows the SDS-PAGE of isolated natural(n) OCIF protein and recombinant(r) OCIF proteins under non-reducing conditions. r0CIF(E) and rOCIF(C) were produced in 293/EBNA cells and in CHO cells, respectively.
   Description of the lanes,
   lane 1 ; molecular weight marker proteins
   lane 2 ; a monomer type nOCIF protein
   lane 3 ; a dimer type nOCIF protein
   lane 4 ; a monomer type rOCIF(E) protein
   lane 5 ; a dimer type rOCIF(E) protein
   lane 6 ; a monomer type rOCIF(C) protein
   lane 7 ; a dimer type rOCIF(C) protein
Figure 7 shows the SDS-PAGE of isolated natural(n) OCIF proteins and recombinant (r) OCIF proteins under reducing conditions. rOCIF(E) and rOCIF(C) were produced in 293/EBNA cells and in CHO cells, respectively.
   Description of the lanes,
   lane 8 ; molecular weight marker proteins
   lane 9 ; a monomer type nOCIF protein
   lane 10 ; a dimer type nOCIF protein
   lane 11 ; a monomer type rOCIF(E) protein
   lane 12 ; a dimer type r0CIF(E) protein
   lane 13 ; a monomer type rOCIF(C) protein
   lane 14 ; a dimer type r0CIF(C) protein
Figure 8 shows the SDS-PAGE of isolated natural(n) OCIF proteins and recombinant(r) OCIF proteins from which N-linked sugar chains were removed under reducing conditions. rOCIF(E) and rOCIF(C) are rOCIF protein produced in 293/EBNA cells and in CHO cells, respectively.
   Description of the lanes,
   lane 15 ; molecular weight marker proteins
   lane 16 ; a monomer type nOCIF protein
   lane 17 a dimer type n0CIF protein
   lane 18 ; a monomer type r0CIF(E protein
   lane 19 ; a dimer type rOCIF(E) protein
   lane 20 ; a monomer type r0CIF(C) protein
   lane 21 ; a dimer type r0CIF(C) protein
Figure 9 shows comparison of amino acid sequences between OCIF and OCIF2.

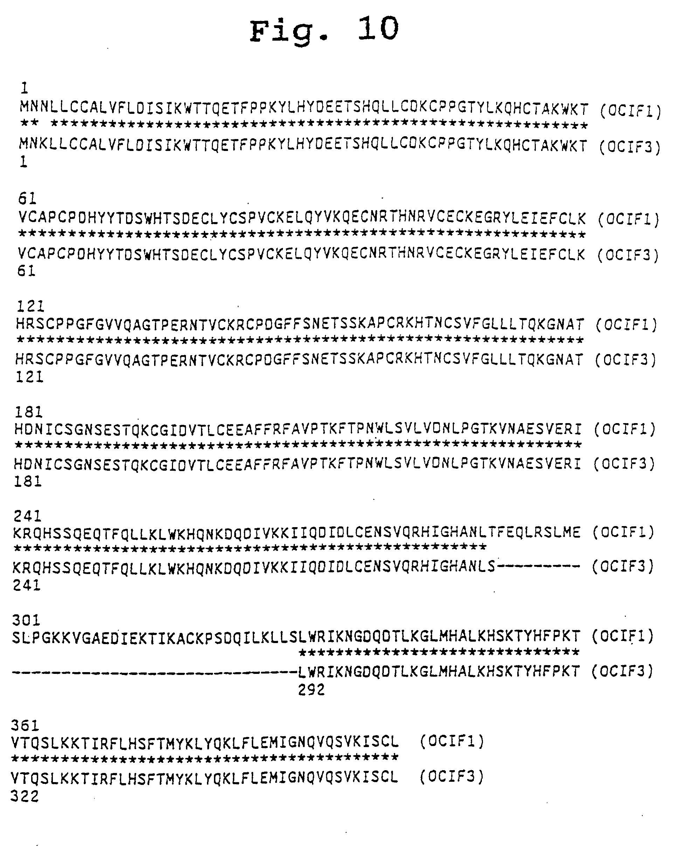

Figure 10 shows comparison of amino acid sequences between OCIF and OCIF3.

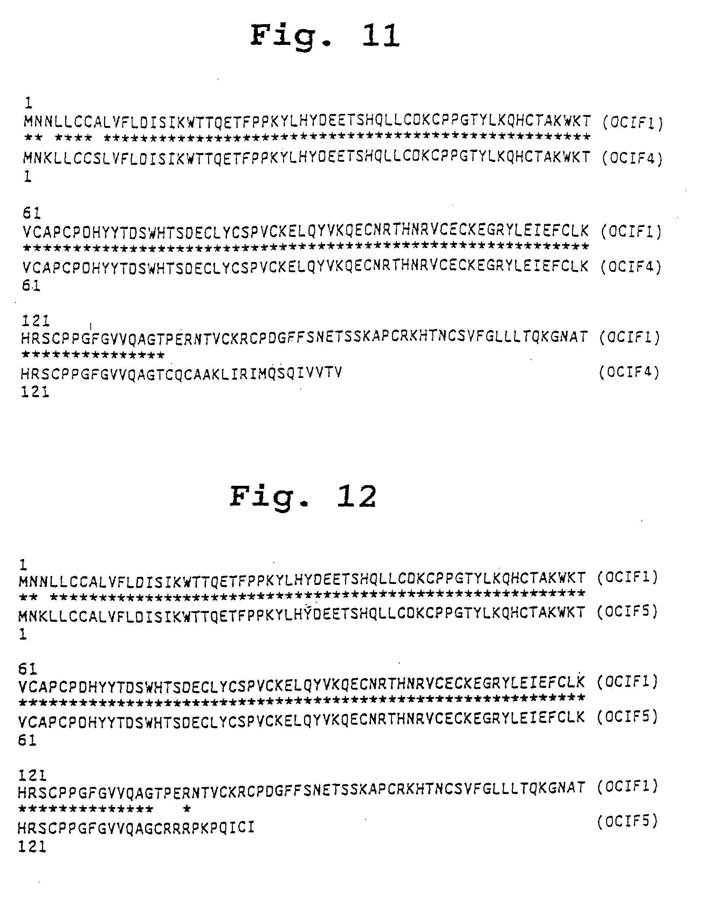

Figure 11 shows comparison of amino acid sequences between OCIF and OCIF4.
Figure 12 shows comparison of amino acid sequences between OCIF and OCIF5.

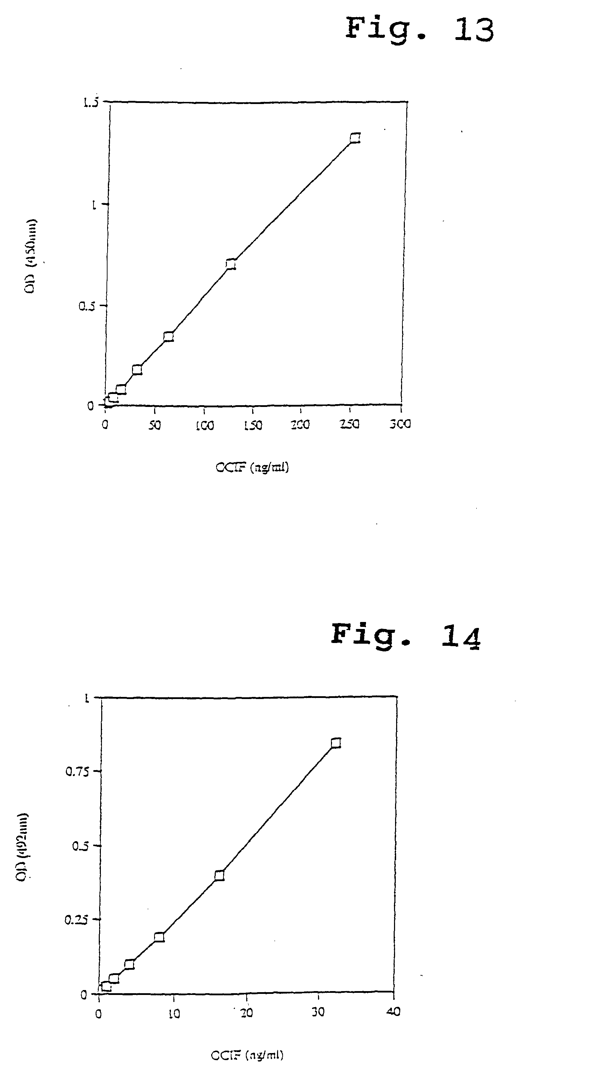

Figure 13 shows standard curve for determination of OCIF protein concentration by an EIA employing anti-OCIF polyclonal antibodies.
Figure 14 shows standard curve for determination of OCIF protein concentration by an EIA employing anti-OCIF monoclonal antibodies.

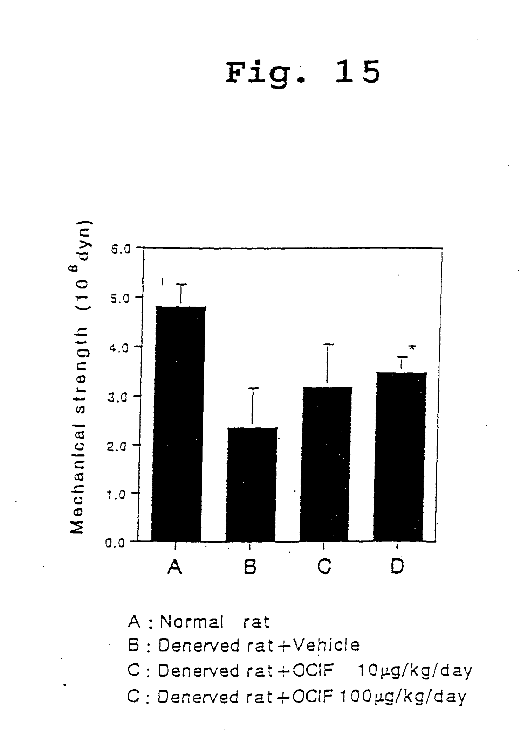

Figure 15 shows the effect of rOCIF protein on osteoporosis.

### Best Mode for Carrying Out the Invention

The present invention will be further explained by the following examples, however, the scope of the invention is not restricted to the examples.

### EXAMPLE 1

### Preparation of a conditioned medium of human fibroblast IMR-90

Human fetal lung fibroblast IMR-90 (ATCC-CCL186) cells were cultured on alumina ceramic pieces (80 g) (alumina: 99.5%, manufactured by Toshiba Ceramic K.K.) in DMEM medium (manufactured by Gibco BRL Co.) supplemented with 5% CS and 10mM HEPES buffer (500 ml/roller bottle) at 37°C under the presence of 5% CO₂ for 7 to 10 days using 60 roller bottles (490 cm², 110 x 171mm, manufactured by Coning Co.)in static culture. The conditioned medium was harvested, and a fresh medium was added to the roller bottles. About 30L of IMR-90 conditioned medium per batch culture was obtained. The conditioned medium was designated as sample 1.

### EXAMPLE 2

### Assay method for osteoclast development inhibitory activity

Osteoclast development inhibitory activity was assayed by measuring tartrate-resistant acid phosphatase(TRAP) activity according to the methods of M. Kumegawa et. al (Protein · Nucleic · Acid · Enzyme, vol. 34 p999, 1989) and N. Takahashi et.al (Endocrynology, vol. 122, p1373, 1988 ) with modifications. Briefly, bone marrow cells obtained from 17 day-old mouse were suspended in α-MEM (manufactured by GIBCO BRL Co.) containing 10% FBS, 2x10⁻⁸M of activated vitamin D₃, and each test sample, and were inoculated to each well of 96-well plate at a cell density of 3x10⁵ cells/0.2 ml/well. The plates were incubated for 7 days at 37°C in humidified 5%CO₂. Cultures were further continued by replacing 0.16 ml of old medium with the same volume of fresh medium on day 3 and day 5 after starting cultivation. On day 7, after washing the plates with phosphate buffered saline, cells were fixed with ethanol/acetone (1:1) for 1 min. at room temperature, and then osteoclast development was tested by determining for phosphatase activity using a kit (Acid Phosphatase, Leucocyte, Catalog No. 387-A, manufactured by Sigma Co.). The decrease of TRAP positive cells was taken as an indication of OCIF activity.

### EXAMPLE 3

### Purification of OCIF

### i) Heparin Sepharose CL-6B column chromatography

The 90L of IMR-90 conditioned medium (sample 1) was filtrated with 0.22 *µ* membrane filter (hydrophilic Milidisk, 2000 cm², Milipore Co.), and was divided into three portions. Each portion (30 1) was applied to a heparin Sepharose CL-6B column (5 x 4.1 cm, Pharmacia Co.) equilibrated with 10mM Tris-HCl containing 0.3M NaCl, pH 7.5. After washing the column with 10mM Tris-HCl, pH 7.5 at a flow rate of 500 ml/hr., heparin Sepharose CL-6B adsorbent protein fraction was eluted with 10mM Tris-HCl, pH 7.5, containing 2M NaCl. The fraction was designated as sample 2.

### ii) HiLoad-Q/FF column chromatography

The heparin Sepharose-adsorbent fraction (sample 2) was dialyzed against 10mM Tris-HCl, pH 7.5, supplemented with CHAPS to a final concentration of 0. 1%, incubated at 4 °C overnight, and divided into two portions. Each portion was then applied to an anion-exchange column (HiLoad-Q/FF, 2.6 x 10 cm, Pharmacia Co.) which was equilibrated with 50mM Tris-HCl, 0.1% CHAPS, pH 7.5 to obtain a non-adsorbent fraction (1000 ml). The fraction was designated as sample 3.

### iii) HiLoad-S/HP column chromatography

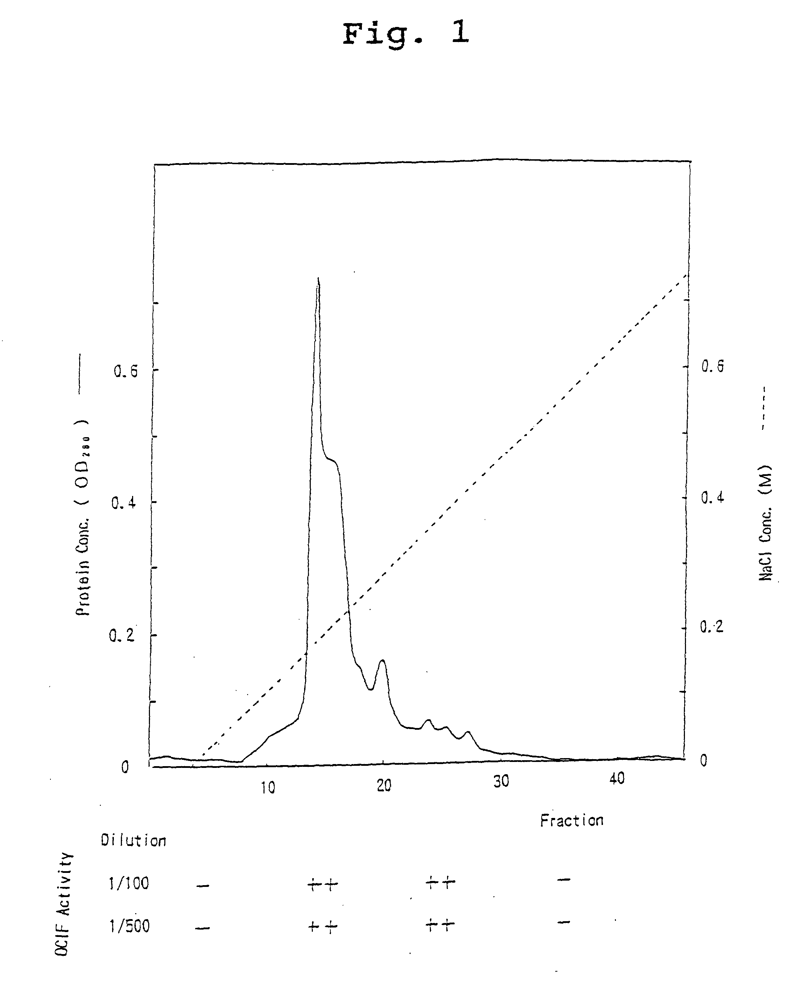

The HiLoad-Q non-adsorbent fraction (sample 3) was applied to a cation-exchange column (HiLoad-S/HP, 2.6 x 10 cm, Pharmacia Co.) which was equilibrated with 50 mM Tris-HCl, 0. 1% CHAPS, pH 7.5. After washing the column with 50 mM Tris-HCl, 0. 1% CHAPS, pH 7.5, the adsorbed protein was eluted with linear gradient from 0 to 1 M NaCl at a flow rate of 8 ml/min for 100 min. and fractions (12 ml) were collected. Each ten fractions from number 1 to 40 was pooled to form one portion. Each 100 µl of the four portions was tested for OCIF activity. OCIF activity was observed in fractions from 11 to 30 (as shown in Figure 1). The fractions from 21 to 30 which had higher specific activity were collected and was designated as sample 4.

### iv) Heparin-5PW affinity column chromatography

One hundred and twenty ml of HiLoad-S fraction from 21 to 30 (sample 4) was diluted with 240 ml of 50 mM Tris-HCl, 0. 1% CHAPS, pH 7.5, and applied to heparin-5PW affinity column (0.8 x 7.5 cm, Tosoh Co.) which was equilibrated with 50mM Tris-HCl, 0. 1% CHAPS, pH 7.5. After washing the column with 50mM Tris-HCl, 0.1% CHAPS, pH 7.5, the adsorbed protein was eluted with linear gradient from 0 to 2M NaCl at a flow rate of 0.5ml/min for 60 min. and fractions (0.5 ml) were collected. Fifty µl was removed from each fraction to test for 0CIF activity. The active fractions, eluted with 0.7 to 1.3M NaCl was pooled and was designated as sample 5.

### v) Blue 5PW affinity column chromatography

Ten ml of sample 5 was diluted with 190 ml of 50mM Tris :dCl, 0. 1% CHAPS, pH 7. 5 and applied to a blue-5PW affinity column, (0. 5x5 cm, Tosoh Co.) which was equilibrated with 50mM Tris-HCl, 0. 1% CHAPS, pH 7. 5. After washing the column with 50mM Tris-HCl, 0.1% CHAPS, pH7.5, the adsorbed protein was eluted with a 30 ml linear gradient from 0 to 2M NaCl at a flow rate of 0.5 ml/min., and fractions (0.5 ml) were collected. Using 25 µl of each fraction, OCIF activity was evaluated. The fractions number 49 to 70, eluted with 1.0-1.6M NaCl had OCIF activity.

### vi) Reverse phase column chromatography

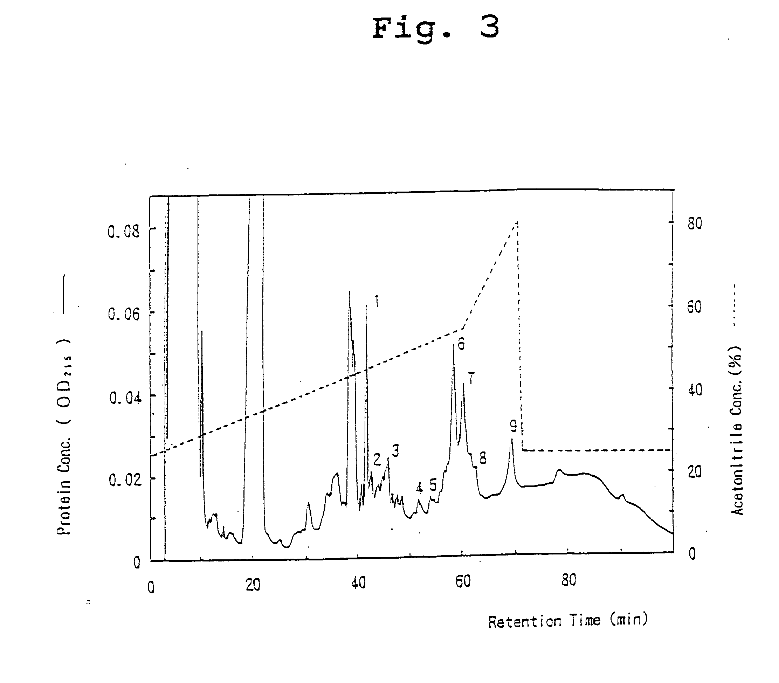

The blue 5PW fraction obtained by collecting fractions from 49 to 50 was acidified with 10 *µ*l of 25% TFA and applied to a reverse phase C4 column (BU-300, 2.1x220mm, manufactured by Perkin-Elmer) which was equilibrated with 0.1% of TFA and 25% of acetonitrile. The adsorbed protein was eluted with linear gradient from 25 to 55% acetonitrile at a flow rate of 0. 2 ml/min. for 60 min., and each protein peak was collected (Fig. 3). One hundred *µ*l of each peak fraction was tested for OCIF activity, and peak 6 and the peak 7 had OCIF activity. The result was shown in Table 1.

**Table 1**

| OCIF activity eluted from reverse phase C4 column | | | | |
|---|---|---|---|---|
| Sample | Dilution | | | |
| | 1/40 | 1/120 | 1/360 | 1/1080 |
| Peak 6 | ++ | ++ | + | - |
| Peak 7 | ++ | + | - | - |
| [ ++ means OCIF activity inhibiting osteoclast development more than 80%, + means OCIF activity inhibiting osteoclast development between 30% and 80%, and - means no OCIF activity.] | | | | |

### EXAMPLE 4

### Molecular weight of OCIF protein

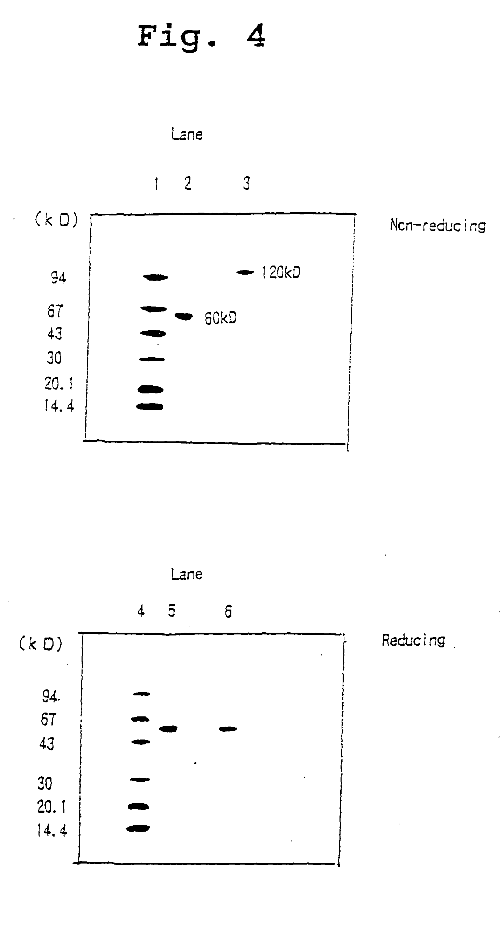

The two protein peaks (6 and 7) with OCIF activity were subjected to SDS-polyacrylamide gel electrophoresis under reducing and non-reducing conditions. Briefly, 20 *µ*l of each peak fraction was concentrated under vacuum and dissolved in 1.5 *µ*l of 10mM Tris-HCl, pH 8, 1mM EDTA, 2.5% SDS, 0.01% bromophenol blue, and incubated at 37°C overnight under non-reducing conditions or under reducing conditions (with 5% of 2-mercaptoethanol). Each 1.0 *µ*l of sample was then analyzed by SDS-polyacrylamide gel electrophoresis with a gradient gel of 10-15% acrylamide (Pharmacia Co.) and an electrophoresis-device (Fast System, Pharmacia Co.). The following molecular weight marker proteins were used to calculate molecular weight : phosphorylase b (94 kD), bovine serum albumin (67 kD), ovalbumin (43 kD), carbonic anhydrase (30 kD), trypsin inhibitor (20.0 kD), and lactalbumin (14.4 kD). After electrophoresis, protein bands were visualized by silver stain using Phast Silver Stain Kit. The results were shown in Fig. 4.

A protein band with an apparent 60 KD was detected in the peak 6 protein under both reducing and non-reducing conditions. A protein band with an apparent 60 KD was detected under reducing conditions and a protein band with an apparent 120 KD was detected under non-reducing conditions in the peak 7 protein. Therefore, the protein of peak 7 was considered to be a homodimer of the protein of peak 6.

### EXAMPLE 5

### Thermostability of OCIF

Twenty µl of sample from the blue-5PW fractions 51 and 52 was diluted to 30 µl with 10 mM phosphate buffered saline, pH 7.2, and incubated for 10 min. at 70°C or 90 °C, or for 30 min. at 56°C. The heat-treated samples were tested for OCIF activity. The results were shown in Table 2.

**Table 2**

| Thermostability of 0CIF | | | |
|---|---|---|---|
| Sample | Dilution | | |
| | 1/300 | 1/900 | 1/2700 |
| untreated | ++ | + | - |
| 70°C, 10 min | + | - | - |
| 56°C, 30 min | + | - | - |
| 90°C, 10 min | - | - | - |
| [ ++ means OCIF activity inhibiting osteoclast development more than 80%, +means OCIF activity inhibiting osteoclast development between 30% and 80%, and - means no OCIF activity.] | | | |

### EXAMPLE 6

### Internal amino acid sequence of OCIF protein

Each 2 fractions (1 ml) from No. 51-70 of blue-5PW fraction was acidified with 10 µl of 25% TFA, and was applied to a reverse phase C4 column (BU-300, 2. 1x220mm, manufactured by Perkin-Elmer Co.) equilibrated with 25% of acetonitrile containing 0.1 % TFA. The adsorbed protein was eluted with a 12 ml linear gradient of 25 to 55% acetonitrile at a flow rate of 0. 2 ml/min, and the protein fractions corresponding to peak 6 and peak 7 were collected, respectively. The protein of each peak was applied to a protein sequencer (PROCISE 494, Perkin-Elmer Co.). However, the N-terminal sequence of the protein of each peak could not be analyzed. Therefore, N-terminal of the protein of each peak was considered to be blocked. So, internal amino acid sequences of these proteins were analyzed.

The protein of peak 6 or peak 7 purified by C4-HPLC was concentrated by centrifugation and pyridilethylated under reducing conditions. Briefly, 50 µl 1 of 0. 5 M Tris-HCl, pH 8. 5, containing 100 µg of dithiothreitol, 10mM EDTA, 7 M guanidine-HCl, and 1% CHAPS was added to each samples, and the mixture was incubated overnight in the dark at a room temperature. Each the mixture was acidified with 25% TFA (a final concentration 0.1%) and was applied to a reversed phase C4 column (BU-300, 2.1x30mm, Perkin-Elmer Co.) equilibrated with 20 % acetonitrile containing 0.1 % TFA. The pyridil-ethylated OCIF protein was eluted with a 9 ml linear gradient from 20 to 50% acetonitrile at a flow rate of 0.3 ml/min, and each protein peak was collected. The pyridil-ethyrated OCIF protein was concentrated under vacuum , and dissolved in 25 µl of 0.1 M Tris-HCl, pH 9, containing 8 M Urea, and 0. 1 % Tween 80. Seventy three µl of 0. 1 M Tris-HCl, pH 9, and 0. 02 µg of lysyl endopeptidase (Wako Pure Chemical, Japan) were added to the tube, and incubated at 37°C for 15 hours. Each digest was acidified with 1 µl of 25% TFA and was applied to a reverse phase C8 column (RP-300, 2.1x220mm, Perkin-Elmer Co.) equilibrated with 0.1% TFA.
The peptide fragments were eluted from the column with linear gradient from 0 to 50 % acetonitrile at a flow rate of 0.2 ml/min for 70 min., and each peptide peak was collected. Each peptide fragment (P1 - P3) was applied to the protein sequencer. The sequences of the peptides were shown in Sequence Numbers 1 - 3, respectively.

### EXAMPLE 7

### Determination of nucleotide sequence of the OCIF cDNA

### i) Isolation of poly(A) + RNA from IMR-90 cells

About 10 ug of poly(A) + RNA was isolated from 1x10⁸ cells of IMR-90 by using Fast Track mRNA isolation kit (Invitrogen) according to the manufacturer's instructions.

### ii) Preparation of mixed primers

The following two mixed primers were synthesized based on the amino acid sequences of two peptides (peptide P2 and peptide P3, sequence numbers 2 and 3, respectively). All the oligonucleotides in the mixed primers No. 2F can code for the amino acid sequence from the sixth residue, glutamine (GIn) to the twelfth residue, leucine (Leu), in peptide P2. All the oligonucleotides in the mixed primers No. 3R can code for the amino acid sequence from the sixth residue, histidine (His), to the twelfth residue, lysine (Lys), in peptide P3. The sequences of the mixed primers No. 2F and No. 3R were shown in Table 3.

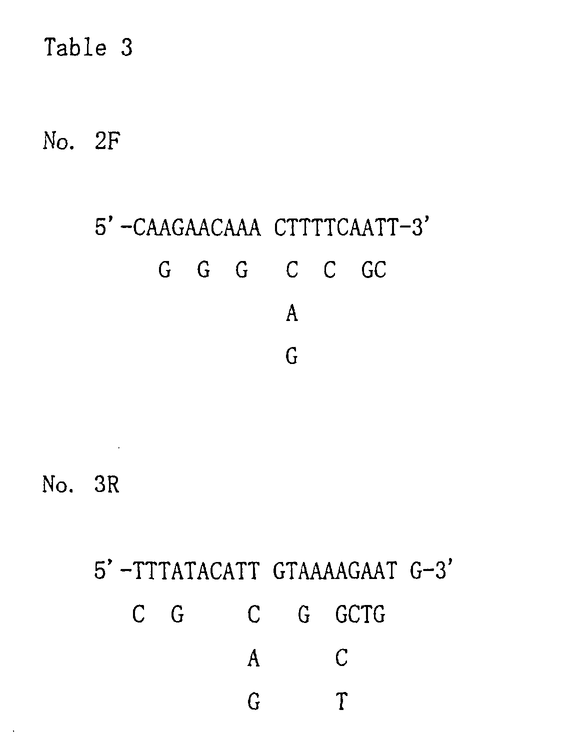

### iii) Amplification of OCIF cDNA fragment by PCR (Polymerase chain reaction)

First strand cDNA was generated using Superscript II cDNA synthesis kit (Gibco BRL) and 1 ug of poly(A) + RNA obtained in the example 7-i) according to the manufacturer's instructions. The DNA fragment encoding OCIF was obtained by PCR using the cDNA template and the primers shown in EXAMPLE 7-ii).
PCR was performed with the conditions as follows;

| | |
|---|---|
| 10X Ex Taq Buffer (Takara Shuzo) | 5 ul |
| 2.5 mM solution of dNTPs | 4 ul |
| cDNA solution | 1 ul |
| Ex Taq (Takara Shuzo) | 0.25 ul |
| sterile distilled water | 29.75 ul |
| 40 uM solution of primers No. 2F | 5 ul |
| 40 uM solution of primers No. 3R | 5 ul |

The components of the reaction were mixed in a microcentrifuge tube. An initial denaturation step at 95 °C for 3 min was followed by 30 cycles of denaturation at 95°C for 30 sec annealing at 50 °C for 30 sec and extention at 70 °C for 2min. After the amplification, final extention step was performed at 70 °C for 5min. The size of PCR products were determined on a 1.5 % agarose gel electrophoresis. About 400 bp OCIF DNA fragment was obtained.

### EXAMPLE 8

### Cloning of the OCIF cDNA fragment amplified by PCR and determination of its DNA sequence

The OCIF cDNA fragment amplified by PCR In EXAMPLE 7-iii) was inserted in the plasmid, pBluescript II SK⁻ using DNA ligation kit ver. 2 (Takara Shuzo) according to the method by Marchuk, D. et al. (Nucleic Acids Res., vol 19, p1154, 1991). E. coli. DH5 α (Gibco BRL) was transformed with ligation mixture. The transformants were grown and a plasmid containing the OCIF cDNA (about 400 bp) was purified using the commonly used method. This plasmid was called pBSOCIF. The sequence of OCIF cDNA in pBSOCIF was determined using Taq Dye Deoxy Terminater Cycle Sequencing kit (Perkin Elmer). The size of the OCIF cDNA is 397 bp. The OCIF cDNA encodes an amino acid sequence containing 132 residues. The amino acid sequences of the internal peptides (peptide P2 and peptide P3, sequence number 2 and 3, respectively) that were used to design the primers were found at N- or C- terninal side in the amino acid sequence of the 132 amino acid polypeptide predicted by the 397 bp OCIF cDNA. In addition, the amino acid sequence of the internal peptide P1 (sequence number 1) was also found in the predicted amino acid sequence of the polypeptide. These data show that the 397 bp OCIF cDNA is a portion of the full length OCIF cDNA.

### EXAMPLE 9

### Preparation of the DNA probe

The 397 bp OCIF cDNA was prepared according to the conditions described in EXAMPLE 7-iii). The OCIF cDNA was subjected to a preparative agarose gel electrophoresis. The OCIF cDNA was purified from the gel using QIAEX gel extraction kit (QIAGEN), labeled with [α³²P]dCTP using Megaprime DNA labeling system (Amersham) and used to select a phage containing the full length OCIF cDNA.

### EXAMPLE 10

### Preparation of the cDNA library

cDNA was generated using Great Lengths cDNA synthesis kit (Clontech), oligo (dT) primer, [α³²]dCTP and 2.5 ug of poly(A) + RNA obtained in the example 7-i) according to the manufacturer's instructions. EcoRI-SalI-NotI adaptor was ligated to the cDNA. The cDNA was separated from the free adaptor and unincorporated free [α³²P]dCTP. The purified cDNA was precipitated with ethanol and dissolved in 10 ul of TE buffer (10 mMTris-HCl (pH8.0), 1 mM EDTA). The cDNA with the adaptor was inserted in λ ZAP EXPRESS vector (Stratagene) at EcoRI site. The recombinant λ ZAP EXPRESS phage DNA containing the cDNA was in vitro packaged using Gigapack gold II packaging extract (Stratagene) and recombinant λ ZAP EXPRESS phage library was prepared.

### EXAMPLE 11

### Screening of recombinant phage

Recombinant phages obtained in EXAMPLE 10 were infected to E. Coli, XL1-Blue MRF' (Stratagene) at 37 °C for 15 min.. The infected E.coli cells were added to NZY medium containing 0.7 % agar at 50°C and plated on the NZY agar plates. After the plates were incubated at 37°C overnight, Hybond N (Amersham) were placed on the surface of plates containing plaques. The membranes were denatured in the alkali solution, neutralized, and washed in 2xSSC according to the standard protocol. The phage DNA was immobilized on the membranes using UV Crosslink (Stratagene). The membranes were incubated in the hybridization buffer (Amersham) containing 100 *µ*g/ml salmon sperm DNA at 65°C for 4 hours and then incubated at 65 °C overnight in the same buffer containing 2x10⁵ cpm/ml denatured OCIF DNA probe. The membranes were washed twice with 2xSSC and twice with a solution containing 0.1xSSC and 0. 1% SDS at 65 °C for 10 min each time. The positive clones were purified by repeating the screening twice. The purified λ ZAP EXPRESS phage clone containing about 1.6 kb DNA insert was used in the experiments described below. This phage was called λ OCIF. The purified λ OCIF and the infected into E. Coli XL1-Blue MRF' (Stratagene) according to a protocol of λ ZAP EXPRESS cloning kit (Stratagene). The culture broth of infected XL1-Blue MRF' was prepared.
Purified 10CIF and ExAssist helper phage (Stratagene) were co-infected into E. coli strain XL-1 blue MRF' according to the protocol supplied with the kit. The culture broth of the co-infected XL-1 blue MRF' was added to a culture of E. coli strain XLOR (Stratagene) to transform them. Thus we obtained a Kanamycin-resistant transformant harboring a plasmid designated pBKOCIF which is a pBKCMV (Stratagene) vector containing the 1.6 kb insert fragment.
The transformant including the plasmid containing about 1.6 kb OCIF cDNA was obtained by picking up the kanamycin-resistant colonies. The plasmid was called pBKOCIF. The transformant has been deposited to National Institute of Bioscience and Human-Technology (NIBH), Agency of Industrial Science and Tecnology as "FERM BP-5267" as pBK/01F10. A national deposit (Accession number, FERM P-14998) was transfered to the international deposit, on October 25, 1995 according to the Budapest treaty. The transformant pBK/01F10 was grown and the plasmid pBK0CIF was purified according to the standard protocol.

### EXAMPLE 12

### Determination of the nucleotide sequence of OCIF cDNA containing the full coding region.

The nucleotide sequence of OCIF cDNA obtained in EXAMPLE 11 was determined using Taq Dye Deoxy Terminater Cycle Sequencing kit (Perkin Elmer). The primers used were T3, T7 primers (Stratagene) and synthetic primers designed according to the OCIF cDNA sequence. The sequences of these primers are shown in sequence numbers 16 to 29. The nucleotide sequence of the OCIF cDNA is shown in sequence number 6 and the amino acid sequence predicted by the cDNA sequence is shown in sequence number 5.

### EXAMPLE 13

### Production of recombinant OCIF by 293/EBNA cells

### i) Construction of the plasmid for expressing OCIF cDNA

pBKOCIF containing about 1.6 kb OCIF cDNA was prepared as described in EXAMPLE 11, and digested with restriction enzymes, BamHI and XhoI. The OCIF cDNA insert was cut out, separated by an agarose gel electrophoresis, and purified using QIAEX gel extraction kit (QIAGEN). The purified OCIF cDNA insert was ligated using DNA ligation kit ver. 2 (Takara Shuzo) to the expression vector pCEP4 (Invitrogen) digested with restriction enzymes, BamHI and XhoI. E. coli. DH5α (Gibco BRL) was transformed with the ligation mixture. The transformants were grown and the plasmid containing the OCIF cDNA (about 1.6 kb) was purified using QIAGEN column (QIAGEN). The expression plasmid pCEPOCIF was precipitated with ethanol, and dissolved in sterile distilled water was used in the expreriments described below.

### ii) Transient expression of OCIF cDNA and analysis of the biological activity

Recombinant OCIF was produced using the expression plasmid, pCEPOCIF prepared in EXAMPLE 13-i) according to the method described below. 8x10⁵ cells of 293/EBNA (Invitrogen) were inoculated in each well of the 6-well plate using IMDM containing 10 % fetal calf serum (Gibco BRL). After the cells were incubated for 24 hours, the culture medium was removed and the cells were washed with serum free IMDM. The expression plasmid, pCEP0CIF and lipofectamine (Gibco BRL) were diluted with 0PTI-MEM (Gibco BRL) and were mixed, and added to the cells in each well according to the manufacture' s instructions. Three µg of pCEP0CIF and 12 µl of lipofectamine were used for each transfection. After the cells were incubated with pCEPOCIF and lipofectamine for 38 hours, the medium was replaced with 1 ml of OPTI-MEM. After the transfected cells were incubated for 30 hours, the conditioned medium was harvested and used for the biological assay. The biological activity of OCIF was analysed according to the method described below. Bone marrow cells obtained from mice, 17 days-old, were suspended in α-MEM (manufactured by GIBCO BRL Co.) containing 10% FBS, 2x10⁻⁸M activated vitamin D₃, and each test sample, and were inoculatd and cultured for 7 days at 37°C in humidified 5%CO₂ as described in EXAMPLE 2. During incubation, 160 µl of old medium in each well was replaced with the same volume of the fresh medium containing test sample diluted with 1x10⁻⁸M of activated vitamin D₃ and α-MEM containing FBS on day 3 and day 5. On day 7, after washing the wells with phosphate buffered saline, cells were fixed with ethanol/acetone (1:1) for 1 min. and then osteoclast development was tested using acid phosphatase activity mesuring kit (Acid Phosphatase, Leucocyte, Catalog No.387-A, Sigma Co.). The decrease of the number of TRAP positive cells was taken as an OCIF activity. As result, the conditioned medium showed the same OCIF activity as natural OCIF protein from IMR-90 conditioned medium (Table 4).

**Table 4**

| OCIF activity of 293/EBNA conditioned medium. | | | | | | | |
|---|---|---|---|---|---|---|---|
| Cultured Cell | Dilution | | | | | | |
| | 1/20 | 1/40 | 1/80 | 1/160 | 1/320 | 1/640 | 1/1280 |
| 0CIF expression vector transfected | ++ | ++ | ++ | ++ | ++ | + | - |
| vector transfected | - | - | - | - | - | - | - |
| untreated | - | - | - | - | - | - | - |
| [ ++ ; OCIF activity inhibiting osteoclast development more than 80%, + ; OCIF activity inhibiting osteoclast development between 30% and 80%, and - ; no 0CIF activity. ] | | | | | | | |

### iii) Isolation of recombinant OCIF protein from 293/EBNA-conditioned medium

293/EBNA-conditioned medium (1.8 1) obtained by cultivating the cells described in example 13-ii) was supplemented with 0.1 % of CHAPS and filtrated with 0.22 *µ*m membrane filter (Steribecs GS, Milipore Co.). The conditioned medium was applied to 50 ml of a heparin Sepharose CL-6B column (2.6 x 10 cm, Pharmacia Co.) equilibrated with 10mM Tris-HCl, pH 7.5. After washing the column with 10mM Tris-HCl, pH 7.5, the adsorbed protein was eluted from the column with linear gradient from 0 to 2 M NaCl at a flow rate of 4 ml/min for 100 min. and fractions (8 ml) were collected. Using 150 *µ*l of each fraction, OCIF activity was assayed according to the method described in EXAMPLE 2. OCIF active fraction (112 ml) eluted with approximately 0.6 to 1.2 M NaCl was obtained.

One hundred twelve ml of the active fraction was diluted to 1000 ml with 10 mM Tris-HCl, 0.1% CHAPS, pH 7.5, and applied to a heparin affinity column (heparin-5PW, 0.8 x 7. 5 cm, Tosoh Co.) equilibrated with 10mM Tris-HCl, 0.1% CHAPS, pH 7.5. After washing the column with 10mM Tris-HCl, 0.1% CHAPS, pH 7.5, the adsorbed protein was eluted from the column with linear gradient from 0 to 2 M NaCl at a flow rate of 0. 5ml/min for 60 min., and fractions (0.5 ml) were collected. Four µl of each fraction was analyzed by SDS-polyacrylamide gel electrophoresis under reducing and non-reducing conditions as described in EXAMPLE 4. On SDS-PAGE under reducing conditions, a single band of r0CIF protein with an apparent 60 KD was detected in fractions from 30 to 32, under non-reducing conditions, bands of rOCIF protein with an apparent 60 KD and 120 KD were also detected in fractions from 30 to 32. The isolated rOCIF fraction from 30 to 32 was designated as recombinant OCIF derived from 293/EBNA (rOCIF(E)). 1.5 ml of the rOCIF(E) (535 *µ*g/ml) was obtained when determined by the method of Lowry using bovine serum albumin as a standard protein.

### EXAMPLE 14

### Production of recombinant OCIF using CHO cells

### i) Construction of the plasmid for expressing OCIF

pBK0CIF containing about 1.6 kb OCIF cDNA was prepared as described in EXAMPLE 11, and digested with restriction enzymes, SalI and EcoRV. About 1.4 kb OCIF cDNA insert was separated by an agarose gel electrophoresis, and purified from the gel using QIAEX gel extraction kit (QIAGEN). The expression vector, pcDL-SR α 296 (Molecular and Cellular Biology, vol 8, p466, 1988) was digested with restriction enzymes, PstI and KpnI. About 3.4 kb of the expression vector fragment was cut out, separated by agarose gel electrophoresis, and purified from the gel using QIAEX gel extraction kit (QIAGEN). The ends of the purified OCIF cDNA insert and the expression vector fragment were blunted using DNA blunting kit (Takara Shuzo). The purified OCIF cDNA insert and the expression vector fragment were ligated using DNA ligation kit ver. 2 (Takara Shuzo). E.coli. DH5a α (Gibco BRL) was transformed with the ligation mixture. The transformant containing the OCIF expression plasmid, pSR α OCIF was obtained.

### ii) Preparation of expression plasmid

The transformant containing the OCIF expression plasmid, pSR α OCIF preprared in the example 13-i) and the transformant containing the mouse DHFR expression plasmid, pBAdDSV shown in W092/01053 were grown according to the standard method. Both plasmids were purified by alkali treatment, polyethylene glycol precipitation, and cesium chrolide density gradient ultra centrifugation according to method of Maniatis et al. (Molecular cloning, 2nd edition).

### iii) Adaptation of CHOdhFr- cells to the protein free medium

CHOdhFr- cells (ATCC, CRL 9096) were cultured in IMDM containing 10 % fetal calf serum. The cells were adapted to EX-CELL 301 (JRH Biosciecnce) and then adapted to EX-CELL PF CHO (JRH Biosciecnce) according to the manufacture's instructions.

### iv) Transfection of the OCIF expression plasmid, and the mouse DHFR expression plasmid, to CHOdhFr- cells.

CHOdhFr- cells prepared in EXAMPLE 14-iii) were transfected by electroporation with pSR α OCIF and pBAdDSV prepared in EXAMPLE 14-ii). 200 *µ*g of pSR α OCIF and 20 *µ*g of pBAdDSV were dissolved under sterile conditions in 0.8 ml of IMDM (Gibco BRL) containing 10 % fetal calf serum CG. 2x10⁷ cells of CHOdhFr- were suspended in 0.8 ml of this medium. The cell suspension was transfered to a cuvette (Bio Rad) and the cells were transfected by electroporation using gene pulser (Bio Rad) under condition of 360 V and 960 µF. The suspension of electroporated cells was transferred to T-flasks (Sumitomo Bakelite) containing 10 ml of EX-CELL PF-CHO, and incubated in the CO₂ incubator for 2 days. Then the transfected cells were inoculated in each well of a 96 well plate (Sumitomo Bakelite) at a density of 5000 cells/well and cultured for about 2 weeks. The transformants expressing DHFR are selected since EX-CELL PF-CHO does not contain nucleotides and the parental cell line CHO dhFr- can not grow in this medium. Most of the transformants expressing DHFR express OCIF since the OCIF expression plasmid was used ten times as much as the mouse DHFR expression plasmid. The transformants whose conditioned medium had high OCIF activity were selected among the transformants expressing DHFR according to the method described in EXAMPLE 2. The transformants that express large amounts of OCIF were cloned by limiting dilution. The clones whose conditioned medium had high OCIF activity were selected as described above and the transformant expressing large amount of OCIF, 5561, was obtained.

### v) Production of recombinant OCIF

To produce recombinant OCIF (r0CIF), EX-CELL 301 medium (3 l) in a 3 l-spiner flask was inoculated with the clone (5561) at a cell-density of 1x10⁵ cells/ml. The 5561 cells were cultured in a spiner flask at 37°C for 4 to 5 days. When the concentration of the 5561 cells reached to 1x10⁶ cells/ml, about 2.7 l of the conditioned medium was harvested. Then about 2.7 l of EX-CELL 301 was added to the spiner flask and the 5561 cells were cultured repeatedly. About 20 l of the conditioned medium was harvested using the three spiner flasks.

### vi) Isolation of recombinant OCIF protein from CHO cells-conditioned medium

CHOcells-conditioned medium (1.0 1) described in EXAMPL 14-v) was supplemented with 1.0 g of CHAPS and filtrated with 0.22 *µ*m membrane filter (Steribecks GS, Milipore Co.). The conditioned medium was applied to a heparin Sepharose-FF column (2.6 x 10 cm, Pharmacia Co.) equilibrated with 10 mM Tris-HCl, pH 7.5. After washing the column with 10 mM Tris-HCl, 0. % CHAPS, pH 7.5, the adsorbed protein was eluted from the column with linear gradient from 0 to 2 M NaCl at a flow rate of 4 ml/min for 100 min. and fractions (8 ml) were collected. Using 150 *µ*l of each fraction, OCIF activity was assayed according to the method described in EXAMPLE 2. Active fraction (112 ml) eluted with approximately 0.6 to 1.2 M NaCl was obtained.

The 112 ml of active fraction was diluted to 1200 ml with 10 mM Tris-HCl, 0.1% CHAPS, pH 7.5, and applied to a affinity column (blue-5PW, 0.5 x 5.0 cm, Tosoh Co.) equilibrated with 10 mM Tris-HCl, 0. 1% CHAPS, pH 7. 5. After washing the column with 10 mM Tris-HCl, 0.1% CHAPS, pH 7.5, the adsorbed protein was eluted from the column with linear gradient from 0 to 3 M NaCl at a flow rate of 0.5ml/min for 60 min., and fractions (0.5 ml) were collected. Four µl of each fraction was subjected to SDS-polyacrylamide gel electrophoresis under reducing and non-reducing conditions as described in EXAMPLE 4. On SDS-PAGE under reducing conditions, a single band of r0CIF protein with apparent 60 KD was detected in fractions 30 to 38, under non-reducing conditions, bands of r0CIF protein with apparent 60 KD and 120 KD were also detected in fractions 30 to 38. The isolated rOCIF fraction, 30 to 38, was designated as purified recombinant OCIF derived from CHO cells (rOCIF(C)). 4.5 ml of the OCIF(C) (113 µg/ml) was obtained when determined by the method of Lowry using bovine serum albumin as a standard protein.

### EXAMPLE 15

### Determination of N-terminal amino acid sequence of rOCIFs

Each 3 *µ*g of the isolated rOCIF(E) and rOCIF(C) was adsorbed to polyvinylidene difluoride (PVDF) membranes with Prospin (PERKIN ELMER Co.). The membranes were washed with 20 % ethanol and the N-terminal amino acid sequences of the adsorbed proteins were analyzed by protein sequencer (PROCISE 492, PERKIN ELMER Co.). The determined N-terminal amino acid sequence is shown in sequence No. 7.

The N-terminal amino acid of r0CIF(E) and r0CIF(C) was the 22th amino acid of glutamine from Met as translation starting point, as shown in sequence number 5. The 21 amino acids from Met to Gln were identified as a signal peptide. The N-terminal amino acid sequence of OCIF isolated from IMR-90 conditioned medium was undetectable. Accordingly, the N-terminal glutamine of OCIF may be blocked by converting from glutamine to pyroglutamine within culturing or purifing.

### EXAMPLE 16

### Biological activity of recombinant(r) OCIF and natural(n) OCIF

### i) Inhibition of vitamin D₃ induced osteoclast formation from murine bone marrow cells

Each the r0CIF(E) and nOCIF sample was diluted with α-MEM (GIBCO BRL Co.) containing 10% FBS and 2x10⁻⁸M of activated vitamin D₃ (a final concentration of 250 ng/ml). Each sample was serially diluted with the same medium, and 100 µl of each diluted sample was added to each well in 96-well plates. Bone marrow cells obtained from mice, 17 days-old, were inoculated at a cell density of 3x10⁵ cells/100 µl/ well to each well in 96-well plates and cultured for 7 days at 37°C in humidified 5%CO₂. On day 7, the cells were fixed and stained with a acid phosphatase mesuring kit (Acid Phosphatase, Leucocyte, No387-A, Sigma) according to the method described in EXAMPLE 2. The decrease of acid phosphatase activity (TRAP) was taken as OCIF activity. The decrease of acid phosphatase-positive cells was evaluated by solubilizing the pigment of dye and measuring absorbance. In detail, 100 µl of a mixture of 0. 1 N NaOH and dimethylsulfoxide (1:1) was added to each well and the well was vibrated to solubilize the dye. After solubilizing the dye completely, an absorbance of each well was measured at 590 nm subtracting the absorbance at 490 nm using microplate reader (Immunoreader NJ-2000, InterMed). The microplate reader was adjusted to 0 absorbance using a well with monolayered bone marrow cells which was cultured in the medium without activated vitamin D₃. The decrease of TRAP activity was expressed as a percentage of the control absorbance value (=100%) of the solubilized dye from wells with bone marrow cells which were cultured in the absence of OCIF. The results are shown in Table 5.

**Table 5**

| Inhibition of vitamin D3-induced osteoclast formation from murine bone marrow cells | | | | | | |
|---|---|---|---|---|---|---|
| OCIF concentration(ng/ml) | 250 | 125 | 63 | 31 | 16 | 0 |
| rOCIF (E) | 0 | 0 | 3 | 62 | 80 | 100 |
| nOCIF | 0 | 0 | 27 | 27 | 75 | 100 (%) |

Both n0CIF and r0CIF(E) inhibited osteoclast formation in a dose dependent manner in the concentration of 16 ng/ml or higher

### ii) Inhibition of vitamin D3-induced osteoclast formation in co-cultures of stromal cells and mouse spleen cells.

Effect of OCIF on osteoclast formation induced by Vitamin D₃ in co-cultures of stromal cells and mouse spleen cells was tested according to the method of N. Udagawa et al. (Endocrinology, vol. 125, p1805-1813, 1989). In detail, each of rOCIF(E), rOCIF(C), and nOCIF sample was serially diluted with α-MEM (GIBC0 BRL Co.) containing 10% FBS, 2x10⁻⁸M of activated vitamin D₃, and 2x10⁻⁷,M dexamethasone, and 100 µl of each the diluted samples was added to each well in 96 well-microwell plates. Murine bone marrow-derived stromal ST2 cells (RIKEN Cell Bank RCB0224) ; 5x10³ cells per 100 µl 1 of α-MEM containing 10% FBS, and spleen cells from ddy mice, 8 weeks-old, ; 1x105 cells per 100 µl in the same medium, were inoculated to each well in 96-well plates and cultured for 5 days at 37°C in humidified 5%CO₂. On day 5, the cells were fixed and stained with a kit for acid phosphatase (Acid Phosphatase, Leucocyte, No387-A, Sigma). The decrease of acid phosphatase-positive cells was taken as OCIF activity. The decrease of acid phosphatase-positive cells was evaluated according to the method described in EXAMPLE 16-i). The results are shown in Table 6 ; rOCIF(E) and rOCIF(C), and Table 7 ; rOCIF(E) and nOCIF.

**Table 6**

| Inhibition of osteoclast formation in co-cultures of stromal cells and mouse spleen cells. | | | | | |
|---|---|---|---|---|---|
| OCIF concentration(ng/ml) | 50 | 25 | 13 | 6 | 0 |
| rOCIF(E) | 3 | 22 | 83 | 80 | 100 |
| rOCIF(C) | 13 | 19 | 70 | 96 | 100 (%) |

**Table 7**

| Inhibition of osteoclast formation in co-cultures of stromal cells and mouse spleen cells. | | | | |
|---|---|---|---|---|
| OCIF concentration(ng/ml) | 250 | 63 | 16 | 0 |
| rOCIF(E) | 7 | 27 | 37 | 100 |
| rOCIF(C) | 13 | 23 | 40 | 100 (%) |
| nOCIF, rOCIF(E) and rOCIF(C) inhibited osteoclast formation in a dose dependent manner in the concentration of 6 - 16 ng/ml or higher | | | | |

### iii) Inhibition of PTH-induced osteoclast formation from murine bone marrow cells.

Effect of OCIF on osteoclast formation induced by PTH was tested according to the method of N. Takahashi et al. (Endocrinology, vol. 122, p1373-1382, 1988). In detail, each the rOCIF(E) and nOCIF sample (125 ng/ml) was serially diluted with α-MEM (manufactured by GIBCO BRL Co.) containing 10% FBS and 2x10⁻⁸M PTH, and 100 *µ*l of each the diluted samples was added to 96 well-plates. Bone marrow cells from ddy mice, 17 days-old, at a cell density of 3x10⁵ cells per 100 *µ*l of α-MEM containing 10% FBS were inoculated to each well in 96-wells plates and cultured for 5 days at 37°C in humidified 5%CO₂. On day 5, the cells were fixed with ethanol/aceton (1:1) for 1 min. at room temperature and stained with a kit for acid phosphatase (Acid Phosphatase, Leucocyte, No387-A, Sigma) according to the method described in EXAMPLE 2. The decrease of acid phosphatase-positive cells was taken as OCIF activity. The decrease of acid phosphatase-positive cells was evaluated according to the method described in EXAMPLE 16-i). The results are shown in Table 8.

**Table 8**

| Inhibition of PTH-induced osteoclast formation from murine bone marrow cells. | | | | | | |
|---|---|---|---|---|---|---|
| OCIF concentration(ng/ml) | 125 | 63 | 31 | 16 | 8 | 0 |
| rOCIF(E) | 6 | 58 | 58 | 53 | 88 | 100 |
| nOCIF | 18 | 47 | 53 | 56 | 91 | 100 |
| nOCIF and rOCIF(E) inhibited osteoclast formation in a dose dependent manner in the concentration of 16 ng/ml or higher | | | | | | |

### iv) Inhibition of IL-11-induced osteoclast formation

Effect of OCIF on osteoclast formation induced by IL-11 was tested according to the method of T. Tamura et al. (Proc. Natl. Acad. Sci. USA, vol. 90, p11924-11928, 1993). In detail, each rOCIF(E) and n0CIF sample was serially diluted with α-MEM (GIBC0 BRL Co.) containing 10% FBS and 20 ng/ml IL-11 and 100 µl of each the diluted sample was added to each well in 96-well plates. Newborn mouse calvaria-derived pre-adipocyte MC3T3-G2/PA6 cells (RIKEN Cell Bank RCB1127) : 5x10³ cells per 100 µl of α-MEM containing 10% FBS, and spleen cells from ddy mouse, 8 weeks-old, ; 1x10⁵ cells per 100 µl in the same medium, were inoculated to each well in 96-well plates and cultured for 5 days at 37 °C in humidified 5%CO₂. On day 5, the cells were fixed and stained with a kit for acid phosphatase (Acid Phosphatase, Leucocyte, No387-A, Sigma). Acid phosphatase positive cells were counted under microscope and a decrease of the cell numbers was taken as OCIF activity. The results are shown in Table 9.

**Table 9**

| | | | | | | | |
|---|---|---|---|---|---|---|---|
| OCIF concentration (ng/ml) | 500 | 125 | 31 | 7.8 | 2.0 | 0.5 | 0 |
| nOCIF | 0 | 0 | 1 | 4 | 13 | 49 | 31 |
| rOCIF(E) | 0 | 0 | 1 | 3 | 10 | 37 | 31 |

Both nOCIF and rOCIF(E) inhibited osteoclast formation in a dose dependent manner in the concentration of 2 ng/ml or higher

The results shown in Table 4-8 indicated that OCIF inhibits all the vitamin D₃, PTH, and IL-11-induced osteoclast formations at almost the same doses. Accordingly, OCIF would be able to be used for treatment of the different types of bone disorders with decreased bone mass, which are caused by different substances which induce bone resorption.

### EXAMPLE 17

### Isolation of monomer-type OCIF and dimer-type OCIF

Each r0CIF(E) and r0CIF(C) sample containing 100 µg of OCIF protein, was supplemented with 1/100 volume of 25 % trifluoro acetic acid and applied to a reverse phase column (PROTEIN-RP, 2.0x250 mm, YMC Co.) equilibrated with 30 % acetonitrile containing 0.1 % trifluoro acetic acid. OCIF protein was eluted from the column with linear gradient from 30 to 55 % acetonitrile at a flow rate of 0.2 ml/min for 50 min. and each OCIF peak was collected. Each the monomer-type OCIF peak fraction and dimer-type OCIF peak fraction was lyophilized, respectively.

### EXAMPLE 18

### Determination of molecular weight of recombinant OCIFs

Each 1 µg of the isolated monomer-type and dimer-type n0CIF purified using reverse phase column according to EXAMPLE 3-iv) and each 1 µg of monomer-type and dimer-type r0CIF described in EXAMPLE 17 was concentrated under vaccum, respectively. Each sample was incubated in the buffer for SDS-PAGE, subjected to SDS-polyacrylamide gel electrophoresis, and protein bands on the gel were stained with silver according to the method described in EXAMPLE 4. Results of electrophoresis under non-reducing conditions and reducing conditions are shown in Figure 6 and Figure 7.

A protein band with an apparent molecular weight of 60 KD was detected in each monomer-type OCIF sample, and a protein band with an apparent molecular weight of 120 KD was detected in each dimer-type OCIF sample in non-reducing conditions. A protein band with an apparent molecular weight of 60 KD was detected in each monomer-type OCIF sample under reducing conditions. Accordingly, molecular weights of monomer-type nOCIF from IMR-90 cells, rOCIF from 293/EBNA cells and rOCIF from CHO cells were almost the same. Molecular weights of dimer-type nOCIF from IMR-90 cells, rOCIF from 293/EBNA cells, and rOCIF from CHO cells were also the same.

### EXAMPLE 19

### Remove N-linked Oligosaccharide chain and Mesuring molecular weight of natural and recombinant OCIF

Each sample containing 5 µg of the isolated monomer-type and dimer-type nOCIF purified using reverse phase column according to EXAMPLE 3-iv) and each sample containing 5 µg of monomer-type and dimer-type r0CIF described in EXAMPLE 17 were concentrated under vaccum. Each sample was dissolved in 9.5 µl of 50 mM sodium phosphate buffer, pH 8.6, containing 100 mM 2-mercaptoethanol, supplemented with 0.5 µl of 250 U/ml N-glycanase (Seikagaku kogyo Co.) and incubated for one day at 37 °C. Each sample was supplemented with 10 µl of 20 mM Tris-HCl, pH 8.0 containing 2 mM EDTA, 5 % SDS, and 0.02 % bromo-phenol blue and heated for 5 min at 100 °C. Each 1 µl of the samples was subjected to SDS-polyacrylamide gel electrophoresis, and protein bands on the gel were stained with silver as described in EXAMPLE 4. The patterns of electrophoresis are shown in Figure 8.

An apparent molecular weight of each the deglycosylated n0CIF from IMR-90 cells, rOCIF from CHO cells, and rOCIF from 293/EBNA cells was 40 KD under reducing conditions. An apparent molecular weight of each untreated n0CIF from IMR-90 cells, rOCIF from 293/EBNA cells, and r0CIF from CHO cells was 60 KD under reducing conditions. Accordingly, the results indicate that the OCIF proteins are glycoproteins with N-linked sugar chains.

### EXAMPLE 20

### Cloning of OCIF variant cDNAs and determination of their DNA squences

The plasmid pBK0CIF, which is inserted OCIF cDNA to pBKCMV (Stratagene), was obtained from one of some purified positive phage as in example 10 and 11. And more, during the screening of the cDNA library with the 397 bp OCIF cDNA probe, the transformants containing plasmids whose insert sizes were different from that of pBKOCIF were obtained. These transformants containing the plasmids were grown and the plasmids were purified according to the standard method. The sequence of the insert DNA in each plasmid was determined using Taq Dye Deoxy Terminater Cycle Sequencing kit (Perkin Elmer). The used primers were T3, T7 primers (Stratagene) and synthetic primers prepared based on the nucleotide sequence of OCIF cDNA. There are four OCIF variants (0CIF2, 3, 4, and 5) in addition to OCIF. The nucleotide sequence of OCIF2 is shown in the sequence number 8 and the amino acid sequence of OCIF 2 predicted by the nucleotide sequence is shown in the sequence number 9. The nucleotide sequence of OCIF3 is shown in the sequence number 10 and the amino acid sequence of 0CIF3 predicted by the nucleotide sequence is shown in the sequence number 11. The nucleotide sequence of OCIF4 is shown in the sequence number 12 and the amino acid sequence of OCIF4 predicted by the nucleotide sequence is shown in the sequence number 13. The nucleotide sequence of OCIF5 is shown in the sequence number 14 and the amino acid sequence of OCIF5 predicted by the nucleotide sequence is shown in the sequence number 15. The structures of OCIF variants are shown in Figures 9 to 12 and are described in brief below. OCIF2

OCIF2 cDNA has a deletion of 21 bp from guanine at nucleotide number 265 to guanine at nucleotide number 285 in OCIF cDNA (sequence number 6). Accordingly OCIF2 has a deletion of 7 amino acids from glutamic acid (Glu) at amino acid number 68 to glutamine (Gln) at amino acid number 74 in OCIF (sequence number 5).

### OCIF3

OCIF3 cDNA has a point mutation at nucleotide number 9 in OCIF cDNA (sequence number 6) where cytidine is replaced with guanine.
Accordingly OCIF3 has a mutation and asparagine (Asn) at amino acid number -19 in OCIF (sequence number 5) is replaced with lysine (Lys). The mutation seems to be located in the signal sequence and have no essential effect on the secreted OCIF3. OCIF3 cDNA has a deletion of 117 bp from guanine at nucleotide number 872 to cytidine at nucleotide number 988 in OCIF cDNA (sequence number 6).
Accordingly OCIF3 has a deletion of 39 amino acids from threonine (Thr) at amino acid number 270 to leucine (Leu) at amino acid number 308 in OCIF (sequence number 5).

### OCIF4

OCIF4 cDNA has two point mutations in OCIF cDNA (sequence number 6). Cytidine at nucleotide number 9 is replaced with guanine and guanine at nucleotide number 22 is replaced with thymidine in OCIF cDNA (sequence number 6).
Accordingly OCIF4 has two mutations. Asparagine (Asn) at amino acid number -19 in OCIF (sequence number 5) is replaced with lysine (Lys), and alanine (Ala) at amino acid number -14 is replaced with serine (Ser). These mutations seem to be located in the signal sequence and have no essential effect on the secreted OCIF4.
OCIF4 cDNA has about 4 kb DNA, which is the intron 2 of OCIF gene, inserted between nucleotide number 400 and nucleotide number 401 in OCIF cDNA (sequence number 6). The open reading frame stops in intron 2.
Accordingly OCIF4 has an additional novel amino acid sequence containing 21 amino acids after alanine (Ala) at amino acid number 112 in OCIF (sequence number 5).

### OCIF5

OCIF5 cDNA has a point mutation at nucleotide number 9 in OCIF cDNA (sequence number 6) where cytidine is replaced with guanine.
Accordingly OCIF5 has a mutation and asparagine (Asn) at amino acid number -19 in OCIF (sequence number 5) is replaced with lysine (Lys). The mutation seems to be located in the signal sequence and have no essential effect on the secreted OCIF5.
0CIF5 cDNA has the latter portion (about 1.8 kb) of intron 2 between nucleotide number 400 and nucleotide number 401 in OCIF cDNA (sequence number 6). The open reading frame stops in the latter portion of intron 2.
Accordingly OCIF5 has an additional novel amino acid sequence containing 12 amino acids after alanine (Ala) at amino acid number 112 in OCIF (sequence number 5).

### EXAMPLE 21

### Production of OCIF variants

### i) Construction of the plasmid for expressing OCIF variants

The plasmid containing OCIF2 or OCIF3 cDNA was obtained as described in EXAMPLE 20 and called pBKOCIF2 and pBKOCIF3, respectively. pBKOCIF2 and pBKOCIF3 were digested with restriction enzymes, BamHI and XhoI. The OCIF2 and OCIF3 cDNA inserts were separated by agarose gel electrophoresis, and purified from the gel using QIAEX gel extraction kit (QIAGEN). The purified OCIF2 and OCIF3 cDNA inserts were individually ligated using DNA ligation kit ver. 2 (Takara Shuzo) to the expression vector pCEP4 (Invitrogen) that had been digested with restriction enzymes, BamHI and XhoI. E.coli. DH5α (Gibco BRL) was transformed with the ligation mixture.
The plasmid containing OCIF4 cDNA was obtained as described in EXAMPLE 20 and called pBKOCIF4. pBKOCIF4 was digested with restriction enzymes, SpeI and XhoI (Takara Shuzo). The OCIF4 cDNA insert was separated by an agarose gel electrophoresis, and purified from the gel using QIAEX gel extraction kit (QIAGEN). The purified OCIF4 cDNA insert was ligated using DNA ligation kit ver. 2 (Takara Shuzo) to the expression vector pCEP4 (Invitrogen) that had been digested with restriction enzymes, NheI and XhoI (Takara Shuzo). E.coli. DH5 α (Gibco BRL) was transformed with the ligation mixture.
The plasmid containing OCIF5 cDNA was obtained as described in EXAMPLE 20 and was called pBKOCIF5. pBKOCIF5 was digested with restriction enzyme, HindIII (Takara Shuzo). The 5'portion of the coding region in the OCIF5 cDNA insert was separated by agarose gel electrophoresis, and purified from the gel using QIAEX gel extraction kit (QIAGEN). The OCIF expression plasmid, pCEPOCIF, obtained in EXAMPLE 13-i) was digested with restriction enzyme, HindIII (Takara Shuzo). The 5'portion of the coding region in the OCIF cDNA was removed. The rest of the plasmid that contains pCEP vector and the 3'portion of the coding region of OCIF cDNA was called pCEPOCIF-3'. pCEPOCIF-3' was separated by an agarose gel electrophoresis, and purified from the gel using QIAEX gel extraction kit (QIAGEN). The OCIF5 cDNA HindIII fragment and pCEP0CIF-3' were ligated using DNA ligation kit ver. 2 (Takara Shuzo). E.coli. DH5 α (Gibco BRL) was transformed with the ligation mixture.
The obtained transformants were grown at 37 °C overnight and the OCIF variants expression plasmids (pCEPOCIF2, pCEPOCIF3, pCEPOCIF4, and pCEPOCIF5) were purified using QIAGEN column (QIAGEN). These OCIF-variants-expression plasmids were precipitated with ethanol, dissolved in sterile distilled water, and used in the expreriments described below.

### ii) Transient expression of OCIF variant cDNAs and analysis of the biological activity of recombinant OCIF variants.

Recombinant OCIF variants were produced using the expression plasmid, pCEPOCIF2, pCEPOCIF3, pCEPOCIF4, and pCEPOCIF5 prepared as described in EXAMPLE 21-i) according to the method described in EXAMPLE 13-ii). The biological activities of recombinant OCIF variants were analysed. The results were that these OCIF variants (0CIF2, OCIF3, OCIF4, and OCIF5) had a weak activity.

### EXAMPLE 22

### Preparation of OCIF mutants

### i) Construction of a plasmid vector for subcloning cDNAs encoding OCIF mutants

The plasmid vector (5 *µ*g) described in EXAMPLE 11 was digested with restriction enzymes Bam HI and Xho I ( Takara Shuzo). The digested DNA was subjected to a preparative agarose gel electrophoresis. DNA fragment with an approximate size of 1.6 kilobase pairs (kb) that contained the entire coding sequence for OCIF was purified from the gel using QIAEX gel extraction kit (QIAGEN). The purified DNA was dissolved in 20 µl of sterile distilled water. This solution was designated DNA solution 1. p Bluescript II SK + (3 µg) (Stratagene) was digested with restriction enzymes Bam HI and Xho I (Takara Shuzo). The digested DNA was subjected to preparative agarose gel electrophoresis. DNA fragment with an approximate size of 3.0 kb was purified from the gel using QIAEX DNA extraction kit (QIAGEN). The purified DNA was dissolved in 20 µl of sterile distilled water. The solution was designated DNA solution 2. One microliter of DNA solution 2, 4 µl of DNA solution 1 and 5 µl of ligation buffer I of DNA ligation kit ver. 2 (Takara Shuzo) were mixed and incubated at 16 °C for 30 min. (The ligation mixture was used for the transformation of E. coli in a manner described below). Conditions for transformation of E. coli were as follows. One hundred microliters of competent E. coli DH5 α cells (GIBCO BRL) and 5 *µ*l of the ligation mixture was mixed in a sterile 15-ml tube (IWAKI glass). The tube was kept on ice for 30 min. After incubation for 45 sec at 42°C, to the cells was added 250 *µ*l of L broth (1% Tryptone, 0.5% yeast extract, 1% NaCl). The cell suspension was then incubated for 1hr. at 37°C with shaking. Fifty microliters of the cell suspension was plated onto an L-agar plate containing 50 *µ*g/ml of ampicillin. The plate was incubated overnight at 37°C.

Six colonies which grew on the plate were individually incubated in 2 ml each of L-broth containing 50 *µ*g/ml of ampicillin overnight at 37°C with shaking. The structure of the plasmids in the colonies was analyzed. A plasmid in which the 1.6-kb DNA fragment containing the entire OCIF cDNA is inserted between the digestion sites of Bam HI and Xho I of pBluescript II SK + was obtained and designated as pSK + -OCIF.

### ii) Preparation of mutants in which one of the Cys residues in OCIF is replaced with Ser residue

### 1) Introduction of mutations into OCIF cDNA

OCIF mutants were prepared in which one of the five Cys residues present in OCIF at positions 174, 181, 256, 298 and 379 (in SEQUENCE NO 4) was replaced with Ser residue and were designated OCIF-C19S(174Cys to Ser), OCIF-C20S (181Cys to Ser), OCIF-C21S (256Cys to Ser), OCIF-C22S (298Cys to Ser) and OCIF-C23S (379Cys to Ser), respectively.

To prepare the mutants, nucleotides encoding the corresponding Cys residues were replaced with those encoding Ser. Mutagenesis was carried out by a two-step polymerase chain reaction (PCR). The first step of the PCRs consisted of two reactions, PCR 1 and PCR 2.

| | | |
|---|---|---|
| PCR 1 | 10X Ex Taq Buffer (Takara Shuzo) | 10 *µ*l |
| | 2.5 mM solution of dNTPs | 8 *µ*l |
| | the plasmid vector described in EXAMPLE 11 (8ng/ml) | 2 *µ*l |
| | sterile distilled water | 73.5 *µ*l |
| | 20 µM solution of primer 1 | 5 µl |
| | 100 µM solution of primer 2 (for mutagenesis) | 1 µl |
| | Ex Taq (Takara Shuzo) | 0.5 µl |
| | | |
| PCR 2 | 10X Ex Taq Buffer (Takara Shuzo) | 10 µl |
| | 2.5 mM solution of dNTPs | 8 µl |
| | the plasmid vector described in EXAMPLE 11 (8ng/ml) | 2 µl |
| | sterile distilled water | 73.5 µl |
| | 20 *µ*M solution of primer 3 | 5 µl |
| | 100 *µ*M solution of primer 4 (for mutagenesis) | 1 µl |
| | Ex Taq (Takara Shuzo) | 0.5 µl |

Specific sets of primers were used for each mutation and other components were unchanged. Primers used for the reactions are shown in Table 10. The nucleotide sequences of the primers are shown in SEQUENCE NO: 20,23,27 and 30-40. The PCRs were performed under the following conditions as follows. An initial denaturation step at 97°C for 3 min was followed by 25 cycles of denaturation at 95°C for 1 min annealing at 55°C for 1 min and extension at 72°C for 3 min. After these amplification cycles, final extension was performed at 70°C for 5 min. The size of the PCR prodcts was confirmed by agarose gel electrophoresis using reaction solution. After the first PCR, excess primers were removed using Amicon microcon (Amicon). The final volume of the solutions that contained the PCR products were made to 50µl with sterile distilled water. These purified PCR products were used for the second PCR (PCR 3).

| | | |
|---|---|---|
| PCR 3 | 10X Ex Taq Buffer (Takara Shuzo) | 10 - µl |
| | 2.5 mM solution of dNTPs | 8 µl |
| | solution containing DNA fragment obtained from PCR 1 | 5 µl |
| | solution containing DNA fragment obtained from PCR 2 | 5 µl |
| | sterile distilled water | 61. 5 µl |
| | 20 µM solution of primer 1 | 5 µl |
| | 20 µM solution of primer 3 | 5 µl |
| | Ex Taq (Takara Shuzo) | 0.5 µl |

**Table 10**

| mutants | primer-1 | primer-2 | primer-3 | primer-4 |
|---|---|---|---|---|
| OCIF-C19S | IF 10 | C19SR | IF 3 | C19SF |
| OCIF-C20S | IF 10 | C20SR | IF 3 | C20SF |
| OCIF-C21S | IF 10 | C21SR | IF 3 | C21SF |
| OCIF-C22S | IF 10 | C22SR | IF 14 | C22SF |
| OCIF-C23S | IF 6 | C23SR | IF 14 | C23SF |

The reaction conditions were exactly the same as those for PCR 1 or PCR 2. The size of the PCR prodcts was confirmed by 1.0 % or 1.5 % agarose gel electrophoresis. The DNA fragments were precipitated with ethanol, dried under vacuum and dissolved in 40 *µ*l of sterile distilled water. The solutions containing DNA fragments with mutation C19S, C20S, C21S, C22S and C23S were designated as DNA solution A, DNA solution B, DNA solution C, DNA solution D and DNA solution E, respectively.
The DNA fragment which is contained in solution A (20 µl) was digested with restriction enzymes Nde I and Sph I (Takara Shuzo). A DNA fragment with an approximate size of 400 base pairs (bp) was extracted from a preparative agarose gel and dissolved in 20 µl of sterile distilled water. This DNA solution was designated DNA solution 3. Two micrograms of pSK + -OCIF was digested with restriction enzymes Nde I and Sph I. A DNA fragment with an approximate size of 4.2 kb was purified from a preparative agarose gel with QIAEX gel extraction kit and dissolved in 20 µl of sterile distilled water. This DNA solution was designated as DNA solution 4. Two microliters of DNA solution 3, 3 µl of DNA solution 4 and 5 µl of ligation buffer I of DNA ligation kit ver. 2 were mixed and ligation reaction was carried out.
Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C19S.

The DNA fragment which is contained in solution B (20 µl) was digested with restriction enzymes Nde I and Sph I. A DNA fragment with an approximate size of 400 bp was extracted from a preparative agarose gel with QIAEX gel extraction kit and dissolved in 20 µl of sterile distilled water. This DNA solution was designated DNA solution 5. Two microliters of DNA solution 5, 3 µl of DNA solution 4 and 5 µl of ligation buffer I of DNA ligation kit ver. 2 were mixed and ligation reaction was carried out. Competent E. coli DH5 α cells were transformed with 5 *µ*l of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C20S.
The DNA fragment which is contained in solution C (20 µl) was digested with restriction enzymes Nde I and Sph I. A DNA fragment with an approximate size of 400 bp was extracted from a preparative agarose gel with QIAEX gel extraction kit and dissolved in 20 µl of sterile distilled water. This DNA solution was designated as DNA solution 6. Two microliters of DNA solution 6, 3 µl of DNA solution 4 and 5 µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C21S.
The DNA fragment which is contained in solution D (20 µl) was digested with restriction enzymes Nde I and Bst PI. A DNA fragment with an approximate size of 600 bp was extracted from a preparative agarose gel with QIAEX gel extraction kit and dissolved in 20 µl of sterile distilled water. This DNA solution was designated as DNA solution 7. Two micrograms of pSK + -OCIF was digested with restriction enzymes Nde I and Bst PI. A DNA fragment with an approximate size of 4.0 kb was extracted from a preparative agarose gel with QIAEX gel extraction kit and dissolved in 20 µl of sterile distilled water. This DNA solution was designated as DNA solution 8. Two microliters of DNA solution 7, 3 µl of DNA solution 8 and 5 µl of ligation buffer I of DNA ligation kit ver. 2 were mixed and ligation reaction was carried out.
Competent E. coli DH5 α cells were transformed with 5 µl 1 of the ligation mixture. Ampicillin-resistant transformants were screened for a clone containing a plasmid DNA in which the 600-bp Nde I-BstPI fragment with the mutation (the C22S mutation) is substituted for the 600-bp Nde I-Bst PI fragment of pSK+ -OCIF by analyzing the DNA structure. DNA structure was analyzed by restriction enzyme mapping and by DNA sequencing. The plasmid thus obtained was named pSK-OCIF-C22S.

The DNA fragment which is contained in solution E (20 *µ*l) was digested with restriction enzymes Bst PI and Eco RV. A DNA fragment with an approximate size of 120 bp was extracted from a preparative agarose gel with QIAEX gel extraction kit and dissolved in 20 *µ*l of sterile distilled water. This DNA solution was designated as DNA solution 9. Two micrograms of pSK + -OCIF was digested with restriction enzymes Bst EII and Eco RV. A DNA fragment with an approximate size of 4.5 kb was extracted from a preparative agarose gel with QIAEX gel extraction kit and dissolved in 20 *µ*l of sterile distilled water. This DNA solution was designated as DNA solution 10. Two microliters of DNA solution 9, 3 *µl* of DNA solution 10 and 5 *µ*l of ligation buffer I of DNA ligation kit ver. 2 were mixed and ligation was carried out. Competent E. coli DH5 α cells were transformed with 5 *µ*l of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-C23S.

### 2) Construction of vectors for expressing the OCIF mutants

pSK-OCIF-C19S, pSK-OCIF-C20S, pSK-OCIF-C21S, pSK-OCIF-C22S and pSK-OCIF-C23S were digested with restriction enzymes Bam HI and Xho I. The 1.6 kb Bam HI-Xho I DNA fragment encoding each OCIF mutant was isolated and dissolved in 20 µl of sterile distilled water. The DNA solutions that contain 1.6 kb cDNA fragments derived from pSK-OCIF-C19S, pSK-OCIF-C20S, pSK-OCIF-C21S, pSK-OCIF-C22S and pSK-OCIF-C23S were designated C19S DNA solution, C20S DNA solution, C21S DNA solution, C22S DNA solution and C23S DNA solution, respectively. Five micrograms of a expression vector pCEP 4 (Invitrogen) was digested with restriction enzymes Bam HI and Xho I. A DNA fragment with an approximate size of 10 kb was purified and dissolved in 40 µl of sterile distilled water. This DNA solution was designated as pCEP 4 DNA solution. One microliter of pCEP 4 DNA solution and 6 µl of either C19SDNA solution, C20S DNA solution, C21S DNA solution, C22S DNA solution or C23S DNA solution were independently mixed with 7 µl of ligation buffer I of DNA ligation kit ver. 2 and ligation reactions were carried out. Competent E. coli i DH5α cells (100 µl) were transformed with 7 µl of each ligation mixture. Ampicillin-resistant transformants were screened for clones containing plasmid in which a 1.6-kb cDNA fragment is inserted between the recognition sites of Bam HI and Xho I of pCEP 4 by analyzing the DNA structure. The plasmide which were obtained containing the cDNA encoding 0CIF-C19S, OCIF-C20S, OCIF-C21S, OCIF-C22S and OCIF-C23S were designated pCEP4-OCIF-C19S, pCEP4-OCIF-C20S, pCEP4-OCIF-C21S, pCEP4-OCIF-C22S and pCEP4-OCIF-C23S, respectively.

### ii) Preparation of domain-deletion mutants of OCIF

### (1) deletion mutagenesis of OCIF cDNA

A series of OCIF mutants with deletions of from Thr 2 to Ala 42, from Pro 43 to Cys 84, from Glu 85 to Lys 122, from Arg 123 to Cys 164, from Asp 177 to Gln 251 and from Ile 252 to His 326 were prepared (positions of the amino acid residues are shown in SEQUENCE NO: 4). These mutants were designated as 0CIF-DCR1, OCIF-DCR2, OCIF-DCR3, OCIF-DCR4, OCIF-DDD1 and OCIF-DDD2, respectively. Mutagenesis was performed by two-step PCR as described in EXAMPLE 22-(ii). The primer sets for the reactions are shown in Table 11 and the nucleotide sequences of the primers are shown in SEQUENCE NO:19, 25, 40-53, and 54.

**Table 11**

| mutants | primer-1 | primer-2 | primer-3 | primer-4 |
|---|---|---|---|---|
| OCIF-DCR1 | XhoI F | DCR1R | IF 2 | DCR1F |
| OCIF-DCR2 | XhoI F | DCR2R | IF 2 | DCR2F |
| OCIF-DCR3 | XhoI F | DCR3R | IF 2 | DCR3F |
| OCIF-DCR4 | XhoI F | DCR4R | IF 16 | DCR4F |
| OCIF-DDD1 | IF 8 | DDD1R | IF 14 | DDD1F |
| OCIF-DDD2 | IF 8 | DDD2R | IF 14 | DDD2F |

The final PCR products were precipitated with ethanol, dried under vacuum and dissolved in 40 *µ*l of sterile distilled water. Solutions of DNA fragment coding for portions of OCIF-DCR1, OCIF-DCR2, OCIF-DCR3, OCIF-DCR4, OCIF-DDD1 and OCIF-DDD2 were designated as DNA solutions F, G, H, I, J and K, respectively.
The DNA fragment which is contained in solution F (20 *µ*l) was digested with restriction enzymes Nde I and Xho I. A DNA fragment with an approximate size of 500 bp was extracted from a preparative agarose gel with QIAEX gel extraction kit and dissolved in 20 *µ*l of sterile distilled water. This DNA solution was designated DNA solution 11. Two micrograms of pSK+ -OCIF was digested with restriction enzymes Nde I and Xho I. A DNA fragment with an approximate size of 4.0 kb was extracted from a preparative agarose gel with QIAEX gel extraction kit and dissolved in 20 µl of sterile distilled water. This DNA solution was designated DNA solution 12. Two microliters of DNA solution 11, 3 µl of DNA solution 12 and 5 µl of ligation buffer I of DNA ligation kit ver. 2 were mixed and ligation was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR1.

The DNA fragment which is contained in solution G (20 *µ*l) was digested with restriction enzymes Nde I and Xho I. A DNA fragment with an approximate size of 500 bp was extracted from a preparative agarose gel with QIAEX gel extraction kit and dissolved in 20 *µ*l of sterile distilled water. This DNA solution was designated as DNA solution 13. Two microliters of DNA solution 13, 3 *µ*l of DNA solution 12 and 5 *µ*l of ligation buffer I of DNA ligation kit ver. 2 were mixed and ligation was carried out. Competent E. coli DH5a cells were transformed with 5 *µ*l of the ligation mixture. Ampicillin-resistant transformants were screened for a clone containing plasmid DNA. DNA structure was analyzed by restriction enzyme mapping and by DNA sequencing. The plasmid thus obtained was named pSK-OCIF-DCR2.
The DNA fragment which is contained in solution H (20 *µ*l) was digested with restriction enzymes Nde I and Xho I. A DNA fragment with an approximate size of 500 bp was extracted from a preparative agarose gel with QIAEX gel extraction kit and dissolved in 20 *µ*l of sterile distilled water. This DNA solution was designated as DNA solution 14. Two microliters of DNA solution 14, 3 *µ*l of DNA solution 12 and 5 *µ*l of ligation buffer I of DNA ligation kit ver. 2 were mixed and ligation reaction was carried out. Competent E. coli DH5 α cells were transformed with 5 *µ*l of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR3.
The DNA fragment which is contained in solution I (20 µl) was digested with restriction enzymes Xho I and Sph I. A DNA fragment with an approximate size of 900 bp was extracted from a preparative agarose gel with QIAEX gel extraction kit and dissolved in 20 µl of sterile distilled water. This DNA solution was designated as DNA solution 15. Two micrograms of pSK+ -OCIF was digested with restriction enzymes Xho I and Sph I. A DNA fragment with an approximate size of 3.6 kb was extracted from a preparative agarose gel with QIAEX gel extraction kit and dissolved in 20 µl of sterile distilled water. This DNA solution was designated as DNA solution 16. Two microliters of DNA solution 15, 3 µl of DNA solution 16 and 5 µl of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DCR4.
The DNA fragment which is contained in solution J (20 *µ*l) was digested with restriction enzymes BstP I and Nde I. A DNA fragment with an approximate size of 400 bp was extracted from a preparative agarose gel with QIAEX gel extraction kit and dissolved in 20 *µ*l of sterile distilled water. This DNA solution was designated as DNA solution 17. Two microliters of DNA solution 17, 3 *µ*l of DNA solution 8 and 5 *µ*l of ligation buffer I of DNA ligation kit ver. 2 were mixed and ligation reaction was carried out. Competent E. coli DH5 α cells were transformed with 5 *µ*l of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DDD1. The DNA fragment which is contained in solution K (20 µl was digested with restriction enzymes Nde I and BstP I. A DNA fragment with an approximate size of 400 bp was extracted from a preparative agarose gel with QIAEX gel extraction kit and dissolved in 20 µl of sterile distilled water. This DNA solution was designated as DNA solution 18. Two microliters of DNA solution 18, 3µl of DNA solution 8 and 5 *µ*l of ligation buffer I of DNA ligation kit ver. 2 were mixed and ligation reaction was carried out. Competent E. coli DH5 α cells were transformed with 5 µl of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-DDD2.

### 2) Construction of vectors for expressing the OCIF mutants

pSK-OCIF-DCR1, pSK-OCIF-DCR2, pSK-OCIF-DCR3, pSK-OCIF-DCR4, pSK-OCIF-DDD1 and pSK-OCIF-DDD2 were digested with restriction enzymes Bam HI and Xho I. The Bam HI-Xho I DNA fragment containing entire coding sequence for each OCIF mutant was isolated and dissolved in 20 µl of sterile distilled water. These DNA solutions that contain the Bam HI-Xho I fragment derived from pSK-OCIF-DCR1, pSK-OCIF-DCR2, pSK-OCIF-DCR3, pSK-OCIF-DCR4, pSK-OCIF-DDD1 and pSK-OCIF-DDD2 were designated DCR1 DNA solution, DCR2 DNA solution, DCR3 DNA solution, DCR4 DNA solution, DDD1 DNA solution and DDD2 DNA solution, respectively. One microliter of pCEP 4 DNA solution and 6 µl of either DCR1 DNA solution, DCR2 DNA solution, DCR3 DNA solution, DCR4 DNA solution, DDD1 DNA solution or DDD2 DNA solution were independently mixed with 7 µl of ligation buffer I of DNA ligation kit ver. 2 and ligation reactions were carried out. Competent E. coli DH5 *α* cells (100 *µ*l) were transformed with 7 µl of each ligation mixture. Ampicillin-resistant transformants were screened for a clone containing a plasmid DNA in which the DNA fragment with deletions is inserted between the recognition sites of Bam HI and Xho I of pCEP 4 by analyzing the DNA structure. The plasmids containing the cDNA encoding OCIF-DCR1, OCIF-DCR2, OCIF-DCR3, OCIF-DCR4, OCIF-DDD1 and OCIF-DDD2 were designated as pCEP4-OCIF-DCR1, pCEP4-OCIF-DCR2, pCEP4-OCIF-DCR3, pCEP4-OCIF-DCR4, pCEP4-OCIF-DDD1 and pCEP4-OCIF-DDD2, respectively.

### iii) Preparation of OCIF with C-terminal domain truncation

### (1) mutagenesis of OCIF cDNA

A series of OCIF mutants with deletions of from Cys at amino acid residue 379 to Leu 380, from Ser 331 to Leu 380, from Asp 252 to Leu 380, from Asp 177 to Leu 380, from Arg 123 to Leu 380 and from Cys 86 to Leu 380 was prepared. Positions of the amino acid residues are shown in SEQUENCE NO: 4. These mutants were designated as OCIF-CL, OCIF-CC, OCIF-CDD2, OCIF-CDD1, OCIF-CCR4 and OCIF-CCR3, respectively.

Mutagenesis for OCIF-CL was performed by the two-step PCR as described in EXAMPLE 22-(ii). The primer set for the reaction is shown in Table 12. The nucleotide sequences of the primers are shown in SEQUENCE NO:23, 40, 55, and 56. The final PCR products were precipitated with ethanol, dried under vacuum and dissolved in 40 *µ*l of sterile distilled water. This DNA solution was designated as solution L.

The DNA fragment which is contained in solution L (20 *µ*l) was digested with restriction enzymes BstP I and EcoR V. A DNA fragment with an approximate size of 100 bp was extracted from a preparative agarose gel with QIAEX gel extraction kit and dissolved in 20 *µ*l of sterile distilled water. This DNA solution was designated as DNA solution 19. Two microliters of DNA solution 19, 3 *µ*l of DNA solution 10 (described in EXAMPLE 22-(ii)) and 5 *µ*l of ligation buffer I of DNA ligation kit ver. 2 were mixed and ligation reaction was carried out. Competent E. coli DH5 α cells were transformed with 5 *µ*l of the ligation mixture. Ampicillin-resistant transformants were screened for a clone containing a plasmid DNA. DNA structure was analyzed by restriction enzyme mapping and by DNA sequencing. The plasmid thus obtained was named pSK-OCIF-CL Mutagenesis of OCIF cDNA to prepare OCIF-CC, OCIF-CDD2, OCIF-CDD1, 0CIF-CCR4 and OCIF-CCR3 was performed by a one-step PCR. PCR reactions for mutagenesis to prepare OCIF-CC, OCIF-CDD2, OCIF-CDD1, OCIF-CCR4 and OCIF-CCR3

| | |
|---|---|
| 10X Ex Taq Buffer (Takara Shuzo) | 10 *µ*l |
| 2. 5 mM solution of dNTPs | 8 *µ*l |
| the plasmid vector containing the entire OCIF cDNA described in EXAMPLE 11 (8ng/ml) | 2 *µ*l |
| sterile distilled water | 73.5 *µ*l |
| 20 *µ*M solution of primer OCIF Xho F | 5 *µ*l |
| 100 *µ*M solution of primer (for mutagenesis) | 1 *µ*l |
| Ex Taq (Takara Shuzo) | 0.5 *µ*l |

**Table 12**

| mutants | primer-1 | primer-2 | primer-3 | primer-4 |
|---|---|---|---|---|
| OCIF-CL | IF 6 | CL R | IF 14 | CL F |

Specific primers were used for each mutagenesis and other components were unchanged.
Primers used for the mutagenesis are shown in Table 13. Their nucleotide sequences are shown in SEQUENCE NO:57-61. The components of each PCR were mixed in a microcentrifuge tube and PCR was performed as follows. The microcentrifuge tubes were treated for 3 minutes at 97 °C and then incubated sequentially, for 30 seconds at 95 °C, 30 seconds at 50 °C and 3 minutes at 70 °C. This three-step incubation procedure was repeated 25 times, and after that, the tubes were incubated for 5 minutes at 70 °C. An aliquot of the reaction mixture was removed from each tube and analyzed by an agarose gel electrophoresis to confirm the size of each product.
The size of the PCR products was confirmed on an agarose gel. Excess primers in the PCRs were removed using Amicon microcon (Amicon) after completion of the reaction. The DNA fragments were precipitated with ethanol, dried under vacuum and dissolved in 40 µl of sterile distilled water. The DNA fragment in each DNA solution was digested with restriction enzymes Xho I and Bam HI. After the reactions, DNA was precipitated with ethanol, dried under vacuum and dissolved in 20 ul of sterile distilled water.

The solutions containing DNA fragment with the CC deletion, the CDD2 deletion, the CDD1 deletion, the CCR4 deletion and the CCR3 deletion were designated as CC DNA solution, CDD2 DNA solution, CDD1 DNA solution, CCR4 DNA solution and CC R3 DNA solution, respectively.

**Table 13**

| mutants | primers for the mutagenesis |
|---|---|
| OCIF-CC | CC R |
| OCIF-CDD2 | CDD2 R |
| OCIF-CDD1 | CDD1 R |
| OCIF-CCR4 | CCR4 R |
| OCIF-CCR3 | CCR3 R |

### (2) Construction of vectors for expressing the OCIF mutants

pSK-OCIF-CL was digested with restriction enzymes Bam HI and Xho I. The Bam HI-Xho I DNA fragment containing the entire coding sequence for OCIF-CL was isolatedand dissolved in 20 *µ*l of sterile distilled water. This DNA solution was designated as CL DNA solution. One microliter of pCEP 4 DNA solution and 6 *µ*l of either of CL DNA solution, CC DNA solution, CDD2 DNA solution, CDD1 DNA solution, CCR4 DNA solution or CCR3 DNA solution were independently mixed with 7 *µ*l of ligation buffer I of DNA ligation kit ver. 2 and ligation reactions were carried out. Competent E. coli DH5 α cells (100 *µ*l) were transformed with 7 *µ*l of each ligation mixture. Ampicillin-resistant transformants were screened for clones containing plasmids which have the desirable mutations in OCIF cDNA by analyzing the DNA structure. In each plasmid, OCIF cDNA fragment having a deletion were inserted between the recognition sites of Xho I and Bam HI of pCEP 4. The plasmids containing the cDNA encoding OCIF-CL, OCIF-CC, OCIF-CDD1, OCIF-CDD2, OCIF-CCR4 and OCIF-CCR3 were designated pCEP4-OCIF-CL, pCEP4-OCIF-CC, pCEP4-OCIF-CDD2, pCEP4-OCIF-CDD1, pCEP4-OCIF-CCR4 and pCEP4-OCIF-CCR3, respectively.

### iv) Preparation of OCIF mutants with C-terminal truncation

### (1) Introduction of C-terminal truncation to OCIF

A series of OCIF mutants with C-terminal truncation was prepared. OCIF mutant in which 10 residues of from Gln at 371 to Leu at 380 are replaced with 2 residues of Leu-Val was designated OCIF-CBst. OCIF mutant in which 83 residues of from Cys 298 to Leu 380 are replaced with 3 residues of Ser-Leu-Asp was designated OCIF-CSph. OCIF mutant in which 214 residues of from Asn 167 to Leu 380 are removed was designated OCIF-CBsp. OCIF muatant in which 319 residues of from Asp 62 to Leu 380 are replaced with 2 residues of Leu-Val was designated OCIF-CPst. Positions of the amino acid residues are shown in SEQUENCE NO: 4.

Two micrograms each of pSK + -OCIF was digested with one of the restriction enzymes, Bst PI, Sph I, PstI (Takara Shuzo), and Bsp EI (New England Biolabs), and followed by phenol extraction and ethanol precipitation. The precipitated DNA was dissolved in 10 µl of sterile distilled water. Ends of the DNAs in 2 µl of each solution were blunted using a DNA blunting kit in final volumes of 5 µl. To the reaction mixtures, 1 µg (1 µl) of an Amber codon-containing Xba I linker (5'-CTAGTCTAGACTAG-3') and 6 *µ*l of ligation buffer I of DNA ligation kit ver. 2 were added.

After the ligation reactions, 6 *µ*l each of the reaction mixtures was used to transform E. coli DH5 α. Ampicillin-resistant transformants were screened for clones containing plasmids. DNA structure was analyzed by restriction enzyme mapping and by DNA sequencing. The plasmids thus obtained were named pSK-OCIF-CBst, pSK-OCIF-CSph, pSK-OCIF-CBsp and pSK-OCIF-CPst, respectively.

### (2) Construction of vectors for expressing the OCIF mutants

pSK-OCIF-CBst, pSK-OCIF- CSph, pSK-OCIF-CBsp and pSK-OCIF-CPst were digested with restriction enzymes Bam HI and Xho I. The 1.5 kb of DNA fragment containing entire coding sequence for each OCIF mutant was isolated and dissolved in 20 µl of sterile distilled water. These DNA solutions that contain the Bam HI-XhoI fragment derived from pSK-OCIF-CBst, pSK-OCIF- CSph, pSK-OCIF-CBsp and pSK-OCIF-CPst were designated as CBst DNA solution, CSph DNA solution, CBsp DNA solution and CPst DNA solution, respectively. One microliter of pCEP 4 DNA solution (described in EXAMPLE 22-ii)) and 6 µl of either CBst DNA solution, CSph DNA solution, CBsp DNA solution or CPst DNA solution were independently mixed with 7 µl of ligation buffer I of DNA ligation kit ver. 2 and ligation reactions were carried out. Competent E. coli DH5 α cells (100 *µ*l) were transformed with 7 *µ*l of each ligation mixture. Ampicillin-resistant transformants were screened for clones containing plasmids in which cDNA fragment is inserted between the recognition sites of Bam HI and Xho I of pCEP 4 by analyzing the DNA structure. The plasmids containing the cDNA encoding OCIF-CBst, OCIF-CSph, OCIF-CBsp and OCIF-CPst were designated as pCEP4-OCIF-CBst, pCEP4-OCIF- CSph, pCEP4-OCIF-CBsp and pCEP4-OCIF-CPst, respectively.

### v) Preparetion of vectors for expressing the OCIF mutants

E. coli clones harboring the expression vectors for OCIF mutants (total of 21 clones) were grown and the vectors were purified by QIAGEN column (QIAGEN). All the expression vectors were precipitated with ethanol and dissolved in appropriate volumes of sterile distilled water and used for further manipupations shown below.

### vi) Transient expression of the cDNAs for OCIF mutants and biological activities of the mutants

OCIF mutants were produced using the expression vectors prepared in EXAMPLE 22-v). The method was essentially the same as described in EXAMPLE 13. Only the modified points are described below. A 24-well plate was used for the DNA transfection. 2X10⁵ cells of 293/EBNA suspended in IMDM containing 10% fetal bovine serum were seeded into each well of the plate. One microgram of purified vector DNA and 4 µl of lipofectamine were used for each transfection. Mixture of an expression vector and lipofectamine in OPTI-MEM (GIBCO BRL) in a final volume of 0.5 ml was added to the cells in a well. After the cells were incubated at 37°C for 24 hr in a CO₂ incubator, the medium was replaced with 0.5 ml of Ex-cell 301 medium (JSR). The cells were incubated at 37 °C for 48 more hours in the CO₂ incubator. The conditioned medium was collected and used for assay for in vitro biological activity. The nucleotide sequences of cDNAs for the OCIF mutants are shown in SEQUENCE NO:83-103. The deduced amino acid sequences for the OCIF mutants are shown in SEQUENCE NO: 62-82. The assay for in vitro biological activity was performed as described in EXAMPLE 13. Antigen concentration of each conditioned medium was determined by ELISA as described in EXAMPLE 24. Table 14 shows specific activity of the mutants relative to that of the unaltered OCIF.

**Table 14**

| mutants | activity |
|---|---|
| the unaltered 0IF | ++ |
| OCIF-C19S | + |
| OCIF-C20S | ± |
| OCIF-C21S | ± |
| OCIF-C22S | + |
| OCIF-C23S | + + |
| OCIF-DCR1 | ± |
| OCIF-DCR2 | ± |
| OCIF-DCR3 | ± |
| OCIF-DCR4 | ± |
| OCIF-DDD1 | + |
| OCIF-DDD2 | ± |
| OCIF-CL | + + |
| OCIF-CC | + + |
| OCIF-CDD2 | + + |
| OCIF-CDD1 | + |
| OCIF-CCR4 | ± |
| OCIF-CCR3 | ± |
| OCIF-CBst | ++ |
| OCIF-CSph | + + |
| OCIF-CBsp | ± |
| OCIF-CPst | ± |
| ++ indicates relative activity more than 50% of that of the unaltered OCIF | |
| + indicates relative activity between 10% and 50% ± indicates relative activity less than 10%, or production level too low to determine the accurate biological activity | |

### vii) western blot analysis

Ten microliters of the final conditioned medium was used for western blot analysis. Ten microliters of the sample were mixed with 10 µl of SDS-PAGE sample buffer (0.5 M Tris-HCl, 20% glycerol, 4% SDS, 20 µg/ml bromo phenol blue, pH 6.8) boiled for 3 min. and subjected to a 10 % SDS polyacryl amide gel electrophoresis under non-reducing conditions. After the electrophoresis, the separated proteins were blotted to PVDF membrane (ProBlott^{R}, Perkin Elmer) using a semi-dry electroblotter (BIO-RAD). The membrane was incubated at 37°C with horseradish peroxidase labeled anti-OCIF antibodies for 2 hr. After the membrane was washed, protein bands which react with the labeled antibodies were detected using ECL system (Amersham). Two protein bands with approximate molecular masses of 60kD and 120kD were detected for the unaltered OCIF. On the other hand, almost exclusively 60kD protein band was detected for OCIF-C23S, OCIF-CL and OCIF CC. Protein bands with an approximate masses of 40kD-50kD and 30kD-40kD were the major ones for OCIF-CDD2 and OCIF-CDDI, respectively. These results indicate that Cys at 379 is responsible for the dimer formation, both the monomers and the dimers maintain the biological activity and a deletion of residues from Asp at 177 to Leu at 380 does not abolish the biological activity of OCIF (positions of the amino acid resare shown in SEQUENCE NO: 4).

### EXAMPLE 23

### Isolation of human genomic OCIF gene

### i) Screening of a human genomic library

An amplified human placenta genomic library in Lambda FIX II vector purchased from STRATAGENE was screened for the gene encoding human OCIF using the human OCIF cDNA as a probe. Essentially, screening was done according to the instruction manual supplied with the genomic library. The basic protocols described in Molecular Cloning: A Laboratory Manual also were employed to manipulate phage, E. coli, and DNA .

The library was titered, and 1x10⁶ pfu of phage was mixed with XL1-Blue MRA host E. coli cells and plated on 20 plates (9 cm x 13 cm) with 9 ml per plate of top agarose. The plates were incubated overnight at 37°C. Filter plaque lifts were prepared using Hybond-N nylon membranes (Amersham). The membranes were processed by denaturation in a solution containing 1.5 M NaCl and 0.5 M NaOH for 1 minute at room temperature. The membranes were then neutralized by placing successively for one minute each in 1 M Tris-HCl (pH7. 5) and a solution containing 1.5 M NaCl and 0. 5 M Tris-HCl (pH 7.5). The membranes were then transferred onto a filter paper wet with 2xSSC. Phage DNA was fixed on the membranes with 1200µ Joules of UV energy in STRATALINKER UV crosslinker 2400 (STRATAGENE) and the membranes were air dried. The membranes were immersed in Rapid Hybridization buffer (Amersham) and incubated for one hour at 65 °C before hybridization with ³²P-labeled cDNA probe in the same buffer overnight at 65°C. Screening probe was prepared by labeling the OCIF cDNA with ³²P using the Megaprime DNA labeling system (Amersham). Approximately, 5x10⁵cpm probe was used for each ml of hybridization buffer. After the hybridization, the membranes were rinsed in 2xSSC for five minutes at room temperature. The membranes were then washed four times, 20 minutes each time, in 0.5xSSC containing 0.1 % SDS at 65 °C. After the final wash, the membranes were dried and subjected to autoradiography at -80 °C with SUPER HR-H X-ray film (FUJI PFOTO FILM Co., Ltd. ) and an intensifying screen. Upon examination of the autoradiograms, six positive signals were detected. Agar plugs were picked from the regions corresponded to these signals for phage purification. Each agar plug was soaked overnight in 0.5 ml of SM buffer containing 1% chloroform to extract phage. Each extract containing phage was diluted 1000 fold with SM buffer and an aliquot of 1 ml or 20 ml was mixed with host E. coli described above. The mixture was plated on agar plates with top agarose as described above. The plates were incubated overnight at 37 °C, and filter lifts were prepared, prehybridized, hybridized, washed and autoradiographed as described above. This process of phage purification was applied to all six positive signals initially detected on the autoradiograms and was repeated until all phage plaques on agar plates hybridize with the cDNA probe. After purification, agar plugs of each phage isolate were soaked in SM buffer containing 1% chloroform and stored at 4 °C. Six individual phage isolates were designated λ0IF3, λ0IF8, λ0IF9, λ0IF11, λ0IF12 and λ0IF17, respectively.

### ii) Analysis of the genomic clones by restriction enzyme digestion and Southern blot hybridization

DNA was prepared from each phage isolate by the plate lysate method as described in Molecular Cloning: A Laboratory Manual. DNA prepared from each phage was digested with restriction enzymes and the fragments derived from the digestion were separated on agarose gels. The fragments were then transferred to nylon membranes and subjected to Southern blot hybridization using OCIF cDNA as a probe. The results of the analysis revealed that the six phage isolates are individual clones. Among these fragments derived from the restriction enzyme digestion, those fragments which hybridized with the OCIF cDNA probe were subcloned into plasmid vectors and subjected to the nucleotide sequence analysis as described below.

### iii) Subcloning restriction fragments derived from genomic clones into plasmid vectors and determination of the nucleotide sequence.

λ0IF8 DNA was digested with restriction enzymes EcoRI and NotI, and the DNA fragments derived these from were separated on a 0.7% agarose gel. The 5.8 kilobase pairs (kb). EcoRI/NotI fragment was extracted from the gel using QIAEX II Gel Extraction Kit (QIAGEN) according to the procedure recommended by the manufacturer. The 5.8 kb EcoRI/NotI fragment was ligated with pBluescript II SK+ vector (STRATAGENE) which had been linearized with restriction enzymes EcoRI and NotI, using Ready-To-Go T4 DNA Ligase (Pharmacia) according to the procedure recommended by the manufacturer. Competent DH5 α E. coli cells (Amersham) were transformed with the recombinant plasmid and transformants were selected on L-plates containing 50 µg/ml of ampicillin. A clone harboring the recombinant plasmid containing the 5.8 kb EcoRI/NotI fragment was isolated and this plasmid was termed pBSGB-5.8. pBSG8-5.8 was digested with HindIII and 0.9 kb of DNA fragment derived from this digestion was isolated in the same manner as described above. This 0.9 kb fragment was then cloned in pBluescript II SK- at the HindIII site as described above. This recombinant plasmid containing 0.9 kb HindIII fragment was denoted pBS8H0.9.

λ 0IF11 DNA was digested with EcoRI and 6 kb, 3.6 kb, 2.6 kb EcoRI fragments were isolated in the same manner as described above and cloned in pBluescript II SK+ vector at the EcoRI site as described above. These recombinant plasmids were termed pBSG11-6, pBSG11-3.6, and pBSG11-2.6, respectively. pBSG11-6 was digested with HindIII and the digest was applied on a 0.7 % agarose gel. Three fragments, 2.2 kb, 1. 1 kb, and 1.05 kb in length, were extracted from the gel and cloned independently in pBluescript II SK- vector at the HindIII site in the same manner as described above. These recombinant plasmids were termed pBS6H2.2, pBS6 H1.1 and pBS6H1.05, respectively.

The nucleotide sequence of the cloned genomic DNA was determined using ABI Dyedeoxy Terminator Cycle Sequencing Ready Reaction Kit (PERKIN ELMER) and 373A DNA Sequencing system (Applied Biosystems). Plasmids pBSG8-5.8, pBS8H0.9, pBSG11-6, pBSG11-3.6, pBSG11-2.6, pBS6H2.2, pBS6H 1. and pBS6H1.05 were prepared according to the alkaline-SDS procedure as described in Molecular Cloning: A Laboratory Manual and used as templates for the DNA sequence analysis. Nucleotide sequence of the human OCIF gene was presented in Sequence No 104 and Sequence No 105. The nucleotide sequence of the DNA, between exon 1 and exon 2 was not entirely determined. There is a stretch of approximately 17 kb of nucleotides between the sequences given in sequence No. 104 and sequence No. 105.

### EXAMPLE 24

### Quantitation of OCIF by EIA

### i) Preparation of anti-OCIF antibody

Male JW rabbits (Kitayama LABES Co.,LTD) weighing 2.5-3.0 kg were used for immunization for preparing antisera. Three male JW rabbits (Kitayama LABES Co., LTD) weighing 2.5-3.0 kg were used for immunization. For immunization, emulsion was prepared by mixing an equal volume of r0CIF (200 µg/ml) and complete Freund's adjuvant (Difco, Cat. 0638-60-7). The rabbits were immunized subcutaneously six times at the interval of one week with 1 ml of emulsion per injection. The rabbits were injected six times at the interval of seven days subcutaneously. Whole blood was obtained ten days after the final immunization and serum was separated. Antibody was purified from serum as follows. Antiserum was diluted two-fold with PBS. After adding ammonium sulfate at a final concentration of 40 w/v %, antiserum was allowed to stand at 4 °C for 1 hr.. Precipitate obtained by centrifugation at 8000 x g for 20 min. was dissolved in a small volume of PBS and was dialyzed against PBS. The resulting solution was loaded onto a Protein G-Sepharose column (Pharmacia). After washing with PBS, absorbed immunoglobulin G was eluted with 0.1 M glycine-HCL buffer (pH 3.0). Elutes were neutralized with 1.5 M Tris-HCL buffer (pH 8.7) immediately and were dialyzed against PBS. Protein concentration was determined by absorbance at 280nm (E¹³ 13.5).

Horseradish peroxidase labeled antibody was prepared using ImmunoPure Maleimide Activated Horseradish Peroxidase Kit (Pierce, Cat.31494). Briefly, one mg of IgG was incubated with 80 ug of N-succinimidyl-S-acetylthioacetate for 30 min. After deacetylation with 5 mg of hydroxylamine HCl, modified IgG was separeted by polyacrylamide desalting column. Protein pool mixed with one mg of maleimide activated horseradish peroxidase was incubated at room temperature for 1 hr.

### ii) Quantitation of OCIF by sandwich EIA

Microtiter plates (Nunc MaxiSorp Immunoplate) were coated with rabbit anti-OCIF IgG by incubating 0.2 ug in 100 ul of 50 mM sodium bicarbonate buffer pH 9.6 at 4C overnight. After blocking the plates by incubating for 1 hour at 37°C with 300 ul of 25% BlockAce/PBS (Snow Brand Milk Products ), 100ul of samples were incubated for 2 hours at room temperature. After washing the plates three times with PBST (PBS containing 0.05% Tween20), 100 ul of 1:10000 diluted horseradish peroxidase labeled anti-OCIF IgG was added and incubated for 2 hours at room temperture. The amount of OCIF was determined by incubation with 100 ul of a substrate solution (TMB, ScyTek Lab.,Cat.TM4999) and measurement of the absorbance at 450 nm using an ImmunoReader (Nunc NJ2000). Purified recombinant OCIF was used as a standard protein and a typical standared curve was shown in Fig. 13.

### EXAMPLE 25

### Anti-OCIF monoclonal antibody

### i) Preparation of hybridoma producing anti-OCIF monoclonal antibody.

OCIF was purified to homogeneity from culture medium of human fibroblasts, IMR-90 by the purification method described in Eample 11. Purified OCIF was dissolved in PBS at a concentration of 10 *µ*g/100 µl. BALB/c mice were immunized by administrating this solution intraperitoneally three times every two weeks. In the first and the second immunizations, the emulsion composed of an equal volume of OCIF and Freund's complete adjuvant was administered. Three days after the final administration, the spleen was taken out, lymphocytes were isolated and fused with mouse myeloma p3x63-Ag8.653 cells according to the conventinal method using polyethyleneglycol. Then the fused cells were cultured in HAT medium to select hybridoma. Subsequently, to check whether the selected hybridomas produce anti-OCIF antibody, anti-OCIF antibody in each culture medium of hybridomas was determined by solid phase ELISA which was prepared by coating each well in 96-well immunoplates (Nunc) with 100 *µ*l of purified OCIF (10 µg/ml in 0.1 M NaHCO₃) and by blocking each well with 50% BlockAce (Snow Brand Milk Products Co. Ltd.). The hybridoma clones secreting anti-OCIF antibody were established by cloning 3 - 5 times by limit dilution and by screening using the above solid phase ELISA. Among thus obtained hybridoma clones, several hybridoma clones with high production of anti-OCIF antibody were selected.

### ii) Production of anti-OCIF monoclonal antibodies.

Each hybridoma clone secreting anti-OCIF antibody, which was obtained in EXAMPLE 25-i), was transplanted intraperitoneally to mice given Pristane (Aldrich) at a cell density of 1 x 10⁶ cells/mouse. The accumulated ascites was collected 10 - 14 days after the transplantation and the ascites containing anti-OCIF specific monoclonal antibody of the present invention was obtained. Purified antibodies were obtained by Affigel protein A Sepharose

chromatography (BioRad) according to the maufacturer' s manual. That is, the ascites was diluted with equal volume of a binding buffer (BioRad) and applied to protein A column. The column was washed with a sufficient volume of the binding buffer and eluted with an elution buffer (BioRad). After neutralizing, the obtained eluate was dialyzed in water and subsequently lyophilized. The purity of the obtained antibody was analyzed by SDS/PAGE and a homogenous band with a molecular weight of about 150,000 was detected.

### iii) Selection of monoclonal antibody having high affinity to OCIF

Each antibody obtained in EXAMPLE 25-ii) was dissolved in PBS and the concentration of protein in the solution was determined by the method of Lowry. Each antibody solution with the same concentration was prepared and then serially diluted with PBS. Monoclonal antibodies, which can recognize 0CIF even at highly diluted solution, were selected by solid phase ELISA described in EXAMPLE 25-ii). Thus three monoclonal antibodies A1G5, E3H8 and D2F4 can be selected.

### iv) Determination of class and subclass of antibodies

The class and subclass of the antibodies of the present invention obtained in EXAMPLE 25-iii) were analyzed using an immunoglobulin class and subclass analysis kit (Amersham). The procedure was carried out according to the protocol disclosed in the directions. The results were shown in Table 15. The antibodies of the present invention, E3H8, A1G5 and D2F4 belong to IgG₁, IgG₂ₐ and IgG_{2b}, respectively.

**Table 15**

| Analysis of class and subclass of the antibodies in the present invention. | | | | | | | |
|---|---|---|---|---|---|---|---|
| Antibody | IgG₁ | IgG₂ₐ | IgG_{2b} | IgG₃ | IgA | IgM | κ |
| A1G5 | - | + | - | - | - | - | + |
| E3H8 | + | - | - | - | - | - | + |
| D2F4 | - | - | + | - | - | - | + |

### v) Determination of OCIF by ELISA

Three kinds of monoclonal antibodies, A1G5, E3H8 and D2F4, which were obtained in EXAMPLE 25-iv), were used as solid phase antibodies and enzyme-labeled antibodies, respectively. Sandwich ELISA was constructed by each combination of solid phase antibody and labeled antibody. The labeled antibody was prepared using Immuno Pure Maleimide Activated Horseradish Peroxidase Kit (Pierce, Cat. No. 31494). Each monoclonal antibody was dissolved in 0. 1 M NaHCO₃ at a concentration of 10 µg/ml, and 100 µl of the solution was added to each well in 96-well immunoplates (Nunc, MaxiSorp Cat. No. 442404) followed by allowing to stand at room temperature overnight. Subsequently, each well in the plates was blocked with 50% Blockace (Snow Brand Milk Products, Co., Ltd.) at room temperature for 50 minutes, and then was washed three times with PBS containing 0.1% Tween 20 (washing buffer).

A series of concentrations of OCIF was prepared by diluting OCIF with 1st reaction buffer (0.2 M Tris-HCl bufer, pH 7.4, containing 40% Blockace and 0.1% Tween 20). Each well in 96-well immunoplates was filled with 100 *µ*l of the prepared OCIF solution with each concentration, allowed to stand at 37°C for 3 hours, and subsequently washed three times with the washing buffer. For dilution of POD-labeled antibody, 2nd reaction buffer (0.1 M Tris-HCl buffer, pH 7.4, containing 25% Blockace and 0. 1% Tween 20) was used. POD-labeled antibody was diluted 400-fold with 2nd reaction buffer, and 100 *µ*l of the diluted solution was added to each well in the immunoplates. Each imunoplate was allowed to stand at 37 °CC for 2 hours, and subsequently washed three times with the washing buffer. After washing, 100 µl of a substrate solution (0.1 M citrate-phosphate buffer, pH 4.5, containing 0.4 mg/ml of o-phenylenediamine HCl and 0.006% H₂O₂) was added to each well in the immunoplates and the immunoplates were incubated at 37°C for 15 min. The enzyme reaction was terminated by adding 50 µl of 6 N H₂SO₄ to each well. The optical density of each well was determined at 492 nm using an immunoreader (ImmunoReader NJ 2000, Nunc).

Using three kinds of monoclonal antibody in the present invention, each combination of solid phase and POD-labeled antibodies leads to a accurate determination of OCIF. Each monoclonal antibody in the present invention was confirmed to recognize a different epitope of OCIF. A typical standard curve of OCIF using a combination of solid phase antibody, A1G5 and POD-labeled antibody, E3H8 was shown in Fig. 14.

### vi) Determination of OCIF in human serum

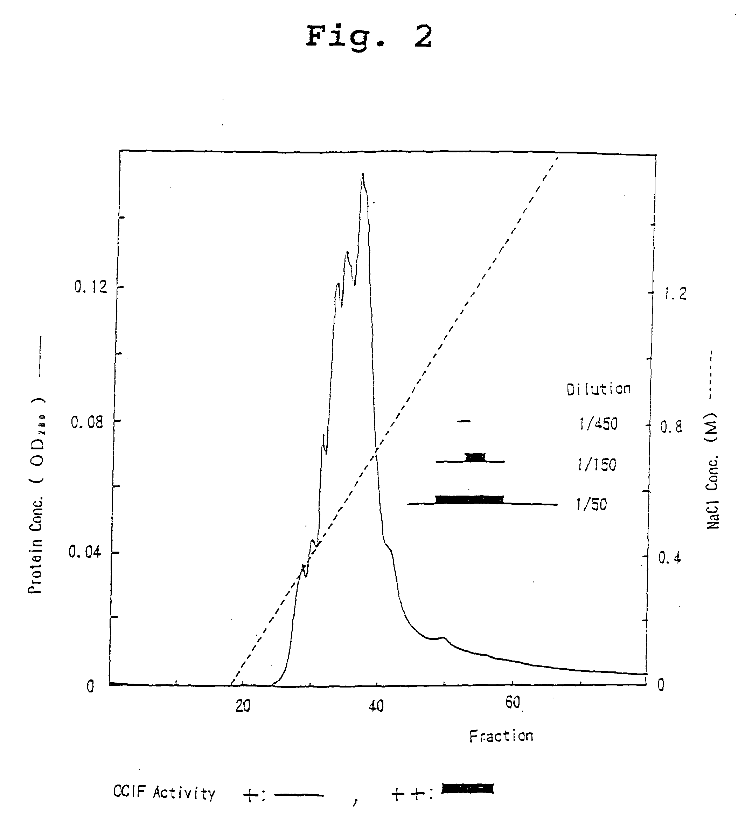

Concentration of OCIF in five samples of normal human serum was determined using an EIA system described in EXAMPLE 25-v). The immunoplates were coated with A1G5 as described in EXAMPLE 25-v), and 50 µl of 1st. reaction buffer was added to each well in the immunoplates. Subsequently, 50 µl of each human serum was added to each well in the immunoplates. The immnuoplates were incubated at 37°C for 3 hours and then washed three times with the washing buffer. After washing, each well in the immunoplates was filled with 100 µl of P0D-E3H8 antibody diluted 400-fold with 2nd. reaction buffer and incubated at 37°C for 2 hours. After washing the immunoplates three times with the washing buffer, 100 µl 1 of the substrate solution described in EXAMPLE 25-v) was added to each well and incubated at 37°C for 15 min. The enzyme reaction was terminated by adding 50 µl of 6 N H₂SO₄ to each well in the immunoplates. The optical density of each well was determined at 492 nm using an immunoreader (ImmunoReader NJ 2000, Nunc).
1st. reaction buffer containing the known amount of OCIF was treated in the same way and a standard curve of OCIF as shown in fig. 2 was obtained. Using the standard curve of OCIF, the amount of OCIF in human serum sample was determined. The results were shown in Table 14.

**Table 14**

| The amount of OCIF in normal human serum | |
|---|---|
| Serum Sample | OCIF Concentration (ng/ml) |
| 1 | 5.0 |
| 2 | 2.0 |
| 3 | 1.0 |
| 4 | 3.0 |
| 5 | 1.5 |

### EXAMPLE 26

### Therapeutic effect on osteoporosis

### (1) Method

Male Fischer rats, 6 weeks-old, were subjected to denervation of left forelimb. These rats were assigned to four groups(10 rats/group) and treated as follows ; group A, sham operated rats without administration ; group B, denervated rats with intravenous administration of vehicle ; group C, denervated rats administered OCIF intravenously at a dose of 5 *µ*g/kg twice a day ; group D, denervated rats administered OCIF intravenously at a dose of 50 *µ*g/kg twice a day. After denervation, OCIF was administered daily for 14 days. After 2 weeks treatment, the animals were sacrificed and their forelimbs were dissected. Thereafter bones were tested for mechanical strength.

### (2) Results

Decrease of bone strength was observed in the animals of control groups as compared to those animals of the normal groups while bone strength was increase in the groups of animal received 50 mg of OCIF per kg body weight.

### Industrial availability

The present invention provides both a novel protein which inhibits formation of osteoclasts and a efficient procedure to produce the protein. The protein of the present invention has an activity to inhibit formation of osteoclasts. The protein will be useful for the treatment of many diseases accompanying bone loss, such as osteoporosis, and as an antigen to be used for the immunological diagnosis of such diseases.

### Referring to the deposited the microorgainsm

### Name and Address of the Depositary Authority

| | |
|---|---|
| Name | National Institute of Bioscience and Human-Technology Agency of Industrial Science and Technology Ministry of International Trade and Industry |
| Address | 1-3, Higashi 1-chome, Tsukuba-shi, Ibaraki-ken 305, JAPAN |
| Deposited date | June 21, 1995 |

(It was transferred from Bikkoken No. P-14998, which was deposited on June 21, 1995. Transferred date: October 25, 1995)

Acession Number: FERM BP-5267
(1) GENERAL INFORMATION:
   (i) APPLICANT:
      (A) NAME: SNOW BRAND MILK PRODUCTS CO., LTD.
      (B) STREET: 1-1, NAEBOCHO 6-CHOME
      (C) CITY: HIGASHI-KU, SAPPORO-SHI
      (D) STATE: HOKKAIDO
      (E) COUNTRY: JP
      (F) POSTAL CODE (ZIP): NONE
   (ii) TITLE OF INVENTION: NOVEL PROTEINS AND METHODS FOR PRODUCING THE PROTEINS
   (iii) NUMBER OF SEQUENCES: 105
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 96902484.3
   (vi) PRIOR APPLICATION DATA:
      (A) APPLICATION NUMBER: JP 54977/95
      (B) FILING DATE: 20-FEB-1995
   (vi) PRIOR APPLICATION DATA:
      (A) APPLICATION NUMBER: JP 207508/95
      (B) FILING DATE: 21-JUL-1995
(2) INFORMATION FOR SEQ ID NO:1:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide (an internal amino acid sequence of the protein)
   (xi) SEQUENCE DESCRIPTION: SEQ ID NO:1:
   Xaa Tyr His Phe Pro Lys
     1               5
(2) INFORMATION FOR SEQ ID NO:2:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide (an internal amino acid sequence of the protein)
   (xi) SEQUENCE DESCRIPTION: SEQ ID NO:2:
   Xaa Gln His Ser Xaa Gln Glu Gln Thr Phe Gln Leu Xaa Lys
     1               5                   10
(2) INFORMATION FOR SEQUENCE ID NO: 3:
   (i) SEQUENCE CHARACTERISTICS:
      (A) LENGTH : 12 amino acids
      (B) TYPE : amino acid
      (D) TOPOLOGY : linear
   (ii) MOLECULE TYPE : peptide (an internal amino acid sequence of the protein)
   (xi) SEQUENCE DESCRIPTION :SEQ ID NO: 3:
   Xaa Ile Arg Phe Leu His Ser Phe Thr Met Tyr Lys
   1               5                   10
(2) INFORMATION FOR SEQUENCE ID NO: 4:
   (i) SEQUENCE CHARACTERISTICS:
      (A) LENGTH : 380 amino acids
      (B) TYPE : amino acid
      (D) TOPOLOGY : linear
   (ii) MOLECULE TYPE : protein (OCIF protein without signal peptide)
   (xi) SEQUENCE DESCRIPTION :SEQ ID NO:4:
   Glu Thr Phe Pro Pro Lys Tyr Leu His Tyr Asp Glu Glu Thr Ser
    1               5                  10                  15
   His Gln Leu Leu Cys Asp Lys Cys Pro Pro Gly Thr Tyr Leu Lys
                   20                  25                  30
   Gln His Cys Thr Ala Lys Trp Lys Thr Val Cys Ala Pro Cys Pro
                   35                  40                  45
   Asp His Tyr Tyr Thr Asp Ser Trp His Thr Ser Asp Glu Cys Leu
                   50                  55                  60
   Tyr Cys Ser Pro Val Cys Lys Glu Leu Gln Tyr Val Lys Gln Glu
                   65                  70                  75
   Cys Asn Arg Thr His Asn Arg Val Cys Glu Cys Lys Glu Gly Arg
                   80                  85                  90
   Tyr Leu Glu Ile Glu Phe Cys Leu Lys His Arg Ser Cys Pro Pro
                   95                  100                 105
   Gly Phe Gly Val Val Gln Ala Gly Thr Pro Glu Arg Asn Thr Val
                   110                 115                 120
   Cys Lys Arg Cys Pro Asp Gly Phe Phe Ser Asn Glu Thr Ser Ser
                   125                 130                 135
   Lys Ala Pro Cys Arg Lys His Thr Asn Cys Ser Val Phe Gly Leu
                   140                 145                 150
   Leu Leu Thr Gln Lys Gly Asn Ala Thr His Asp Asn Ile Cys Ser
                   155                 160                 165
   Gly Asn Ser Glu Ser Thr Gln Lys Cys Gly Ile Asp Val Thr Leu
                   170                 175                 180
   Cys Glu Glu Ala Phe Phe Arg Phe Ala Val Pro Thr Lys Phe Thr
                   185                 190                 195
   Pro Asn Trp Leu Ser Val Leu Val Asp Asn Leu Pro Gly Thr Lys
                   200                 205                 210
   Val Asn Ala Glu Ser Val Glu Arg Ile Lys Arg Gln His Ser Ser
                   215                 220                 225
   Gln Glu Gln Thr Phe Gln Leu Leu Lys Leu Trp Lys His Gln Asn
                   230                 235                 240
   Lys Asp Gln Asp Ile Val Lys Lys Ile Ile Gln Asp Ile Asp Leu
                   245                 250                 255
   Cys Glu Asn Ser Val Gln Arg His Ile Gly His Ala Asn Leu Thr
                   260                 265                 270
   Phe Glu Gln Leu Arg Ser Leu Met Glu Ser Leu Pro Gly Lys Lys
                   275                 280                 285
   Val Gly Ala Glu Asp Ile Glu Lys Thr Ile Lys Ala Cys Lys Pro
                   290                 295                 300
   Ser Asp Gln Ile Leu Lys Leu Leu Ser Leu Trp Arg Ile Lys Asn
                   305                 310                 315
   Gly Asp Gln Asp Thr Leu Lys Gly Leu Met His Ala Leu Lys His
                   320                 325                 330
   Ser Lys Thr Tyr His Phe Pro Lys Thr Val Thr Gln Ser Leu Lys
                   335                 340                 345
   Lys Thr Ile Arg Phe Leu His Ser Phe Thr Met Tyr Lys Leu Tyr
                   350                 355                 360
   Gln Lys Leu Phe Leu Glu Met Ile Gly Asn Gln Val Gln Ser Val
                   365                 370                 375
   Lys Ile Ser Cys Leu
                   380
(2) INFORMATION FOR SEQUENCE ID NO: 5:
   (i) SEQUENCE CHARACTERISTICS:
      (A) LENGTH : 401 amino acids
      (B) TYPE : amino acid
      (D) TOPOLOGY : linear
   (ii) MOLECULE TYPE : protein (OCIF protein with signal peptide)
   (xi) SEQUENCE DESCRIPTION : SEO ID NO: 5:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Cys Leu
   370                 375                 380
(2) INFORMATION FOR SEQUENCE ID NO: 6:
   (i) SEQUENCE CHARACTERISTICS:
      (A) LENGTH : 1206 base pairs
      (B) TYPE : nucleic acid
      (C) STRANDEDNESS . single
      (D) TOPOLOGY: linear
   (ii) MOLECULE TYPE : CDNA (OCIF)
   (xi) SEQUENCE DESCRIPTION :SEQ ID NO: 6:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                            1206
(2) INFORMATION FOR SEQUENCE ID NO: 7:
   (i) SEQUENCE CHARACTERISTICS:
      (A) LENGTH : 15 amino acids
      (B) TYPE : amino acid
      (D) TOPOLOGY : linear
   (ii) MOLECULE TYPE : peptide (a N-terminal amino acid sequence of the protein)
   (xi) SEQUENCE DESCRIPTION :SEQ ID NO:7:
   Glu Thr Phe Pro Pro Lys Tyr Leu His Tyr Asp Glu Glu Thr Ser
   1               5                   10                  15
(2) INFORMATION FOR SEQUENCE NO ID NO: 8:
   (i) SEQUENCE CHARACTERISTICS:
      (A) LENGTH : 1185 base pairs
      (B) TYPE : nucleic acid
      (C) STRANDEDNESS . single
      (D; TOPOLOGY : linear
   (ii) MOLECULE TYPE : cDNA (OCIF2)
   (xi) SEQUENCE DESCRIPTION :SEQ ID NO:8
(2) INFORMATION FOR SEQUENCE ID NO: 9:
   (i) SEQUENCE CHARACTERISTICS:
      (A) LENGTH : 394 amino acids
      (B) TYPE : amino acid
      (D) TOPOLOGY : linear
   (ii) MOLECULE TYPE : protein (OCIF2)
   (xi) SEQUENCE DESCRIPTION: SEQ ID NO: 9:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
        -5                  -1   1              5
   Tyr Asp Glu Glu Thr Ser His Gln Leu Leu Cys Asp Lys Cys Pro
    10                  15                  20
   Pro Gly Thr Tyr Leu Lys Gln His Cys Thr Ala Lys Trp Lys Thr
    25                  30                  35
   Val Cys Ala Pro Cys Pro Asp His Tyr Tyr Thr Asp Ser Trp His
    40                  45                  50
   Thr Ser Asp Glu Cys Leu Tyr Cys Ser Pro Val Cys Lys Glu Cys
    55                  60                  65
   Asn Arg Thr His Asn Arg Val Cys Glu Cys Lys Glu Gly Arg Tyr
    70                  75                  80
   Leu Glu Ile Glu Phe Cys Leu Lys His Arg Ser Cys Pro Pro Gly
    85                  90                  95
   Phe Gly Val Val Gln Ala Gly Thr Pro Glu Arg Asn Thr Val Cys
   100                 105                 110
   Lys Arg Cys Pro Asp Gly Phe Phe Ser Asn Glu Thr Ser Ser Lys
   115                 120                 125
   Ala Pro Cys Arg Lys His Thr Asn Cys Ser Val Phe Gly Leu Leu
   130                 135                 140
   Leu Thr Gln Lys Gly Asn Ala Thr His Asp Asn Ile Cys Ser Gly
   145                 150                 155
   Asn Ser Glu Ser Thr Gln Lys Cys Gly Ile Asp Val Thr Leu Cys
   160                 165                 170
   Glu Glu Ala Phe Phe Arg Phe Ala Val Pro Thr Lys Phe Thr Pro
   175                 180                 185
   Asn Trp Leu Ser Val Leu Val Asp Asn Leu Pro Gly Thr Lys Val
   190                 195                 200
   Asn Ala Glu Ser Val Glu Arg Ile Lys Arg Gln His Ser Ser Gln
   205                 210                 215
   Glu Gln Thr Phe Gln Leu Leu Lys Leu Trp Lys His Gln Asn Lys
   220                 225                 230
   Asp Gln Asp Ile Val Lys Lys Ile Ile Gln Asp Ile Asp Leu Cys
   235                 240                 245
   Glu Asn Ser Val Gln Arg His Ile Gly His Ala Asn Leu Thr Phe
   250                 255                 260
   Glu Gln Leu Arg Ser Leu Met Glu Ser Leu Pro Gly Lys Lys Val
   265                 270                 275
   Gly Ala Glu Asp Ile Glu Lys Thr Ile Lys Ala Cys Lys Pro Ser
   280                 285                 290
   Asp Gln Ile Leu Lys Leu Leu Ser Leu Trp Arg Ile Lys Asn Gly
   295                 300                 305
   Asp Gln Asp Thr Leu Lys Gly Leu Met His Ala Leu Lys His Ser
   310                 315                 320
   Lys Thr Tyr His Phe Pro Lys Thr Val Thr Gln Ser Leu Lys Lys
   325                 330                 335
   Thr Ile Arg Phe Leu His Ser Phe Thr Met Tyr Lys Leu Tyr Gln
   340                 345                 350
   Lys Leu Phe Leu Glu Met Ile Gly Asn Gln Val Gln Ser Val Lys
   355                 360                 365
   Ile Ser Cys Leu
   370         373
(2) INFORMATION FOR SEQUENCE ID NO: 10:
   (i) SEQUENCE CHARACTERISTICS:
      (A) LENGTH : 1089 base pairs
      (B) TYPE : nucleic acid
      (C) STRANDEDNESS : single
      (D) TOPOLOGY : linear
   (ii) MOLECULE TYPE : cDNA (0CIF3)
   (xi) SEQUENCE DESCRIPTION ID NO: 10:
   ATGAACAAGT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC AGTTTGTGGC GAATAAAAAA TGGCGACCAA  900
   GACACCTTGA AGGGCCTAAT GCACGCACTA AAGCACTCAA AGACGTACCA CTTTCCCAAA  960
   ACTGTCACTC AGAGTCTAAA GAAGACCATC AGGTTCCTTC ACAGCTTCAC AATGTACAAA 1020
   TTGTATCAGA AGTTATTTTT AGAAATGATA GGTAACCAGG TCCAATCAGT AAAAATAAGC 1080
   TGCTTATAA                                                        1089
(2) INFORMATION FOR SEQUENCE ID NO: 11:
   (i) SEQUENCE CHARACTERISTICS:
      (A) LENGTH : 362 amino acids
      (B) TYPE : amino acid
      (C) STRANDEDNESS : single
      (D) TOPOLOGY : linear
   (ii) MOLECULE TYPE : protein (OCIF3)
   (xi) SEQUENCE DESCRIPTION: SEQ ID NO: 11:
   Met Asn Lys Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
        -5              -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Ser Leu Trp Arg Ile Lys Asn Gly Asp Gln
   265                 270                 275
   Asp Thr Leu Lys Gly Leu Met His Ala Leu Lys His Ser Lys Thr
   280                 285                 290
   Tyr His Phe Pro Lys Thr Val Thr Gln Ser Leu Lys Lys Thr Ile
   295                 300                 305
   Arg Phe Leu His Ser Phe Thr Met Tyr Lys Leu Tyr Gln Lys Leu
   310                 315                 320
   Phe Leu Glu Met Ile Gly Asn Gln Val Gln Ser Val Lys Ile Ser
   325                 330                 335
   Cys Leu
   340 341
(2) INFORMATION FOR SEQUENCE ID NO: 12:
   (i) SEQUENCE CHARACTERISTICS:
      (A) LENGTH : 465 base pairs
      (B) TYPE : nucleic acid
      (C) STRANDEDNESS : single
      (D) TOPOLOGY : linear
   (ii) MOLECULE TYPE : cDNA (OCIF4)
   (xi) SEQUENCE DESCRIPTION :SEQ ID NO: 12:
   ATGAACAAGT TGCTGTGCTG CTCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GTACGTGTCA ATGTGCAGCA  420
   AAATTAATTA GGATCATGCA AAGTCAGATA GTTGTGACAG TTTAG                  465
(2) INFORMATION FOR SEQUENCE ID NO: 13:
   (i) SEQUENCE CHARACTERISTICS:
      (A) LENGTH :154 amino acids
      (B) TYPE : amino acid
      (C) STRANDEDNESS : single
      (D) TOPOLOGY : linear
   (ii) MOLECULE TYPE : protein (OCIF4)
   (xi) SEQUENCE DESCRIPTION: SEQ ID NO: 13:
(2) INFORMATION FOR SEQUENCE ID NO: 14:
   (i) SEQUENCE CHARACTERISTICS:
      (A) LENGTH : 438 base pairs
      (B) TYPE : nucleic acid
      (C) STRANDEDNESS : single
      (D) TOPOLOGY : linear
   MOLECULE TYPE : cDNA (OCIF5)
   (xi) SEQUENCE DESCRIPTION ID NO: 14:
   ATGAACAAGT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GATGCAGGAG AAGACCCAAG  420
   CCACAGATAT GTATCTGA                                                438
(2) INFORMATION FOR SEQUENCE ID NO: 15:
   (i) SEQUENCE CHARACTERISTICS:
      (A) LENGTH :145 amino acids
      (B) TYPE : amino acid
      (C) STRANDEDNESS : single
      (D) TOPOLOGY : linear
   (ii) MOLECULE TYPE : protein (OCIF5)
   (xi) SEQUENCE DESCRIPTION: ID NO: 15:
(2) INFORMATION FOR SEQUENCE ID NO: 16:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer T3)
   (xi) SEQUENCE DESCRIPTION: SEQ ID NO: 16:
   AATTAACCCT CACTAAAGGG                                              20
(2) INFORMATION FOR SEQUENCE ID NO: 17:
   (i) SEQUENCE CHARACTERISTICS:
      (A) LENGTH : 22 base pairs
      (B) TYPE: nucleic acid
      (C) STRANDEDNESS : single
      (D) TOPOLOGY : linear
   (ii) MOLECULE TYPE : synthetic DNA (primer T7)
   (xi) SEQUENCE DESCRIPTION: SEQ ID NO: 17:
   GTAATACGAC TCACTATAGG GC 22
(2) INFORMATION FOR SEQUENCE ID NO: 18:
   (i) SEQUENCE CHARACTERISTICS :
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF1)
   (xi) SEQUENCE DESCRIPTION :SEQ ID NO: 18:
   ACATCAAAAC AAAGACCAAG 20
(2) INFORMATION FOR SEQUENCE ID NO: 19:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF2)
   (xi) SEQUENCE DESCRIPTION :SEQ ID NO: 19:
   TCTTGGTCTT TGTTTTGATG 20
(2) INFORMATION FOR SEQUENCE ID NO: 20:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF3)
   (xi) SEQUENCE DESCRIPTION :SEQ ID NO: 20:
   TTATTCGCCA CAAACTGAGC 20
(2) INFORMATION FOR SEQUENCE ID NO: 21:
   (i) SEQUENCE CHARACTERISTICS:
      (A) LENGTH : 20 base pairs
      (B) TYPE : nucleic acid
      (C) STRANDEDBNESS : single
      (D) TOPOLOGY : linear
   (ii) MOLECULE TYPE : synthetic DNA (primer IF4)
   (xi) SEQUENCE DESCRIPTION :SEQ ID NO: 21:
   TTGTGAAGCT GTGAAGGAAC 20
(2) INFORMATION FOR SEQUENCE ID NO: 22:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF5)
   (xi) SEQUENCE DESCRIPTION :SEQ ID NO: 22:
   GCTCAGTTTG TGGCGAATAA 20
(2) INFORMATION FOR SEQUENCE ID NO: 23:
   (i) SEQUENCE CHARACTERISTICS:
      (A) LENGTH ; 20 base pairs
      (B) TYPE : nucleic acid
      (C) STRANDEDNESS : single
      (D) TOPOLOGY : linear
   (ii) MOLECULE TYPE : synthetic DNA (primer IF6)
   (xi) SEOUENCE DESCRIPTION :SEO ID NO: 23:
(2) INFORMATION FOR SEQUENCE ID NO: 24:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF7)
   (xi) SEQUENCE DESCRIPTION :SEQ ID NO: 24:
   AATGAACAAC TTGCTGTGCT 20
(2) INFORMATION FOR SEQUENCE ID NO: 25:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF8)
   (xi) SEQUENCE DESCRIPTION :SEQ ID NO: 25:
   TGACAAATGT CCTCCTGGTA 20
(2) INFORMATION FOR SEQUENCE ID NO: 26:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (it) MOLECULE TYPE : synthetic DNA (primer IF9)
   (xi) SEQUENCE DESCRIPTION :SEQ ID NO: 26:
   AGGTAGGTAC CAGGAGGACA 20
(2) INFORMATION FOR SEQUENCE ID NO: 27:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF10)
   (xi) SEQUENCE DESCRIPTION :SEQ ID NO: 27:
   GAGCTGCCCT CCTGGATTTG 20
(2) INFORMATION FOR SEQUENCE ID NO: 28:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF11)
   (xi) SEQUENCE DESCRIPTION :SEQ ID NO: 28:
   CAAACTGTAT TTCGCTCTGG 20
(2) INFORMATION FOR SEQUENCE ID NO: 29:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF12)
   (xi) SEQUENCE DESCRIPTION :SEQ ID NO: 29:
   GTGTGAGGAG GCATTCTTCA                                                    20
(2) INFORMATION FOR SEQUENCE ID NO: 30:
   (i) SEQUENCE CHARACTERISTICS:
      (A) LENGTH : 32 base pairs
      (B) TYPE : nucleic acid
      (C) STRANDEDNESS : single
      (D) TOPOLOGY : linear
   (ii) MOLECULE TYPE : synthetic DNA (primer C19SF)
   (xi) SEQUENCE DESCRIPTION :SEQ ID NO: 30:
   GAATCAACTC AAAAAAGTGG AATAGATGTT AC                                      32
(2) INFORMATION FOR SEQUENCE ID NO: 31:
   (i) SEQUENCE CHARACTERISTICS:
      (A) LENGTH : 32 base pairs
      (B) TYPE : nucleic acid
      (C) STRANDEDNESS : single
      (D) TOPOLOGY : linear
   (ii) MOLECULE TYPE : synthetic DNA (primer C19SR)
   (xi) SEQUENCE DESCRIPTION :SEQ ID NO: 31:
   GTAACATCTA TTCCACTTTT TTGAGTTGAT TC                                      32
(2) INFORMATION FOR SEQUENCE ID NO: 32:
   (i) SEQUENCE CHARACTERISTICS:
      (A) LENGTH : 30 base pairs
      (B) TYPE : nucleic acid
      (C) STRANDEDNESS : single
      (D) TOPOLOGY : linear
   (ii) MOLECULE TYPE : synthetic DNA (primer C20SF)
   (xi) SEQUENCE DESCRIPTION :SEQ ID NO: 32:
   ATAGATGTTA CCCTGAGTGA GGAGGCATTC                                         30
(2) INFORMATION FOR SEQUENCE ID NO: 33:
   (i) SEQUENCE CHARACTERISTICS:
      (A) LENGTH : 30 base pairs
      (B) TYPE : nucleic acid
      (C) STRANDEDNESS : single
      (D) TOPOLOGY: linear
   (ii) MOLECULE TYPE : synthetic DNA (primer C20SR)
   (xi) SEQUENCE DESCRIPTION :SEQ ID NO: 33:
   GAATGCCTCC TCACTCAGGG TAACATCTAT 30
(2) INFORMATION FOR SEQUENCE ID NO: 34:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C21SF)
   (xi) SEQUENCE DESCRIPTION :SEQ ID NO: 34:
   CAAGATATTG ACCTCAGTGA AAACAGCGTG C 31
(2) INFORMATION FOR SEQUENCE ID NO: 35:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C21SR)
   (xi) SEQUENCE DESCRIPTION :SEQ ID NO: 35:
   GCACGCTGTT TTCACTGAGG GCAATATCTT G 31
(2) INFORMATION FOR SEQUENCE ID NO: 36:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C22SF)
   (xi) SEQUENCE DESCRIPTION :SEQ ID NO: 36:
   AAAACAATAA AGGCAAGCAA ACCCAGTGAC C 31
(2) INFORMATION FOR SEQUENCE ID NO: 37:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C22SR)
   (xi) SEQUENCE DESCRIPTION :SEQ ID NO: 37:
   GGTCACTGGG TTTGCTTGCC TTTATTGTTT T 31
(2) INFORMATION FOR SEQUENCE ID NO: 38:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C23SF)
   (xi) SEQUENCE DESCRIPTION :SEQ ID NO: 38:
   TCAGTAAAAA TAAGCAGCTT ATAACTGGCC A 31
(2) INFORMATION FOR SEQUENCE ID NO: 39:
   (i) SEQUENCE CHARACTERISTICS:
      (A) LENGTH : 31 base pairs
      (B) TYPE : nucleic acid
      (C) STRANDEDNESS : single
      (D) TOPOLOGY : linear
   (ii) MOLECULE TYPE : synthetic DNA (primer C23SR)
   (xi) SEQUENCE DESCRIPTION :SEQ ID NO: 39:
   TGGCCAGTTA TAAGCTGCTT ATTTTTACTG A 31
(2) INFORMATION FOR SEQUENCE ID NO: 40:
   (i) SEQUENCE CHARACTERISTICS:
      (A) LENGTH : 22 base pairs
      (B) TYPE : nucleic acid
      (C) STRANDEDNESS : single
      (D) TOPOLOGY : linear
   (ii) MOLECULE TYPE : synthetic DNA (primer IF 14)
   (xi) SEQUENCE DESCRIPTION :SEQ ID NO: 40:
   TTGGGGTTTA TTGGAGGAGA TG 22
(2) INFORMATION FOR SEQUENCE ID NO: 41:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR1F)
   (xi) SEQUENCE DESCRIPTION :SEQ ID NO: 41:
   ACCACCCAGG AACCTTGCCC TGACCACTAC TACACA                36
(2) INFORMATION FOR SEQUENCE ID NO: 42:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR1R)
   (xi) SEQUENCE DESCRIPTION :SEQ ID NO: 42:
   GTCAGGGCAA GGTTCCTGGG TGGTCCACTT AATGGA                36
(2) INFORMATION FOR SEQUENCE ID NO: 43:
   (i) SEQUENCE CHARACTERISTICS:
      (A) LENGTH : 36 base pairs
      (B) TYPE : nucleic acid
      (C) STRAD1DEDNESS : single
      (D) TOPOLOGY : linear
   (ii) MOLECULE TYPE : synthetic DNA (primer DCR2F)
   (xi) SEQUENCE DESCRIPTION :SEQ ID NO: 43:
   ACCGTGTGCG CCGAATGCAA GGAAGGGCGC TACCTT                36
(2) INFORMATION FOR SEQUENCE ID NO: 44:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR2R)
   (xi) SEQUENCE DESCRIPTION :SEQ ID NO: 44:
   TTCCTTGCAT TCGGCGCACA CGGTCTTCCA CTTTGC 36
(2) INFORMATION FOR SEQUENCE ID NO: 45:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR3F)
   (xi) SEQUENCE DESCRIPTION : SEQ ID NO: 45:
   AACCGCGTGT GCAGATGTCC AGATGGGTTC TTCTCA 36
(2) INFORMATION FOR SEQUENCE ID NO: 46:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR3R)
   (xi) SEQUENCE DESCRIPTION :SEQ ID NO: 46:
   ATCTGGACAT CTGCACACGC GGTTGTGGGT GCGATT 36
(2) INFORMATION FOR SEQUENCE ID NO: 47:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR4F)
   (xi) SEQUENCE DESCRIPTION :SEQ ID NO: 47:
   ACAGTTTGCA AATCCGGAAA CAGTGAATCA ACTCAA 36
(2) INFORMATION FOR SEQUENCE ID NO: 48:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DCR4R)
   (xi) SEQUENCE DESCRIPTION :SEQ ID NO: 48:
   ACTGTTTCCG GATTTGCAAA CTGTATTTCG CTCTGG 36
(2) INFORMATION FOR SEQUENCE ID NO: 49:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DDD1F)
   (xi) SEQUENCE DESCRIPTION :SEQ ID NO: 49:
   AATGTGGAAT AGATATTGAC CTCTGTGAAA ACAGCG 36
(2) INFORMATION FOR SEQUENCE ID NO: 50:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DDD1R)
   (xi) SEQUENCE DESCRIPTION :SEQ ID NO: 50:
   AGAGGTCAAT ATCTATTCCA CATTTTTGAG TTGATT 36
(2) INFORMATION FOR SEQUENCE ID NO: 51:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DDD2F)
   (xi) SEQUENCE DESCRIPTION :SEQ ID NO: 51:
   AGATCATCCA AGACGCACTA AAGCACTCAA AGACGT 36
(2) INFORMATION FOR SEQUENCE ID NO: 52:
   (i) SEQUENCE CHARACTERISTICS:
      (A) LENGTH : 36 base pairs
      (B) TYPE : nucleic acid
      (C) STRANDEDNESS : single
      (D) TOPOLOGY : linear
   (ii) MOLECULE TYPE : synthetic DNA (primer DDD2R)
   (xi) SEQUENCE DESCRIPTION :SEQ ID NO: 52:
   GCTTTAGTGC GTCTTGGATG ATCTTCTTGA CTATAT 36
(2) INFORMATION FOR SEQUENCE ID NO: 53:
   (i) SEQUENCE CHARACTERISTICS:
      (A) LENGTH : 29 base pairs
      (B) TYPE : nucleic acid
      (C) STRANDEDNESS : single
      (D) TOPOLOGY : linear
   (ii) MOLECULE TYPE : synthetic DNA (primer XhoI F)
   (xi) SEQUENCE DESCRIPTION :SEO ID NO: 53:
   GGCTCGAGCG CCCAGCCGCC GCCTCCAAG 29
(2) INFORMATION FOR SEQUENCE ID NO: 54:
   (i) SEQUENCE CHARACTERISTICS:
      (A) LENGTH : 20 base pairs
      (B) TYPE : nucleic acid
      (C) STRANDEDNESS : single
      (D) TOPOLOGY : linear
   (ii) MOLECULE TYPE : synthetic DNA (primer IF 16)
   (xi) SEQUENCE DESCRIPTION :SEQ ID NO: 54:
   TTTGAGTGCT TTAGTGCGTG 20
(2) INFORMATION FOR SEQUENCE ID NO: 55:
   (i) SEQUENCE CHARACTERISTICS:
      (A) LENGTH : 30 base pairs
      (B) TYPE : nucleic acid
      (C) STRANDEDNESS : single
      (D) TOPOLOGY : linear
   (ii) MOLECULE TYPE : synthetic DNA (primer CL F)
   (xi) SEQUENCE DESCRIPTION :SEQ ID NO: 55:
   TCAGTAAAAA TAAGCTAACT GGAAATGGCC 30
(2) INFORMATION FOR SEQUENCE ID NO: 56:
   (i) SEQUENCE CHARACTERISTICS:
      (A) LENGTH : 30 base pairs
      (B) TYPE : nucleic acid
      (C) STRANDEDNESS : single
      (D) TOPOLOGY : linear
   (ii) MOLECULE TYPE : synthetic DNA (primer CL R)
   (xi) SEQUENCE DESCRIPTION :SEQ ID NO: 56:
   GGCCATTTCC AGTTAGCTTA TTTTTACTGA 30
(2) INFORMATION FOR SEQUENCE ID NO: 57:
   (i) SEQUENCE CHARACTERISTICS:
      (A) LENGTH : 29 base pairs
      (B) TYPE : nucleic acid
      (C) STRANDEDNESS : single
      (D) TOPOLOGY : linear
   (ii) MOLECULE TYPE : synthetic DNA (primer CC R)
   (xi) SEQUENCE DESCRIPTION :SEQ ID NO: 57:
   CCGGATCCTC AGTGCTTTAG TGCGTGCAT 29
(2) INFORMATION FOR SEQUENCE ID NO: 58:
   (i) SEQUENCE CHARACTERISTICS:
      (A) LENGTH : 29 base pairs
      (B) TYPE : nucleic acid
      (C) STRANDEDNESS : single
      (D) TOPOLOGY : linear
   (ii) MOLECULE TYPE : synthetic DNA (primer CCD2 R)
   (xi) SEQUENCE DESCRIPTION :SEQ ID NO: 58:
   CCGGATCCTC ATTGGATGAT CTTCTTGAC 29
(2) INFORMATION FOR SEQUENCE ID NO: 59:
   (i) SEQUENCE CHARACTERISTICS:
      (A) LENGTH : 29 base pairs
      (B) TYPE : nucleic acid
      (C) STRANDEDNESS : single
      (D) TOPOLOGY: linear
   (ii) MOLECULE TYPE : synthetic DNA (primer CCD1 R)
   (xi) SEQUENCE DESCRIPTION :SEQ ID NO: 59:
   CCGGATCCTC ATATTCCACA TTTTTGAGT 29
(2) INFORMATION FOR SEQUENCE ID NO: 60:
   (i) SEQUENCE CHARACTERISTICS:
      (A) LENGTH : 29 base pairs
      (B) TYPE : nucleic acid
      (C) STRANDEDNESS : single
      (D) TOPOLOGY : linear
   (ii) MOLECULE TYPE : synthetic DNA (primer CCR4 R)
   (xi) SEQUENCE DESCRIPTION :SEQ ID NO: 60:
   CCGGATCCTC ATTTGCAAAC TGTATTTCG                                29
(2) INFORMATION FOR SEQUENCE ID NO: 61:
   (i) SEQUENCE CHARACTERISTICS:
      (A) LENGTH : 29 base pairs
      (B) TYPE : nucleic acid
      (C) STRANDEDNESS : single
      (D) TOPOLOGY : linear
   (ii) MOLECULE TYPE : synthetic DNA (primer CCR3 R)
   (xi) SEQUENCE DESCRIPTION :SEQ ID NO: 61:
   CCGGATCCTC ATTCGCACAC GCGGTTGTG                                29
(2) INFORMATION FOR SEQUENCE ID NO: 62:
   (i) SEQUENCE CHARACTERISTICS:
      (A) LENGTH : 401 amino acids
      (B) TYPE : amino acid
      (C) STRANDEDNESS : single
      (D) TOPOLOGY : linear
   (ii) MOLECULE TYPE : Protein (0CIF-C19S)
   (xi) SEQUENCE DESCRIPTION :SEQ ID NO: 62:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1                  5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Ser
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Cys Leu
   370                 375                 380
(2) INFORMATION FOR SEQUENCE ID NO: 63:
   (i) SEQUENCE CHARACTERISTICS:
      (A) LENGTH : 401 amino acids
      (B) TYPE : amino acid
      (C) STRANDEDNESS : single
      (D) TOPOLOGY : linear
   (ii) MOLECULE TYPE : Protein (OCIF-C20S)
   (xi) SEQUENCE DESCRIPTION :SEQ ID NO: 63:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Ser Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Cys Leu
   370                 375                 380
(2) INFORMATION FOR SEQUENCE ID NO: 64:
   (i) SEQUENCE CHARACTERISTICS:
      (A) LENGTH : 401 amino acids
      (B) TYPE : amino acid
      (C) STRANDEDNESS : single
      (D) TOPOLOGY : linear
   (ii) MOLECULE TYPE : Protein (OCIF-C21S)
   (xi) SEQUENCE DESCRIPTION :SEQ ID NO: 64:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1                5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Ser Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Cys Leu
   370                 375                 380
(2) INFORMATION FOR SEQUENCE ID NO: 65:
   (i) SEQUENCE CHARACTERISTICS:
      (A)LENGTH : 401 amino acids
      (B) TYPE : amino acid
      (C) STRANDEDNESS : single
      (D) TOPOLOGY : linear
   (ii) MOLECULE TYPE : Protein (OCIF-C22S)
   (xi) SEQUENCE DESCRIPTION :SEQ ID NO: 65:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Ser Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Cys Leu
   370                 375                 380
(2) INFORMATION FOR SEQUENCE ID NO: 66:
   (i) SEQUENCE CHARACTERISTICS:
      (A) LENGTH : 401 amino acids
      (B) TYPE : amino acid
      (C) STRANDEDNESS : single
      (D) TOPOLOGY : linear
   (ii) MOLECULE TYPE : Protein (OCIF-C23S)
   (xi) SEQUENCE DESCRIPTION :SEQ ID NO: 66:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1                5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser Ser Leu
   370                 375                 380
(2) INFORMATION FOR SEQUENCE ID NO: 67:
   (i) SEQUENCE CHARACTERISTICS:
      (A) LENGTH : 360 amino acids
      (B) TYPE : amino acid
      (C) STRANDEDNESS : single
      (D) TOPOLOGY : linear
   (ii) MOLECULE TYPE : Protein (OCIF-DCR1)
   (xi) SEQUENCE DESCRIPTION :SEQ ID NO: 67:
   Met Asn Asn Leu Leu Cys Cys Ala Leu Val Phe Leu Asp Ile Ser
       -20                 -15                 -10
   Ile Lys Trp Thr Thr Gln Glu Pro Cys Pro Asp His Tyr Tyr Thr
       -5              -1   1                   5
   Asp Ser Trp His Thr Ser Asp Glu Cys Leu Tyr Cys Ser Pro Val
   10                  15                  20
   Cys Lys Glu Leu Gln Tyr Val Lys Gln Glu Cys Asn Arg Thr His
   25                  30                  35
   Asn Arg Val Cys Glu Cys Lys Glu Gly Arg Tyr Leu Glu Ile Glu
   40                  45                  50
   Phe Cys Leu Lys His Arg Ser Cys Pro Pro Gly Phe Gly Val Val
   55                  60                  65
   Gln Ala Gly Thr Pro Glu Arg Asn Thr Val Cys Lys Arg Cys Pro
   70                  75                  80
   Asp Gly Phe Phe Ser Asn Glu Thr Ser Ser Lys Ala Pro Cys Arg
   85                  90                  95
   Lys His Thr Asn Cys Ser Val Phe Gly Leu Leu Leu Thr Gln Lys
   100                 105                 110
   Gly Asn Ala Thr His Asp Asn Ile Cys Ser Gly Asn Ser Glu Ser
   115                 120                 125
   Thr Gln Lys Cys Gly Ile Asp Val Thr Leu Cys Glu Glu Ala Phe
   130                 135                 140
   Phe Arg Phe Ala Val Pro Thr Lys Phe Thr Pro Asn Trp Leu Ser
   145                 150                 155
   Val Leu Val Asp Asn Leu Pro Gly Thr Lys Val Asn Ala Glu Ser
   160                 165                 170
   Val Glu Arg Ile Lys Arg Gln His Ser Ser Gln Glu Gln Thr Phe
   175                 180                 185
   Gln Leu Leu Lys Leu Trp Lys His Gln Asn Lys Asp Gln Asp Ile
   190                 195                 200
   Val Lys Lys Ile Ile Gln Asp Ile Asp Leu Cys Glu Asn Ser Val
   205                 210                 215
   Gln Arg His Ile Gly His Ala Asn Leu Thr Phe Glu Gln Leu Arg
   220                 225                 230
   Ser Leu Met Glu Ser Leu Pro Gly Lys Lys Val Gly Ala Glu Asp
   235                 240                 245
   Ile Glu Lys Thr Ile Lys Ala Cys Lys Pro Ser Asp Gln Ile Leu
   250                 255                 260
   Lys Leu Leu Ser Leu Trp Arg Ile Lys Asn Gly Asp Gln Asp Thr
   265                 270                 275
   Leu Lys Gly Leu Met His Ala Leu Lys His Ser Lys Thr Tyr His
   280                 285                 290
   Phe Pro Lys Thr Val Thr Gln Ser Leu Lys Lys Thr Ile Arg Phe
   295                 300                 305
   Leu His Ser Phe Thr Met Tyr Lys Leu Tyr Gln Lys Leu Phe Leu
   310                 315                 320
   Glu Met Ile Gly Asn Gln Val Gln Ser Val Lys Ile Ser Cys Leu
   325                 330                 335
(2) INFORMATION FOR SEQUENCE ID NO: 68:
   (i) SEQUENCE CHARACTERISTICS:
      (A) LENGTH : 359 amino acids
      (B) TYPE : amino acid
      (C) STRANDEDNESS : single
      (D) TOPOLOGY : linear
   (ii) MOLECULE TYPE : Protein (OCIF-DCR2)
   (xi) SEQUENCE DESCRIPTION :SEQ ID NO: 68:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Glu Cys Lys Glu Gly Arg Tyr Leu Glu Ile Glu Phe
   40                  45                  50
   Cys Leu Lys His Arg Ser Cys Pro Pro Gly Phe Gly Val Val Gln
   55                  60                  65
   Ala Gly Thr Pro Glu Arg Asn Thr Val Cys Lys Arg Cys Pro Asp
   70                  75                  80
   Gly Phe Phe Ser Asn Glu Thr Ser Ser Lys Ala Pro Cys Arg Lys
   85                  90                  95
   His Thr Asn Cys Ser Val Phe Gly Leu Leu Leu Thr Gln Lys Gly
   100                 105                 110
   Asn Ala Thr His Asp Asn Ile Cys Ser Gly Asn Ser Glu Ser Thr
   115                 120                 125
   Gln Lys Cys Gly Ile Asp Val Thr Leu Cys Glu Glu Ala Phe Phe
   130                 135                 140
   Arg Phe Ala Val Pro Thr Lys Phe Thr Pro Asn Trp Leu Ser Val
   145                 150                 155
   Leu Val Asp Asn Leu Pro Gly Thr Lys Val Asn Ala Glu Ser Val
   160                 165                 170
   Glu Arg Ile Lys Arg Gln His Ser Ser Gln Glu Gln Thr Phe Gln
   175                 180                 185
   Leu Leu Lys Leu Trp Lys His Gln Asn Lys Asp Gln Asp Ile Val
   190                 195                 200
   Lys Lys Ile Ile Gln Asp Ile Asp Leu Cys Glu Asn Ser Val Gln
   205                 210                 215
   Arg His Ile Gly His Ala Asn Leu Thr Phe Glu Gln Leu Arg Ser
   220                 225                 230
   Leu Met Glu Ser Leu Pro Gly Lys Lys Val Gly Ala Glu Asp Ile
   235                 240                 245
   Glu Lys Thr Ile Lys Ala Cys Lys Pro Ser Asp Gln Ile Leu Lys
   250                 255                 260
   Leu Leu Ser Leu Trp Arg Ile Lys Asn Gly Asp Gln Asp Thr Leu
   265                 270                 275
   Lys Gly Leu Met His Ala Leu Lys His Ser Lys Thr Tyr His Phe
   280                 285                 290
   Pro Lys Thr Val Thr Gln Ser Leu Lys Lys Thr Ile Arg Phe Leu
   295                 300                 305
   His Ser Phe Thr Met Tyr Lys Leu Tyr Gln Lys Leu Phe Leu Glu
   310                 315                 320
   Met Ile Gly Asn Gln Val Gln Ser Val Lys Ile Ser Cys Leu
   325                 330                 335
(2) INFORMATION FOR SEQUENCE ID NO: 69:
   (i) SEQUENCE CHARACTERISTICS:
      (A) LENGTH : 363 amino acids
      (B) TYPE : amino acid
      (C) STRANDEDNESS : single
      (D) TOPOLOGY : linear
   (ii) MOLECULE TYPE : protein (OCIF-DCR3)
   (xi) SEQUENCE DESCRIPTION :SEQ ID NO: 69:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1              5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Arg Cys Pro Asp Gly Phe Phe Ser Asn Glu Thr Ser Ser Lys Ala
   85                  90                  95
   Pro Cys Arg Lys His Thr Asn Cys Ser Val Phe Gly Leu Leu Leu
   100                 105                 110
   Thr Gln Lys Gly Asn Ala Thr His Asp Asn Ile Cys Ser Gly Asn
   115                 120                 125
   Ser Glu Ser Thr Gln Lys Cys Gly Ile Asp Val Thr Leu Cys Glu
   130                 135                 140
   Glu Ala Phe Phe Arg Phe Ala Val Pro Thr Lys Phe Thr Pro Asn
   145                 150                 155
   Trp Leu Ser Val Leu Val Asp Asn Leu Pro Gly Thr Lys Val Asn
   160                 165                 170
   Ala Glu Ser Val Glu Arg Ile Lys Arg Gln His Ser Ser Gln Glu
   175                 180                 185
   Gln Thr Phe Gln Leu Leu Lys Leu Trp Lys His Gln Asn Lys Asp
   190                 195                 200
   Gln Asp Ile Val Lys Lys Ile Ile Gln Asp Ile Asp Leu Cys Glu
   205                 210                 215
   Asn Ser Val Gln Arg His Ile Gly His Ala Asn Leu Thr Phe Glu
   220                 225                 230
   Gln Leu Arg Ser Leu Met Glu Ser Leu Pro Gly Lys Lys Val Gly
   235                 240                 245
   Ala Glu Asp Ile Glu Lys Thr Ile Lys Ala Cys Lys Pro Ser Asp
   250                 255                 260
   Gln Ile Leu Lys Leu Leu Ser Leu Trp Arg Ile Lys Asn Gly Asp
   265                 270                 275
   Gln Asp Thr Leu Lys Gly Leu Met His Ala Leu Lys His Ser Lys
   280                 285                 290
   Thr Tyr His Phe Pro Lys Thr Val Thr Gln Ser Leu Lys Lys Thr
   295                 300                 305
   Ile Arg Phe Leu His Ser Phe Thr Met Tyr Lys Leu Tyr Gln Lys
   310                 315                 320
   Leu Phe Leu Glu Met Ile Gly Asn Gln Val Gln Ser Val Lys Ile
   325                 330                 335
   Ser Cys Leu
   340
(2) INFORMATION FOR SEQUENCE ID NO: 70:
   (i) SEQUENCE CHARACTERISTICS:
      (A) LENGTH : 359 amino acids
      (B) TYPE : amino acid
      (C) STRANDEDNESS : single
      (D) TOPOLOGY : linear
   (ii) MOLECULE TYPE : protein (OCIF-DCR4)
   (xi) SEQUENCE DESCRIPTION :SEQ ID NO: 70:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Ser Gly Asn Ser Glu Ser Thr
   115                 120                 125
   Gln Lys Cys Gly Ile Asp Val Thr Leu Cys Glu Glu Ala Phe Phe
   130                 135                 140
   Arg Phe Ala Val Pro Thr Lys Phe Thr Pro Asn Trp Leu Ser Val
   145                 150                 155
   Leu Val Asp Asn Leu Pro Gly Thr Lys Val Asn Ala Glu Ser Val
   160                 165                 170
   Glu Arg Ile Lys Arg Gln His Ser Ser Gln Glu Gln Thr Phe Gln
   175                 180                 185
   Leu Leu Lys Leu Trp Lys His Gln Asn Lys Asp Gln Asp Ile Val
   190                 195                 200
   Lys Lys Ile Ile Gln Asp Ile Asp Leu Cys Glu Asn Ser Val Gln
   205                 210                 215
   Arg His Ile Gly His Ala Asn Leu Thr Phe Glu Gln Leu Arg Ser
   220                 225                 230
   Leu Met Glu Ser Leu Pro Gly Lys Lys Val Gly Ala Glu Asp Ile
   235                 240                 245
   Glu Lys Thr Ile Lys Ala Cys Lys Pro Ser Asp Gln Ile Leu Lys
   250                 255                 260
   Leu Leu Ser Leu Trp Arg Ile Lys Asn Gly Asp Gln Asp Thr Leu
   265                 270                 275
   Lys Gly Leu Met His Ala Leu Lys His Ser Lys Thr Tyr His Phe
   280                 285                 290
   Pro Lys Thr Val Thr Gln Ser Leu Lys Lys Thr Ile Arg Phe Leu
   295                 300                 305
   His Ser Phe Thr Met Tyr Lys Leu Tyr Gln Lys Leu Phe Leu Glu
   310                 315                 320
   Met Ile Gly Asn Gln Val Gln Ser Val Lys Ile Ser Cys Leu
   325                 330                 335
(2) INFORMATION FOR SEQUENCE ID NO: 71:
   (i) SEQUENCE CHARACTERISTICS:
      (A) LENGTH : 326 amino acids
      (B) TYPE : amino acid
      (C) STRANDEDNESS : single
      (D) TOPOLOGY : linear
   (ii) MOLECULE TYPE : protein (OCIF-DDD1)
   (xi) SEQUENCE DESCRIPTION :SEQ ID NO: 71:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1                5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Ile Asp Leu Cys Glu Asn Ser Val Gln Arg His Ile
   175                 180                 185
   Gly His Ala Asn Leu Thr Phe Glu Gln Leu Arg Ser Leu Met Glu
   190                 195                 200
   Ser Leu Pro Gly Lys Lys Val Gly Ala Glu Asp Ile Glu Lys Thr
   205                 210                 215
   Ile Lys Ala Cys Lys Pro Ser Asp Gln Ile Leu Lys Leu Leu Ser
   220                 225                 230
   Leu Trp Arg Ile Lys Asn Gly Asp Gln Asp Thr Leu Lys Gly Leu
   235                 240                 245
   Met His Ala Leu Lys His Ser Lys Thr Tyr His Phe Pro Lys Thr
   250                 255                 260
   Val Thr Gln Ser Leu Lys Lys Thr Ile Arg Phe Leu His Ser Phe
   265                 270                 275
   Thr Met Tyr Lys Leu Tyr Gln Lys Leu Phe Leu Glu Met Ile Gly
   280                 285                 290
   Asn Gln Val Gln Ser Val Lys Ile Ser Cys Leu
   295                 300                 305
(2) INFORMATION FOR SEQUENCE ID NO: 72:
   (i) SEQUENCE CHARACTERISTICS:
      (A) LENGTH: 327 amino acids
      (B) TYPE: amino acid
      (C) STRANDEDNESS : single
      (D) TOPOLOGY : linear
   (ii) MOLECULE TYPE : protein (OCIF-DDD2)
   (xi) SEQUENCE DESCRIPTION :SEQ ID NO: 72:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1                   5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ala Leu Lys His Ser Lys Thr Tyr His Phe Pro Lys
   250                 255                 260
   Thr Val Thr Gln Ser Leu Lys Lys Thr Ile Arg Phe Leu His Ser
   265                 270                 275
   Phe Thr Met Tyr Lys Leu Tyr Gln Lys Leu Phe Leu Glu Met Ile
   280                 285                 290
   Gly Asn Gln Val Gln Ser Val Lys Ile Ser Cys Leu
   295                 300                 305
(2) INFORMATION FOR SEQUENCE ID NO: 73:
   (i) SEQUENCE CHARACTERISTICS:
      (A) LENGTH : 399 amino acids
      (B) TYPE : amino acid
      (C) STRANDEDNESS : single
      (D) TOPOLOGY : linear
   (ii) MOLECULE TYPE : protein (OCIF-CL)
   (xi) SEQUENCE DESCRIPTION :SEQ ID NO: 73:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Gln Val Gln Ser Val Lys Ile Ser
   370                 375
(2) INFORMATION FOR SEQUENCE ID NO: 74:
   (i) SEQUENCE CHARACTERISTICS:
      (A) LENGTH : 351 amino acids
      (B) TYPE : amino acid
      (C) STRANDEDNESS : single
      (D) TOPOLOGY : linear
   (ii) MOLECULE TYPE : protein (OCIF-CC)
   (xi) SEQUENCE DESCRIPTION :SEQ ID NO: 74:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His
   325                 330
(2) INFORMATION FOR SEQUENCE ID NO: 75:
   (i) SEQUENCE CHARACTERISTICS:
      (A) LENGTH : 272 amino acids
      (B) TYPE : amino acid
      (C) STRANDEDNESS : single
      (D) TOPOLOGY : linear
   (ii) MOLECULE TYPE : Protein (OCIF-CDD2)
   (xi) SEQUENCE DESCRIPTION :SEQ ID NO: 75:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1                   5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln
   250
(2) INFORMATION FOR SEQUENCE ID NO: 76:
   (i) SEQUENCE CHARACTERISTICS:
      (A) LENGTH : 197 amino acids
      (B) TYPE : amino acid
      (C) STRANDEDNESS : single
      (D) TOPOLOGY : linear
   (ii) MOLECULE TYPE : Protein (OCIF-CDD1)
   (xi) SEQUENCE DESCRIPTION :SEQ ID NO: 76:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1  1                   5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile
   175
(2) INFORMATION FOR SEQUENCE ID NO: 77:
   (i) SEQUENCE CHARACTERISTICS:
      (A) LENGTH : 143 amino acids
      (B) TYPE : amino acid
      (C) STRANDEDNESS : single
      (D) TOPOLOGY : linear
   (ii) MOLECULE TYPE : Protein (OCIF-CCR4)
   (xi) SEQUENCE DESCRIPTION :SEQ ID NO: 77:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys
   115                 120
(2) INFORMATION FOR SEQUENCE ID NO: 78:
   (i) SEQUENCE CHARACTERISTICS:
      (A) LENGTH : 106 amino acids
      (B) TYPE : amino acid
      (C) STRANDEDNESS : single
      (D) TOPOLOGY : linear
   (ii) MOLECULE TYPE : Protein (OCIF-CCR3)
   (xi) SEQUENCE DESCRIPTION :SEQ ID NO: 78:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu
   85
(2) INFORMATION FOR SEQUENCE ID NO: 79:
   (i) SEQUENCE CHARACTERISTICS:
      (A) LENGTH : 393 amino acids
      (B) TYPE : amino acid
      (D) TOPOLOGY : linear
   (ii) MOLECULE TYPE : Protein (OCIF-CBst)
   (xi) SEQUENCE DESCRIPTION :SEQ ID NO: 79:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5              -1   1                  5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Cys Lys Pro Ser Asp Gln Ile Leu Lys Leu Leu Ser
   295                 300                 305
   Leu Trp Arg Ile Lys Asn Gly Asp Gln Asp Thr Leu Lys Gly Leu
   310                 315                 320
   Met His Ala Leu Lys His Ser Lys Thr Tyr His Phe Pro Lys Thr
   325                 330                 335
   Val Thr Gln Ser Leu Lys Lys Thr Ile Arg Phe Leu His Ser Phe
   340                 345                 350
   Thr Met Tyr Lys Leu Tyr Gln Lys Leu Phe Leu Glu Met Ile Gly
   355                 360                 365
   Asn Leu Val
   370
(2) INFORMATION FOR SEQUENCE ID NO: 80:
   (i) SEQUENCE CHARACTERISTICS:
      (A) LENGTH : 321 amino acids
      (B) TYPE : amino acid
      (D) TOPOLOGY : linear
   (ii) MOLECULE TYPE : Protein (OCIF-CSph)
   (xi) SEQUENCE DESCRIPTION :SEQ ID NO: 80:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1  1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly Asn Ser Glu Ser Thr Gln Lys Cys
   160                 165                 170
   Gly Ile Asp Val Thr Leu Cys Glu Glu Ala Phe Phe Arg Phe Ala
   175                 180                 185
   Val Pro Thr Lys Phe Thr Pro Asn Trp Leu Ser Val Leu Val Asp
   190                 195                 200
   Asn Leu Pro Gly Thr Lys Val Asn Ala Glu Ser Val Glu Arg Ile
   205                 210                 215
   Lys Arg Gln His Ser Ser Gln Glu Gln Thr Phe Gln Leu Leu Lys
   220                 225                 230
   Leu Trp Lys His Gln Asn Lys Asp Gln Asp Ile Val Lys Lys Ile
   235                 240                 245
   Ile Gln Asp Ile Asp Leu Cys Glu Asn Ser Val Gln Arg His Ile
   250                 255                 260
   Gly His Ala Asn Leu Thr Phe Glu Gln Leu Arg Ser Leu Met Glu
   265                 270                 275
   Ser Leu Pro Gly Lys Lys Val Gly Ala Glu Asp Ile Glu Lys Thr
   280                 285                 290
   Ile Lys Ala Ser Leu Asp
   295                 300
(2) INFORMATION FOR SEQUENCE ID NO: 81:
   (i) SEQUENCE CHARACTERISTICS:
      (A) LENGTH : 187 amino acids
      (B) TYPE : amino acid
      (D) TOPOLOGY : linear
   (ii) MOLECULE TYPE : Protein (OCIF-CBsp)
   (xi) SEQUENCE DESCRIPTION :SEQ ID NO: 81:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Cys Ser Pro Val Cys Lys Glu Leu
   55                  60                  65
   Gln Tyr Val Lys Gln Glu Cys Asn Arg Thr His Asn Arg Val Cys
   70                  75                  80
   Glu Cys Lys Glu Gly Arg Tyr Leu Glu Ile Glu Phe Cys Leu Lys
   85                  90                  95
   His Arg Ser Cys Pro Pro Gly Phe Gly Val Val Gln Ala Gly Thr
   100                 105                 110
   Pro Glu Arg Asn Thr Val Cys Lys Arg Cys Pro Asp Gly Phe Phe
   115                 120                 125
   Ser Asn Glu Thr Ser Ser Lys Ala Pro Cys Arg Lys His Thr Asn
   130                 135                 140
   Cys Ser Val Phe Gly Leu Leu Leu Thr Gln Lys Gly Asn Ala Thr
   145                 150                 155
   His Asp Asn Ile Cys Ser Gly
   160                 165             166
(2) INFORMATION FOR SEQUENCE ID NO: 82:
   (i) SEQUENCE CHARACTERISTICS:
      (A) LENGTH : 84 amino acids
      (B) TYPE : amino acid
      (D) TOPOLOGY : linear
   (ii) MOLECULE TYPE : Protein (OCIF-CPst)
   (xi) SEQUENCE DESCRIPTION :SEQ ID NO: 82:
   Met Asn Asn Leu Leu Cys Cys Ala Leu Val Phe Leu Asp Ile Ser
       -20                 -15                 -10
   Ile Lys Trp Thr Thr Gln Glu Thr Phe Pro Pro Lys Tyr Leu His
       -5                  -1   1               5
   Tyr Asp Glu Glu Thr Ser His Gln Leu Leu Cys Asp Lys Cys Pro
   10                  15                  20
   Pro Gly Thr Tyr Leu Lys Gln His Cys Thr Ala Lys Trp Lys Thr
   25                  30                  35
   Val Cys Ala Pro Cys Pro Asp His Tyr Tyr Thr Asp Ser Trp His
   40                  45                  50
   Thr Ser Asp Glu Cys Leu Tyr Leu Val
   55                  60          63
(2) INFORMATION FOR SEQUENCE ID NO: 83:
   (i) SEQUENCE CHARACTERISTICS:
      (A) LENGTH : 1206 base pairs
      (B) TYPE : nucleic acid
      (C) STRANDEDNESS : single
      (D) TOPOLOGY : linear
   (it) MOLECULE TYPE : _{C}DNA (OCIF-C19S)
   (xi) SEQUENCE DESCRIPTION :SEQ ID NO: 83:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AAAGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                            1206
(2) INFORMATION FOR SEQUENCE ID NO: 84:
   (i) SEQUENCE CHARACTERISTICS:
      (A) LENGTH : 1206 base pairs
      (B) TYPE : nucleic acid
      (C) STRANDEDNESS : single
      (D) TOPOLOGY : linear
   (ii) MOLECULE TYPE : cDNA (OCIF-C20S)
   (xi) SEQUENCE DESCRIPTION :SEQ ID NO: 84:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGAGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                           1206
(2) INFORMATION FOR SEQUENCE ID NO: 85:
   (i) SEQUENCE CHARACTERISTICS:
      (A) LENGTH : 1206 base pairs
      (B) TYPE : nucleic acid
      (C) STRANDEDNESS : single
      (D) TOPOLOGY : linear
   (ii) MOLECULE TYPE : cDNA (OCIF-C21S)
   (xi) SEQUENCE DESCRIPTION :SEQ ID NO: 85:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCAG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                           1206
(2) INFORMATION FOR SEQUENCE ID NO: 86:
   (i) SEQUENCE CHARACTERISTICS:
      (A) LENGTH : 1206 base pairs
      (B) TYPE : nucleic acid
      (C) STRANDEDNESS : single
      (D) TOPOLOGY : linear
   (ii) MOLECULE TYPE : CDNA (OCIF-C22S)
   (xi) SEQUENCE DESCRIPTION :SEQ ID NO: 86:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGATA TTGACCTCTG TGAAAACAGC 840
   GTGCAGCGGC ACATTGGACA TGCTAACCTC ACCTTCGAGC AGCTTCGTAG CTTGATGGAA 900
   AGCTTACCGG GAAAGAAAGT GGGAGCAGAA GACATTGAAA AAACAATAAA GGCAAGCAAA
   960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTGC 1200
   TTATAA                                                           1206
(2) INFORMATION FOR SEQUENCE ID NO: 87:
   (i) SEQUENCE CHARACTERISTICS:
      (A) LENGTH : 1206 base pairs
      (B) TYPE : nucleic acid
      (C) STRANDEDNESS : single
      (D) TOPOLOGY : linear
   (ii) MOLECULE TYPE : cDNA (OCIF-C23S)
   (xi) SEQUENCE DESCRIPTION :SEQ ID NO: 87:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCAGC 1200
   TTATAA                                                           1206
(2) INFORMATION FOR SEQUENCE ID NO: 88:
   (i) SEQUENCE CHARACTERISTICS:
      (A) LENGTH : 1083 base pairs
      (B) TYPE : nucleic acid
      (C) STRANDEDNESS : single
      (D) TOPOLOGY : linear
   (ii) MOLECULE TYPE : CDNA (OCIF-DCR1)
   (xi) SEQUENCE DESCRIPTION :SEQ ID NO: 88:
   ATGAACAACT TGCTGTGCTG CGCGCTCGTG TTTCTGGACA TCTCCATTAA GTGGACCACC   60
   CAGGAACCTT GCCCTGACCA CTACTACACA GACAGCTGGC ACACCAGTGA CGAGTGTCTA  120
   TACTGCAGCC CCGTGTGCAA GGAGCTGCAG TACGTCAAGC AGGAGTGCAA TCGCACCCAC  180
   AACCGCGTGT GCGAATGCAA GGAAGGGCGC TACCTTGAGA TAGAGTTCTG CTTGAAACAT  240
   AGGAGCTGCC CTCCTGGATT TGGAGTGGTG CAAGCTGGAA CCCCAGAGCG AAATACAGTT  300
   TGCAAAAGAT GTCCAGATGG GTTCTTCTCA AATGAGACGT CATCTAAAGC ACCCTGTAGA  360
   AAACACACAA ATTGCAGTGT CTTTGGTCTC CTGCTAACTC AGAAAGGAAA TGCAACACAC  420
   GACAACATAT GTTCCGGAAA CAGTGAATCA ACTCAAAAAT GTGGAATAGA TGTTACCCTG  480
   TGTGAGGAGG CATTCTTCAG GTTTGCTGTT CCTACAAAGT TTACGCCTAA CTGGCTTAGT  540
   GTCTTGGTAG ACAATTTGCC TGGCACCAAA GTAAACGCAG AGAGTGTAGA GAGGATAAAA  600
   CGGCAACACA GCTCACAAGA ACAGACTTTC CAGCTGCTGA AGTTATGGAA ACATCAAAAC  660
   AAAGACCAAG ATATAGTCAA GAAGATCATC CAAGATATTG ACCTCTGTGA AAACAGCGTG  720
   CAGCGGCACA TTGGACATGC TAACCTCACC TTCGAGCAGC TTCGTAGCTT GATGGAAAGC  780
   TTACCGGGAA AGAAAGTGGG AGCAGAAGAC ATTGAAAAAA CAATAAAGGC ATGCAAACCC  840
   AGTGACCAGA TCCTGAAGCT GCTCAGTTTG TGGCGAATAA AAAATGGCGA CCAAGACACC  900
   TTGAAGGGCC TAATGCACGC ACTAAAGCAC TCAAAGACGT ACCACTTTCC CAAAACTGTC  960
   ACTCAGAGTC TAAAGAAGAC CATCAGGTTC CTTCACAGCT TCACAATGTA CAAATTGTAT 1020
   CAGAAGTTAT TTTTAGAAAT GATAGGTAAC CAGGTCCAAT CAGTAAAAAT AAGCTGCTTA 1080
   TAA                                                              1083
(2) INFORMATION FOR SEQUENCE ID NO: 89:
   (i) SEQUENCE CHARACTERISTICS:
      (A) LENGTH : 1080 base pairs
      (B) TYPE : nucleic acid
      (C) STRANDEDNESS : single
      (D) TOPOLOGY : linear
   (ii) MOLECULE TYPE : cDNA (OCIF-DCR2)
   (xi) SEQUENCE DESCRIPTION :SEQ ID NO: 89:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCG AATGCAAGGA AGGGCGCTAC CTTGAGATAG AGTTCTGCTT GAAACATAGG  240
   AGCTGCCCTC CTGGATTTGG AGTGGTGCAA GCTGGAACCC CAGAGCGAAA TACAGTTTGC  300
   AAAAGATGTC CAGATGGGTT CTTCTCAAAT GAGACGTCAT CTAAAGCACC CTGTAGAAAA  360
   CACACAAATT GCAGTGTCTT TGGTCTCCTG CTAACTCAGA AAGGAAATGC AACACACGAC  420
   AACATATGTT CCGGAAACAG TGAATCAACT CAAAAATGTG GAATAGATGT TACCCTGTGT  480
   GAGGAGGCAT TCTTCAGGTT TGCTGTTCCT ACAAAGTTTA CGCCTAACTG GCTTAGTGTC  540
   TTGGTAGACA ATTTGCCTGG CACCAAAGTA AACGCAGAGA GTGTAGAGAG GATAAAACGG  600
   CAACACAGCT CACAAGAACA GACTTTCCAG CTGCTGAAGT TATGGAAACA TCAAAACAAA  660
   GACCAAGATA TAGTCAAGAA GATCATCCAA GATATTGACC TCTGTGAAAA CAGCGTGCAG  720
   CGGCACATTG GACATGCTAA CCTCACCTTC GAGCAGCTTC GTAGCTTGAT GGAAAGCTTA  780
   CCGGGAAAGA AAGTGGGAGC AGAAGACATT GAAAAAACAA TAAAGGCATG CAAACCCAGT  840
   GACCAGATCC TGAAGCTGCT CAGTTTGTGG CGAATAAAAA ATGGCGACCA AGACACCTTG  900
   AAGGGCCTAA TGCACGCACT AAAGCACTCA AAGACGTACC ACTTTCCCAA AACTGTCACT  960
   CAGAGTCTAA AGAAGACCAT CAGGTTCCTT CACAGCTTCA CAATGTACAA ATTGTATCAG 1020
   AAGTTATTTT TAGAAATGAT AGGTAACCAG GTCCAATCAG TAAAAATAAG CTGCTTATAA 1080
(2) INFORMATION FOR SEQUENCE ID NO: 90:
   (i) SEQUENCE CHARACTERISTICS:
      (A) LENGTH : 1092 base pairs
      (B) TYPE : nucleic acid
      (C) STRANDEDNESS : single
      (D) TOPOLOGY : linear
   (ii) MOLECULE TYPE : cDNA (OCIF-DCR3)
   (xi) SEQUENCE DESCRIPTION :SEQ ID NO: 90:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCAGATG TCCAGATGGG TTCTTCTCAA ATGAGACGTC ATCTAAAGCA 360
   CCCTGTAGAA AACACACAAA TTGCAGTGTC TTTGGTCTCC TGCTAACTCA GAAAGGAAAT 420
   GCAACACACG ACAACATATG TTCCGGAAAC AGTGAATCAA CTCAAAAATG TGGAATAGAT 480
   GTTACCCTGT GTGAGGAGGC ATTCTTCAGG TTTGCTGTTC CTACAAAGTT TACGCCTAAC 540
   TGGCTTAGTG TCTTGGTAGA CAATTTGCCT GGCACCAAAG TAAACGCAGA GAGTGTAGAG 600
   AGGATAAAAC GGCAACACAG CTCACAAGAA CAGACTTTCC AGCTGCTGAA GTTATGGAAA 660
   CATCAAAACA AAGACCAAGA TATAGTCAAG AAGATCATCC AAGATATTGA CCTCTGTGAA 720
   AACAGCGTGC AGCGGCACAT TGGACATGCT AACCTCACCT TCGAGCAGCT TCGTAGCTTG 780
   ATGGAAAGCT TACCGGGAAA GAAAGTGGGA GCAGAAGACA TTGAAAAAAC AATAAAGGCA 840
   TGCAAACCCA GTGACCAGAT CCTGAAGCTG CTCAGTTTGT GGCGAATAAA AAATGGCGAC 900
   CAAGACACCT TGAAGGGCCT AATGCACGCA CTAAAGCACT CAAAGACGTA CCACTTTCCC 960
   AAAACTGTCA CTCAGAGTCT AAAGAAGACC ATCAGGTTCC TTCACAGCTT CACAATGTAC 1020
   AAATTGTATC AGAAGTTATT TTTAGAAATG ATAGGTAACC AGGTCCAATC AGTAAAAATA 1080
   AGCTGCTTAT AA                                                    1092
(2) INFORMATION FOR SEQUENCE ID NO: 91:
   (i) SEQUENCE CHARACTERISTICS:
      (A) LENGTH : 1080 base pairs
      (B) TYPE : nucleic acid
      (C) STRANDEDNESS : single
      (D) TOPOLOGY : linear
   (ii) MOLECULE TYPE : cDNA (OCIF-DCR4)
   (xi) SEQUENCE DESCRIPTION :SEQ ID NO: 91:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAT CCGGAAACAG TGAATCAACT CAAAAATGTG GAATAGATGT TACCCTGTGT 480
   GAGGAGGCAT TCTTCAGGTT TGCTGTTCCT ACAAAGTTTA CGCCTAACTG GCTTAGTGTC 540
   TTGGTAGACA ATTTGCCTGG CACCAAAGTA AACGCAGAGA GTGTAGAGAG GATAAAACGG 600
   CAACACAGCT CACAAGAACA GACTTTCCAG CTGCTGAAGT TATGGAAACA TCAAAACAAA 660
   GACCAAGATA TAGTCAAGAA GATCATCCAA GATATTGACC TCTGTGAAAA CAGCGTGCAG 720
   CGGCACATTG GACATGCTAA CCTCACCTTC GAGCAGCTTC GTAGCTTGAT GGAAAGCTTA 780
   CCGGGAAAGA AAGTGGGAGC AGAAGACATT GAAAAAACAA TAAAGGCATG CAAACCCAGT 840
   GACCAGATCC TGAAGCTGCT CAGTTTGTGG CGAATAAAAA ATGGCGACCA AGACACCTTG 900
   AAGGGCCTAA TGCACGCACT AAAGCACTCA AAGACGTACC ACTTTCCCAA AACTGTCACT 960
   CAGAGTCTAA AGAAGACCAT CAGGTTCCTT CACAGCTTCA CAATGTACAA ATTGTATCAG 1020
   AAGTTATTTT TAGAAATGAT AGGTAACCAG GTCCAATCAG TAAAAATAAG CTGCTTATAA 1080
(2) INFORMATION FOR SEQUENCE ID NO: 92:
   (i) SEQUENCE CHARACTERISTICS:
      (A) LENGTH : 981 base pairs
      (B) TYPE : nucleic acid
      (C) STRANDEDNESS : single
      (D) TOPOLOGY : linear
   (ii) MOLECULE TYPE : cDNA (OCIF-DDD1)
   (xi) SEQUENCE DESCRIPTION :SEQ ID NO: 92:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATATTGAC 600
   CTCTGTGAAA ACAGCGTGCA GCGGCACATT GGACATGCTA ACCTCACCTT CGAGCAGCTT 660
   CGTAGCTTGA TGGAAAGCTT ACCGGGAAAG AAAGTGGGAG CAGAAGACAT TGAAAAAACA 720
   ATAAAGGCAT GCAAACCCAG TGACCAGATC CTGAAGCTGC TCAGTTTGTG GCGAATAAAA 780
   AATGGCGACC AAGACACCTT GAAGGGCCTA ATGCACGCAC TAAAGCACTC AAAGACGTAC 840
   CACTTTCCCA AAACTGTCAC TCAGAGTCTA AAGAAGACCA TCAGGTTCCT TCACAGCTTC 900
   ACAATGTACA AATTGTATCA GAAGTTATTT TTAGAAATGA TAGGTAACCA GGTCCAATCA 960
   GTAAAAATAA GCTGCTTATA A                                          981
(2) INFORMATION FOR SEQUENCE ID NO: 93:
   (i) SEQUENCE CHARACTERISTICS:
      (A) LENGTH : 984 base pairs
      (B) TYPE : nucleic acid
      (C) STRANDEDNESS : single
      (D) TOPOLOGY : linear
   (ii) MOLECULE TYPE : cDNA (OCIF-DDD2)
   (xi) SEQUENCE DESCRIPTION :SEQ ID NO: 93:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAAGACG CACTAAAGCA CTCAAAGACG 840
   TACCACTTTC CCAAAACTGT CACTCAGAGT CTAAAGAAGA CCATCAGGTT CCTTCACAGC 900
   TTCACAATGT ACAAATTGTA TCAGAAGTTA TTTTTAGAAA TGATAGGTAA CCAGGTCCAA 960
   TCAGTAAAAA TAAGCTGCTT ATAA                                       984
(2) INFORMATION FOR SEQUENCE ID NO: 94:
   (i) SEQUENCE CHARACTERISTICS:
      (A) LENGTH : 1200 base pairs
      (B) TYPE : nucleic acid
      (C) STRANDEDNESS : single
      (D) TOPOLOGY : linear
   (ii) MOLECULE TYPE : cDNA (OCIF-CL)
   (xi) SEQUENCE DESCRIPTION :SEQ ID NO: 94:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCAGGTCC AATCAGTAAA AATAAGCTAA 1200
(2) INFORMATION FOR SEQUENCE ID NO: 95:
   (i) SEQUENCE CHARACTERISTICS:
      (A) LENGTH : 1056 base pairs
      (B) TYPE : nucleic acid
      (C) STRANDEDNESS : single
      (D) TOPOLOGY : linear
   (ii) MOLECULE TYPE : cDNA (OCIF-CC)
   (xi) SEQUENCE DESCRIPTION :SEQ ID NO: 95:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTGA                           1056
(2) INFORMATION FOR SEQUENCE ID NO: 96:
   (i) SEQUENCE CHARACTERISTICS:
      (A) LENGTH : 819 base pairs
      (B) TYPE : nucleic acid
      (C) STRANDEDNESS : single
      (D) TOPOLOGY : linear
   (ii) MOLECULE TYPE : cDNA (OCIF-CDD2)
   (xi) SEQUENCE DESCRIPTION :SEQ ID NO: 96:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT AGATGTTACC 600
   CTGTGTGAGG AGGCATTCTT CAGGTTTGCT GTTCCTACAA AGTTTACGCC TAACTGGCTT 660
   AGTGTCTTGG TAGACAATTT GCCTGGCACC AAAGTAAACG CAGAGAGTGT AGAGAGGATA 720
   AAACGGCAAC ACAGCTCACA AGAACAGACT TTCCAGCTGC TGAAGTTATG GAAACATCAA 780
   AACAAAGACC AAGATATAGT CAAGAAGATC ATCCAATGA                       819
(2) INFORMATION FOR SEQUENCE ID NO: 97:
   (i) SEQUENCE CHARACTERISTICS:
      (A) LENGTH : 594 base pairs
      (B) TYPE : nucleic acid
      (C) STRANDEDNESS : single
      (D) TOPOLOGY : linear
   (ii) MOLECULE TYPE : cDNA (OCIF-CDD1)
   (xi) SEQUENCE DESCRIPTION :SEQ ID NO: 97:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG AAACAGTGAA TCAACTCAAA AATGTGGAAT ATGA       594
(2) INFORMATION FOR SEQUENCE ID NO: 98:
   (i) SEQUENCE CHARACTERISTICS:
      (A) LENGTH : 432 base pairs
      (B) TYPE : nucleic acid
      (C) STRANDEDNESS : single
      (D) TOPOLOGY : linear
   (ii) MOLECULE TYPE : cDNA (OCIF-CCR4)
   (xi) SEQUENCE DESCRIPTION :SEQ ID NO: 98:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAT GA                                                    432
(2) INFORMATION FOR SEQUENCE ID NO: 99:
   (i) SEQUENCE CHARACTERISTICS:
      (A) LENGTH : 321 base pairs
      (B) TYPE : nucleic acid
      (C) STRANDEDNESS : single
      (D) TOPOLOGY : linear
   (ii) MOLECULE TYPE : cDNA (OCIF-CCR3)
   (xi) SEQUENCE DESCRIPTION :SEQ ID NO: 99:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG A                                          321
(2) INFORMATION FOR SEQUENCE ID NO: 100:
   (i) SEQUENCE CHARACTERISTICS:
      (A) LENGTH : 1182 base pairs
      (B) TYPE : nucleic acid
      (C) STRANDEDNESS : single
      (D) TOPOLOGY : linear
   (ii) MOLECULE TYPE : cDNA (OCIF-CBst)
   (xi) SEQUENCE DESCRIPTION :SEQ ID NO: 100:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCATGCAAA  960
   CCCAGTGACC AGATCCTGAA GCTGCTCAGT TTGTGGCGAA TAAAAAATGG CGACCAAGAC 1020
   ACCTTGAAGG GCCTAATGCA CGCACTAAAG CACTCAAAGA CGTACCACTT TCCCAAAACT 1080
   GTCACTCAGA GTCTAAAGAA GACCATCAGG TTCCTTCACA GCTTCACAAT GTACAAATTG 1140
   TATCAGAAGT TATTTTTAGA AATGATAGGT AACCTAGTCT AG                   1182
(2) INFORMATION FOR SEQUENCE ID NO: 101:
   (i) SEQUENCE CHARACTERISTICS:
      (A) LENGTH : 966 base pairs
      (B) TYPE : nucleic acid
      (D) TOPOLOGY : linear
   (ii) MOLECULE TYPE : cDNA (OCIF-CSph)
   (xi) SEQUENCE DESCRIPTION :SEQ ID NO: 101:
   ATGAACAACT TGCTGTGCTG CGCGCTCGTG TTTCTGGACA TCTCCATTAA GTGGACCACC   60
   CAGGAAACGT TTCCTCCAAA GTACCTTCAT TATGACGAAG AAACCTCTCA TCAGCTGTTG  120
   TGTGACAAAT GTCCTCCTGG TACCTACCTA AAACAACACT GTACAGCAAA GTGGAAGACC  180
   GTGTGCGCCC CTTGCCCTGA CCACTACTAC ACAGACAGCT GGCACACCAG TGACGAGTGT  240
   CTATACTGCA GCCCCGTGTG CAAGGAGCTG CAGTACGTCA AGCAGGAGTG CAATCGCACC  300
   CACAACCGCG TGTGCGAATG CAAGGAAGGG CGCTACCTTG AGATAGAGTT CTGCTTGAAA  360
   CATAGGAGCT GCCCTCCTGG ATTTGGAGTG GTGCAAGCTG GAACCCCAGA GCGAAATACA  420
   GTTTGCAAAA GATGTCCAGA TGGGTTCTTC TCAAATGAGA CGTCATCTAA AGCACCCTGT  480
   AGAAAACACA CAAATTGCAG TGTCTTTGGT CTCCTGCTAA CTCAGAAAGG AAATGCAACA  540
   CACGACAACA TATGTTCCGG AAACAGTGAA TCAACTCAAA AATGTGGAAT AGATGTTACC  600
   CTGTGTGAGG AGGCATTCTT CAGGTTTGCT GTTCCTACAA AGTTTACGCC TAACTGGCTT  660
   AGTGTCTTGG TAGACAATTT GCCTGGCACC AAAGTAAACG CAGAGAGTGT AGAGAGGATA  720
   AAACGGCAAC ACAGCTCACA AGAACAGACT TTCCAGCTGC TGAAGTTATG GAAACATCAA  780
   AACAAAGACC AAGATATAGT CAAGAAGATC ATCCAAGATA TTGACCTCTG TGAAAACAGC  840
   GTGCAGCGGC ACATTGGACA TGCTAACCTC ACCTTCGAGC AGCTTCGTAG CTTGATGGAA  900
   AGCTTACCGG GAAAGAAAGT GGGAGCAGAA GACATTGAAA AAACAATAAA GGCTAGTCTA  960
   GACTAG                                                            966
(2) INFORMATION FOR SEQUENCE ID NO: 102:
   (i) SEQUENCE CHARACTERISTICS:
      (A) LENGTH : 564 base pairs
      (B) TYPE : nucleic acid
      (C) STRANDEDNESS : single
      (D) TOPOLOGY : linear
   (ii) MOLECULE TYPE : cDNA (OCIF-CBsp)
   (xi) SEQUENCE DESCRIPTION :SEQ ID NO: 102:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACTGCA GCCCCGTGTG CAAGGAGCTG CAGTACGTCA AGCAGGAGTG CAATCGCACC 300
   CACAACCGCG TGTGCGAATG CAAGGAAGGG CGCTACCTTG AGATAGAGTT CTGCTTGAAA 360
   CATAGGAGCT GCCCTCCTGG ATTTGGAGTG GTGCAAGCTG GAACCCCAGA GCGAAATACA 420
   GTTTGCAAAA GATGTCCAGA TGGGTTCTTC TCAAATGAGA CGTCATCTAA AGCACCCTGT 480
   AGAAAACACA CAAATTGCAG TGTCTTTGGT CTCCTGCTAA CTCAGAAAGG AAATGCAACA 540
   CACGACAACA TATGTTCCGG CTAG                                       564
(2) INFORMATION FOR SEQUENCE ID NO: 103:
   (i) SEQUENCE CHARACTERISTICS:
      (A) LENGTH : 255 base pairs
      (B) TYPE : nucleic acid
      (C) STRANDEDNESS : single
      (D) TOPOLOGY : linear
   (ii) MOLECULE TYPE : cDNA (OCIF-CPst)
   (xi) SEQUENCE DESCRIPTION :SEQ ID NO: 103:
   ATGAACAACT TGCTGTGCTG CGCGCTCGTG TTTCTGGACA TCTCCATTAA GTGGACCACC  60
   CAGGAAACGT TTCCTCCAAA GTACCTTCAT TATGACGAAG AAACCTCTCA TCAGCTGTTG 120
   TGTGACAAAT GTCCTCCTGG TACCTACCTA AAACAACACT GTACAGCAAA GTGGAAGACC 180
   GTGTGCGCCC CTTGCCCTGA CCACTACTAC ACAGACAGCT GGCACACCAG TGACGAGTGT 240
   CTATACCTAG TCTAG                                                 255
(2) INFORMATION FOR SEQUENCE ID NO: 104:
   (i) SEQUENCE CHARACTERISTICS:
      (A) LENGTH : 1317 base pairs
      (B) TYPE : nucleic acid
      (C) STRANDEDNESS : double
      (D) TOPOLOGY : linear
   (ii) MOLECULE TYPE : human OCIF genomic DNA-1
   (xi) SEQUENCE DESCRIPTION :SEQ ID NO: 104:
   CTGGAGACAT ATAACTTGAA CACTTGGCCC TGATGGGGAA GCAGCTCTGC AGGGACTTTT    60
   TCAGCCATCT GTAAACAATT TCAGTGGCAA CCCGCGAACT GTAATCCATG AATGGGACCA   120
   CACTTTACAA GTCATCAAGT CTAACTTCTA GACCAGGGAA TTAATGGGGG AGACAGCGAA   180
   CCCTAGAGCA AAGTGCCAAA CTTCTGTCGA TAGCTTGAGG CTAGTGGAAA GACCTCGAGG   240
   AGGCTACTCC AGAAGTTCAG CGCGTAGGAA GCTCCGATAC CAATAGCCCT TTGATGATGG   300
   TGGGGTTGGT GAAGGGAACA GTGCTCCGCA AGGTTATCCC TGCCCCAGGC AGTCCAATTT   360
   TCACTCTGCA GATTCTCTCT GGCTCTAACT ACCCCAGATA ACAAGGAGTG AATGCAGAAT   420
   AGCACGGGCT TTAGGGCCAA TCAGACATTA GTTAGAAAAA TTCCTACTAC ATGGTTTATG   480
   TAAACTTGAA GATGAATGAT TGCGAACTCC CCGAAAAGGG CTCAGACAAT GCCATGCATA   540
   AAGAGGGGCC CTGTAATTTG AGGTTTCAGA ACCCGAAGTG AAGGGGTCAG GCAGCCGGGT   600
   ACGGCGGAAA CTCACAGCTT TCGCCCAGCG AGAGGACAAA GGTCTGGGAC ACACTCCAAC   660
   TGCGTCCGGA TCTTGGCTGG ATCGGACTCT CAGGGTGGAG GAGACACAAG CACAGCAGCT   720
   GCCCAGCGTG TGCCCAGCCC TCCCACCGCT GGTCCCGGCT GCCAGGAGGC TGGCCGCTGG   780
   CGGGAAGGGG CCGGGAAACC TCAGAGCCCC GCGGAGACAG CAGCCGCCTT GTTCCTCAGC   840
   CCGGTGGCTT TTTTTTCCCC TGCTCTCCCA GGGGACAGAC ACCACCGCCC CACCCCTCAC   900
   GCCCCACCTC CCTGGGGGAT CCTTTCCGCC CCAGCCCTGA AAGCGTTAAT CCTGGAGCTT   960
   TCTGCACACC CCCCGACCGC TCCCGCCCAA GCTTCCTAAA AAAGAAAGGT GCAAAGTTTG  1020
   GTCCAGGATA GAAAAATGAC TGATCAAAGG CAGGCGATAC TTCCTGTTGC CGGGACGCTA  1080
   TATATAACGT GATGAGCGCA CGGGCTGCGG AGACGCACCG GAGCGCTCGC CCAGCCGCCG  1140
   CCTCCAAGCC CCTGAGGTTT CCGGGGACCA CA ATG AAC AAG TTG CTG TGC TGC    1193
                                       Met Asn Lys Leu Leu Cys Cys
                                           -20                 -15
   GCG CTC GTG GTAAGTCCCT GGGCCAGCCG ACGGGTGCCC GGCGCCTGGG            1242
   Ala Leu Val
   GAGGCTGCTG CCACCTGGTC TCCCAACCTC CCAGCGGACC GGCGGGGAAA AAGGCTCCAC  1302
   TCGCTCCCTC CCAAG                                                   1317
(2) INFORMATION FOR SEQUENCE ID NO: 105:
   (i) SEQUENCE CHARACTERISTICS:
      (A) LENGTH : 10190 base pairs
      (B) TYPE : nucleic acid
      (C) STRANDEDNESS : double
      (D) TOPOLOGY : linear
   (ii) MOLECULE TYPE : human OCIF genomic DNA-2
   (xi) SEQUENCE DESCRIPTION :SEQ ID NO: 105:
   GCTTACTTTG TGCCAAATCT CATTAGGCTT AAGGTAATAC AGGACTTTGA GTCAAATGAT     60
   ACTGTTGCAC ATAAGAACAA ACCTATTTTC ATGCTAAGAT GATGCCACTG TGTTCCTTTC    120
   TCCTTCTAG TTT CTG GAC ATC TCC ATT AAG TGG ACC ACC CAG GAA ACG TTT    171
             Phe Leu Asp Ile Ser Ile Lys Trp Thr Thr Gln Glu Thr Phe
                 -10                 -5              -1   1
   CCT CCA AAG TAC CTT CAT TAT GAC GAA GAA ACC TCT CAT CAG CTG TTG      219
   Pro Pro Lys Tyr Leu His Tyr Asp Glu Glu Thr Ser His Gln Leu Leu
         5                  10                  15
   TGT GAC AAA TGT CCT CCT GGT ACC TAC CTA AAA CAA CAC TGT ACA GCA      267
   Cys Asp Lys Cys Pro Pro Gly Thr Tyr Leu Lys Gln His Cys Thr Ala
    20                  25                  30                  35
   AAG TGG AAG ACC GTG TGC GCC CCT TGC CCT GAC CAC TAC TAC ACA GAC      315
   Lys Trp Lys Thr Val Cys Ala Pro Cys Pro Asp His Tyr Tyr Thr Asp
                    40                  45                  50
   AGC TGG CAC ACC AGT GAC GAG TGT CTA TAC TGC AGC CCC GTG TGC AAG      363
   Ser Trp His Thr Ser Asp Glu Cys Leu Tyr Cys Ser Pro Val Cys Lys
                55                  60                  65
   GAG CTG CAG TAC GTC AAG CAG GAG TGC AAT CGC ACC CAC AAC CGC GTG      411
   Glu Leu Gln Tyr Val Lys Gln Glu Cys Asn Arg Thr His Asn Arg Val
            70                        75                        80
   TGC GAA TGC AAG GAA GGG CGC TAC CTT GAG ATA GAG TTC TGC TTG AAA      459
   Cys Glu Cys Lys Glu Gly Arg Tyr Leu Glu Ile Glu Phe Cys Leu Lys
        85                        90                        95
   CAT AGG AGC TGC CCT CCT GGA TTT GGA GTG GTG CAA GCT G GTACGTGTCA     509
   His Arg Ser Cys Pro Pro Gly Phe Gly Val Val Gln Ala
   100                    105                       110
   ATGTGCAGCA AAATTAATTA GGATCATGCA AAGTCAGATA GTTGTGACAG TTTAGGAGAA    569
   CACTTTTGTT CTGATGACAT TATAGGATAG CAAATTGCAA AGGTAATGAA ACCTGCCAGG    629
   TAGGTACTAT GTGTCTGGAG TGCTTCCAAA GGACCATTGC TCAGAGGAAT ACTTTGCCAC    689
   TACAGGGCAA TTTAATGACA AATCTCAAAT GCAGCAAATT ATTCTCTCAT GAGATGCATG    749
   ATGGTTTTTT TTTTTTTTTT TAAAGAAACA AACTCAAGTT GCACTATTGA TAGTTGATCT    809
   ATACCTCTAT ATTTCACTTC AGCATGGACA CCTTCAAACT GCAGCACTTT TTGACAAACA    869
   TCAGAAATGT TAATTTATAC CAAGAGAGTA ATTATGCTCA TATTAATGAG ACTCTGGAGT    929
   GCTAACAATA AGCAGTTATA ATTAATTATG TAAAAAATGA GAATGGTGAG GGGAATTGCA    989
   TTTCATTATT AAAAACAAGG CTAGTTCTTC CTTTAGCATG GGAGCTGAGT GTTTGGGAGG   1049
   GTAAGGACTA TAGCAGAATC TCTTCAATGA GCTTATTCTT TATCTTAGAC AAAACAGATT   1109
   GTCAAGCCAA GAGCAAGCAC TTGCCTATAA ACCAAGTGCT TTCTCTTTTG CATTTTGAAC   1169
   AGCATTGGTC AGGGCTCATG TGTATTGAAT CTTTTAAACC AGTAACCCAC GTTTTTTTTC   1229
   TGCCACATTT GCGAAGCTTC AGTGCAGCCT ATAACTTTTC ATAGCTTGAG AAAATTAAGA   1289
   GTATCCACTT ACTTAGATGG AAGAAGTAAT CAGTATAGAT TCTGATGACT CAGTTTGAAG   1349
   CAGTGTTTCT CAACTGAAGC CCTGCTGATA TTTTAAGAAA TATCTGGATT CCTAGGCTGG   1409
   ACTCCTTTTT GTGGGCAGCT GTCCTGCGCA TTGTAGAATT TTGGCAGCAC CCCTGGACTC   1469
   TAGCCACTAG ATACCAATAG CAGTCCTTCC CCCATGTGAC AGCCAAAAAT GTCTTCAGAC   1529
   ACTGTCAAAT GTCGCCAGGT GGCAAAATCA CTCCTGGTTG AGAACAGGGT CATCAATGCT   1589
   AAGTATCTGT AACTATTTTA ACTCTCAAAA CTTGTGATAT ACAAAGTCTA AATTATTAGA   1649
   CGACCAATAC TTTAGGTTTA AAGGCATACA AATGAAACAT TCAAAAATCA AAATCTATTC   1709
   TGTTTCTCAA ATAGTGAATC TTATAAAATT AATCACAGAA GATGCAAATT GCATCAGAGT   1769
   CCCTTAAAAT TCCTCTTCGT ATGAGTATTT GAGGGAGGAA TTGGTGATAG TTCCTACTTT   1829
   CTATTGGATG GTACTTTGAG ACTCAAAAGC TAAGCTAAGT TGTGTGTGTG TCAGGGTGCG   1889
   GGGTGTGGAA TCCCATCAGA TAAAAGCAAA TCCATGTAAT TCATTCAGTA AGTTGTATAT   1949
   GTAGAAAAAT GAAAAGTGGG CTATGCAGCT TGGAAACTAG AGAATTTTGA AAAATAATGG
   2009
   AAATCACAAG GATCTTTCTT AAATAAGTAA GAAAATCTGT TTGTAGAATG AAGCAAGCAG   2069
   GCAGCCAGAA GACTCAGAAC AAAAGTACAC ATTTTACTCT GTGTACACTG GCAGCACAGT   2129
   GGGATTTATT TACCTCTCCC TCCCTAAAAA CCCACACAGC GGTTCCTCTT GGGAAATAAG   2189
   AGGTTTCCAG CCCAAAGAGA AGGAAAGACT ATGTGGTGTT ACTCTAAAAA GTATTTAATA   2249
   ACCGTTTTGT TGTTGCTGTT GCTGTTTTGA AATCAGATTG TCTCCTCTCC ATATTTTATT   2309
   TACTTCATTC TGTTAATTCC TGTGGAATTA CTTAGAGCAA GCATGGTGAA TTCTCAACTG   2369
   TAAAGCCAAA TTTCTCCATC ATTATAATTT CACATTTTGC CTGGCAGGTT ATAATTTTTA   2429
   TATTTCCACT GATAGTAATA AGGTAAAATC ATTACTTAGA TGGATAGATC TTTTTCATAA   2489
   AAAGTACCAT CAGTTATAGA GGGAAGTCAT GTTCATGTTC AGGAAGGTCA TTAGATAAAG   2549
   CTTCTGAATA TATTATGAAA CATTAGTTCT GTCATTCTTA GATTCTTTTT GTTAAATAAC   2609
   TTTAAAAGCT AACTTACCTA AAAGAAATAT CTGACACATA TGAACTTCTC ATTAGGATGC   2669
   AGGAGAAGAC CCAAGCCACA GATATGTATC TGAAGAATGA ACAAGATTCT TAGGCCCGGC
   2729
   ACGGTGGCTC ACATCTGTAA TCTCAAGAGT TTGAGAGGTC AAGGCGGGCA GATCACCTGA   2789
   GGTCAGGAGT TCAAGACCAG CCTGGCCAAC ATGATGAAAC CCTGCCTCTA CTAAAAATAC   2849
   AAAAATTAGC AGGGCATGGT GGTGCATGCC TGCAACCCTA GCTACTCAGG AGGCTGAGAC   2909
   AGGAGAATCT CTTGAACCCT CGAGGCGGAG GTTGTGGTGA GCTGAGATCC CTCTACTGCA   2969
   CTCCAGCCTG GGTGACAGAG ATGAGACTCC GTCCCTGCCG CCGCCCCCGC CTTCCCCCCC   3029
   AAAAAGATTC TTCTTCATGC AGAACATACG GCAGTCAACA AAGGGAGACC TGGGTCCAGG
   3089
   TGTCCAAGTC ACTTATTTCG AGTAAATTAG CAATGAAAGA ATGCCATGGA ATCCCTGCCC   3149
   AAATACCTCT GCTTATGATA TTGTAGAATT TGATATAGAG TTGTATCCCA TTTAAGGAGT   3209
   AGGATGTAGT AGGAAAGTAC TAAAAACAAA CACACAAACA GAAAACCCTC TTTGCTTTGT   3269
   AAGGTGGTTC CTAAGATAAT GTCAGTGCAA TGCTGGAAAT AATATTTAAT ATGTGAAGGT   3329
   TTTAGGCTGT GTTTTCCCCT CCTGTTCTTT TTTTCTGCCA GCCCTTTGTC ATTTTTGCAG   3389
   GTCAATGAAT CATGTAGAAA GAGACAGGAG ATGAAACTAG AACCAGTCCA TTTTGCCCCT   3449
   TTTTTTATTT TCTGGTTTTG GTAAAAGATA CAATGAGGTA GGAGGTTGAG ATTTATAAAT   3509
   GAAGTTTAAT AAGTTTCTGT AGCTTTGATT TTTCTCTTTC ATATTTGTTA TCTTGCATAA   3569
   GCCAGAATTG GCCTGTAAAA TCTACATATG GATATTGAAG TCTAAATCTG TTCAACTAGC   3629
   TTACACTAGA TGGAGATATT TTCATATTCA GATACACTGG AATGTATGAT CTAGCCATGC   3689
   GTAATATAGT CAAGTGTTTG AAGGTATTTA TTTTTAATAG CGTCTTTAGT TGTGGACTGG   3749
   TTCAAGTTTT TCTGCCAATG ATTTCTTCAA ATTTATCAAA TATTTTTCCA TCATGAAGTA   3809
   AAATGCCCTT GCAGTCACCC TTCCTGAAGT TTGAACGACT CTGCTGTTTT AAACAGTTTA   3869
   AGCAAATGGT ATATCATCTT CCGTTTACTA TGTAGCTTAA CTGCAGGCTT ACGCTTTTGA   3929
   GTCAGCGGCC AACTTTATTG CCACCTTCAA AAGTTTATTA TAATGTTGTA AATTTTTACT   3989
   TCTCAAGGTT AGCATACTTA GGAGTTGCTT CACAATTAGG ATTCAGGAAA GAAAGAACTT   4049
   CAGTAGGAAC TGATTGGAAT TTAATGATGC AGCATTCAAT GGGTACTAAT TTCAAAGAAT   4109
   GATATTACAG CAGACACACA GCAGTTATCT TGATTTTCTA GGAATAATTG TATGAAGAAT   4169
   ATGGCTGACA ACACGGCCTT ACTGCCACTC AGCGGAGGCT GGACTAATGA ACACCCTACC   4229
   CTTCTTTCCT TTCCTCTCAC ATTTCATGAG CGTTTTGTAG GTAACGAGAA AATTGACTTG   4289
   CATTTGCATT ACAAGGAGGA GAAACTGGCA AAGGGGATGA TGGTGGAAGT TTTGTTCTGT   4349
   CTAATGAAGT GAAAAATGAA AATGCTAGAG TTTTGTGCAA CATAATAGTA GCAGTAAAAA   4409
   CCAAGTGAAA AGTCTTTCCA AAACTGTGTT AAGAGGGCAT CTGCTGGGAA ACGATTTGAG   4469
   GAGAAGGTAC TAAATTGCTT GGTATTTTCC GTAG GA ACC CCA GAG CGA AAT ACA    4523
                                         Gly Thr Pro Glu Arg Asn Thr
                                                     115
   GTT TGC AAA AGA TGT CCA GAT GGG TTC TTC TCA AAT GAG ACG TCA TCT      4571
   Val Cys Lys Arg Cys Pro Asp Gly Phe Phe Ser Asn Glu Thr Ser Ser
   120                 125                 130                 135
   AAA GCA CCC TGT AGA AAA CAC ACA AAT TGC AGT GTC TTT GGT CTC CTG      4619
   Lys Ala Pro Cys Arg Lys His Thr Asn Cys Ser Val Phe Gly Leu Leu
                   140                 145                 150
   CTA ACT CAG AAA GGA AAT GCA ACA CAC GAC AAC ATA TGT TCC GGA AAC      4667
   Leu Thr Gln Lys Gly Asn Ala Thr His Asp Asn Ile Cys Ser Gly Asn
               155                 160                 165
   AGT GAA TCA ACT CAA AAA TGT GGA ATA G GTAATTACAT TCCAAAATAC         4715
   Ser Glu Ser Thr Gln Lys Cys Gly Ile
           170                 175
   GTCTTTGTAC GATTTTGTAG TATCATCTCT CTCTCTGAGT TGAACACAAG GCCTCCAGCC   4775
   ACATTCTTGG TCAAACTTAC ATTTTCCCTT TCTTGAATCT TAACCAGCTA AGGCTACTCT   4835
   CGATGCATTA CTGCTAAAGC TACCACTCAG AATCTCTCAA AAACTCATCT TCTCACAGAT   4895
   AACACCTCAA AGCTTGATTT TCTCTCCTTT CACACTGAAA TCAAATCTTG CCCATAGGCA   4955
   AAGGGCAGTG TCAAGTTTGC CACTGAGATG AAATTAGGAG AGTCCAAACT GTAGAATTCA   5015
   CGTTGTGTGT TATTACTTTC ACGAATGTCT GTATTATTAA CTAAAGTATA TATTGGCAAC   5075
   TAAGAAGCAA AGTGATATAA ACATGATGAC AAATTAGGCC AGGCATGGTG GCTTACTCCT   5135
   ATAATCCCAA CATTTTGGGG GGCCAAGGTA GGCAGATCAC TTGAGGTCAG GATTTCAAGA   5195
   CCAGCCTGAC CAACATGGTG AAACCTTGTC TCTACTAAAA ATACAAAAAT TAGCTGGGCA   5255
   TGGTAGCAGG CACTTCTAGT ACCAGCTACT CAGGGCTGAG GCAGGAGAAT CGCTTGAACC   5315
   CAGGAGATGG AGGTTGCAGT GAGCTGAGAT TGTACCACTG CACTCCAGTC TGGGCAACAG   5375
   AGCAAGATTT CATCACACAC ACACACACAC ACACACACAC ACACATTAGA AATGTGTACT   5435
   TGGCTTTGTT ACCTATGGTA TTAGTGCATC TATTGCATGG AACTTCCAAG CTACTCTGGT   5495
   TGTGTTAAGC TCTTCATTGG GTACAGGTCA CTAGTATTAA GTTCAGGTTA TTCGGATGCA   5555
   TTCCACGGTA GTGATGACAA TTCATCAGGC TAGTGTGTGT GTTCACCTTG TCACTCCCAC   5615
   CACTAGACTA ATCTCAGACC TTCACTCAAA GACACATTAC ACTAAAGATG ATTTGCTTTT   5675
   TTGTGTTTAA TCAAGCAATG GTATAAACCA GCTTGACTCT CCCCAAACAG TTTTTCGTAC   5735
   TACAAAGAAG TTTATGAAGC AGAGAAATGT GAATTGATAT ATATATGAGA TTCTAACCCA   5795
   GTTCCAGCAT TGTTTCATTG TGTAATTGAA ATCATAGACA AGCCATTTTA GCCTTTGCTT   5855
   TCTTATCTAA AAAAAAAAAA AAAAAAATGA AGGAAGGGGT ATTAAAAGGA GTGATCAAAT
   5915
   TTTAACATTC TCTTTAATTA ATTCATTTTT AATTTTACTT TTTTTCATTT ATTGTGCACT   5975
   TACTATGTGG TACTGTGCTA TAGAGGCTTT AACATTTATA AAAACACTGT GAAAGTTGCT   6035
   TCAGATGAAT ATAGGTAGTA GAACGGCAGA ACTAGTATTC AAAGCCAGGT CTGATGAATC   6095
   CAAAAACAAA CACCCATTAC TCCCATTTTC TGGGACATAC TTACTCTACC CAGATGCTCT   6155
   GGGCTTTGTA ATGCCTATGT AAATAACATA GTTTTATGTT TGGTTATTTT CCTATGTAAT   6215
   GTCTACTTAT ATATCTGTAT CTATCTCTTG CTTTGTTTCC AAAGGTAAAC TATGTGTCTA   6275
   AATGTGGGCA AAAAATAACA CACTATTCCA AATTACTGTT CAAATTCCTT TAAGTCAGTG   6335
   ATAATTATTT GTTTTGACAT TAATCATGAA GTTCCCTGTG GGTACTAGGT AAACCTTTAA   6395
   TAGAATGTTA ATGTTTGTAT TCATTATAAG AATTTTTGGC TGTTACTTAT TTACAACAAT   6455
   ATTTCACTCT AATTAGACAT TTACTAAACT TTCTCTTGAA AACAATGCCC AAAAAAGAAC   6515
   ATTAGAAGAC ACGTAAGCTC AGTTGGTCTC TGCCACTAAG ACCAGCCAAC AGAAGCTTGA   6575
   TTTTATTCAA ACTTTGCATT TTAGCATATT TTATCTTGGA AAATTCAATT GTGTTGGTTT   6635
   TTTGTTTTTG TTTGTATTGA ATAGACTCTC AGAAATCCAA TTGTTGAGTA AATCTTCTGG   6695
   GTTTTCTAAC CTTTCTTTAG AT GTT ACC CTG TGT GAG GAG GCA TTC TTC AGG   6747
                            Asp Val Thr Leu Cys Glu Glu Ala Phe Phe Arg
                                        180                 185
   TTT GCT GTT CCT ACA AAG TTT ACG CCT AAC TGG CTT AGT GTC TTG GTA    6795
   Phe Ala Val Pro Thr Lys Phe Thr Pro Asn Trp Leu Ser Val Leu Val
           190                 195                 200
   GAC AAT TTG CCT GGC ACC AAA GTA AAC GCA GAG AGT GTA GAG AGG ATA    6843
   Asp Asn Leu Pro Gly Thr Lys Val Asn Ala Glu Ser Val Glu Arg Ile
       205                 210                 215
   AAA CGG CAA CAC AGC TCA CAA GAA CAG ACT TTC CAG CTG CTG AAG TTA    6891
   Lys Arg Gln His Ser Ser Gln Glu Gln Thr Phe Gln Leu Leu Lys Leu
   220                 225                 230                 235
   TGG AAA CAT CAA AAC AAA GAC CAA GAT ATA GTC AAG AAG ATC ATC CAA G  6940
   Trp Lys His Gln Asn Lys Asp Gln Asp Ile Val Lys Lys Ile Ile Gln
                   240                 245                 250
   GTAATTACAT TCCAAAATAC GTCTTTGTAC GATTTTGTAG TATCATCTCT CTCTCTGAGT   7000
   TGAACACAAG GCCTCCAGCC ACATTCTTGG TCAAACTTAC ATTTTCCCTT TCTTGAATCT   7060
   TAACCAGCTA AGGCTACTCT CGATGCATTA CTGCTAAAGC TACCACTCAG AATCTCTCAA   7120
   AAACTCATCT TCTCACAGAT AACACCTCAA AGCTTGATTT TCTCTCCTTT CACACTGAAA   7180
   TCAAATCTTG CCCATAGGCA AAGGGCAGTG TCAAGTTTGC CACTGAGATG AAATTAGGAG   7240
   AGTCCAAACT GTAGAATTCA CGTTGTGTGT TATTACTTTC ACGAATGTCT GTATTATTAA   7300
   CTAAAGTATA TATTGGCAAC TAAGAAGCAA AGTGATATAA ACATGATGAC AAATTAGGCC   7360
   AGGCATGGTG GCTTACTCCT ATAATCCCAA CATTTTGGGG GGCCAAGGTA GGCAGATCAC   7420
   TTGAGGTCAG GATTTCAAGA CCAGCCTGAC CAACATGGTG AAACCTTGTC TCTACTAAAA   7480
   ATACAAAAAT TAGCTGGGCA TGGTAGCAGG CACTTCTAGT ACCAGCTACT CAGGGCTGAG   7540
   GCAGGAGAAT CGCTTGAACC CAGGAGATGG AGGTTGCAGT GAGCTGAGAT TGTACCACTG
   7600
   CACTCCAGTC TGGGCAACAG AGCAAGATTT CATCACACAC ACACACACAC ACACACACAC   7660
   ACACATTAGA AATGTGTACT TGGCTTTGTT ACCTATGGTA TTAGTGCATC TATTGCATGG   7720
   AACTTCCAAG CTACTCTGGT TGTGTTAAGC TCTTCATTGG GTACAGGTCA CTAGTATTAA   7780
   GTTCAGGTTA TTCGGATGCA TTCCACGGTA GTGATGACAA TTCATCAGGC TAGTGTGTGT   7840
   GTTCACCTTG TCACTCCCAC CACTAGACTA ATCTCAGACC TTCACTCAAA GACACATTAC   7900
   ACTAAAGATG ATTTGCTTTT TTGTGTTTAA TCAAGCAATG GTATAAACCA GCTTGACTCT   7960
   CCCCAAACAG TTTTTCGTAC TACAAAGAAG TTTATGAAGC AGAGAAATGT GAATTGATAT   8020
   ATATATGAGA TTCTAACCCA GTTCCAGCAT TGTTTCATTG TGTAATTGAA ATCATAGACA   8080
   AGCCATTTTA GCCTTTGCTT TCTTATCTAA AAAAAAAAAA AAAAAAATGA AGGAAGGGGT   8140
   ATTAAAAGGA GTGATCAAAT TTTAACATTC TCTTTAATTA ATTCATTTTT AATTTTACTT   8200
   TTTTTCATTT ATTGTGCACT TACTATGTGG TACTGTGCTA TAGAGGCTTT AACATTTATA   8260
   AAAACACTGT GAAAGTTGCT TCAGATGAAT ATAGGTAGTA GAACGGCAGA ACTAGTATTC   8320
   AAAGCCAGGT CTGATGAATC CAAAAACAAA CACCCATTAC TCCCATTTTC TGGGACATAC   8380
   TTACTCTACC CAGATGCTCT GGGCTTTGTA ATGCCTATGT AAATAACATA GTTTTATGTT   8440
   TGGTTATTTT CCTATGTAAT GTCTACTTAT ATATCTGTAT CTATCTCTTG CTTTGTTTCC   8500
   AAAGGTAAAC TATGTGTCTA AATGTGGGCA AAAAATAACA CACTATTCCA AATTACTGTT   8560
   CAAATTCCTT TAAGTCAGTG ATAATTATTT GTTTTGACAT TAATCATGAA GTTCCCTGTG   8620
   GGTACTAGGT AAACCTTTAA TAGAATGTTA ATGTTTGTAT TCATTATAAG AATTTTTGGC   8680
   TGTTACTTAT TTACAACAAT ATTTCACTCT AATTAGACAT TTACTAAACT TTCTCTTGAA   8740
   AACAATGCCC AAAAAAGAAC ATTAGAAGAC ACGTAAGCTC AGTTGGTCTC TGCCACTAAG   8800
   ACCAGCCAAC AGAAGCTTGA TTTTATTCAA ACTTTGCATT TTAGCATATT TTATCTTGGA   8860
   AAATTCAATT GTGTTGGTTT TTTGTTTTTG TTTGTATTGA ATAGACTCTC AGAAATCCAA   8920
   TTGTTGAGTA AATCTTCTGG GTTTTCTAAC CTTTCTTTAG AT ATT GAC CTC TGT     8974
                                               Asp Ile Asp Leu Cys
                                                           255
   GAA AAC AGC GTG CAG CGG CAC ATT GGA CAT GCT AAC CTC ACC TTC GAG    9022
   Glu Asn Ser Val Gln Arg His Ile Gly His Ala Asn Leu Thr Phe Glu
               260                 265                 270
   CAG CTT CGT AGC TTG ATG GAA AGC TTA CCG GGA AAG AAA GTG GGA GCA     9070
   Gln Leu Arg Ser Leu Met Glu Ser Leu Pro Gly Lys Lys Val Gly Ala
           275                 280                 285
   GAA GAC ATT GAA AAA ACA ATA AAG GCA TGC AAA CCC AGT GAC CAG ATC     9118
   Glu Asp Ile Glu Lys Thr Ile Lys Ala Cys Lys Pro Ser Asp Gln Ile
       290                 295                 300
   CTG AAG CTG CTC AGT TTG TGG CGA ATA AAA AAT GGC GAC CAA GAC ACC     9166
   Leu Lys Leu Leu Ser Leu Trp Arg Ile Lys Asn Gly Asp Gln Asp Thr
   305                 310                 315                 320
   TTG AAG GGC CTA ATG CAC GCA CTA AAG CAC TCA AAG ACG TAC CAC TTT     9214
   Leu Lys Gly Leu Met His Ala Leu Lys His Ser Lys Thr Tyr His Phe
                   325                 330                 335
   CCC AAA ACT GTC ACT CAG AGT CTA AAG AAG ACC ATC AGG TTC CTT CAC     9262
   Pro Lys Thr Val Thr Gln Ser Leu Lys Lys Thr Ile Arg Phe Leu His
               340                 345                 350
   AGC TTC ACA ATG TAC AAA TTG TAT CAG AAG TTA TTT TTA GAA ATG ATA     9310
   Ser Phe Thr Met Tyr Lys Leu Tyr Gln Lys Leu Phe Leu Glu Met Ile
           355                 360                 365
   GGT AAC CAG GTC CAA TCA GTA AAA ATA AGC TGC TTA TAACTGGAAA          9356
   Gly Asn Gln Val Gln Ser Val Lys Ile Ser Cys Leu
       370                 375                 380
   TGGCCATTGA GCTGTTTCCT CACAATTGGC GAGATCCCAT GGATGAGTAA ACTGTTTCTC   9416
   AGGCACTTGA GGCTTTCAGT GATATCTTTC TCATTACCAG TGACTAATTT TGCCACAGGG   9476
   TACTAAAAGA AACTATGATG TGGAGAAAGG ACTAACATCT CCTCCAATAA ACCCCAAATG   9536
   GTTAATCCAA CTGTCAGATC TGGATCGTTA TCTACTGACT ATATTTTCCC TTATTACTGC   9596
   TTGCAGTAAT TCAACTGGAA ATTAAAAAAA AAAAACTAGA CTCCACTGGG CCTTACTAAA   9656
   TATGGGAATG TCTAACTTAA ATAGCTTTGG GATTCCAGCT ATGCTAGAGG CTTTTATTAG   9716
   AAAGCCATAT TTTTTTCTGT AAAAGTTACT AATATATCTG TAACACTATT ACAGTATTGC   9776
   TATTTATATT CATTCAGATA TAAGATTTGG ACATATTATC ATCCTATAAA GAAACGGTAT   9836
   GACTTAATTT TAGAAAGAAA ATTATATTCT GTTTATTATG ACAAATGAAA GAGAAAATAT   9896
   ATATTTTTAA TGGAAAGTTT GTAGCATTTT TCTAATAGGT ACTGCCATAT TTTTCTGTGT   9956
   GGAGTATTTT TATAATTTTA TCTGTATAAG CTGTAATATC ATTTTATAGA AAATGCATTA  10016
   TTTAGTCAAT TGTTTAATGT TGGAAAACAT ATGAAATATA AATTATCTGA ATATTAGATG  10076
   CTCTGAGAAA TTGAATGTAC CTTATTTAAA AGATTTTATG GTTTTATAAC TATATAAATG   10136
   ACATTATTAA AGTTTTCAAA TTATTTTTTA TTGCTTTCTC TGTTGCTTTT ATTT        10190

## Claims

1. An OCIF protein **characterised by** the following properties:
a) molecular weight on SDS-polyacrylamide gel electrophoresis (SDS-PAGE) :
approximately 60 kD under reducing conditions;
approximately 60 kD and 120 kD under non-reducing conditions;
b) a high affinity to cation-exchange column and heparin column;
c) a biological activity to inhibit osteoclast differentiation and/or maturation
its activity being decreased by heating at 70°C for 10 min or at 56°C for 30 min;
its activity being lost by heating at 90°C for 10 min;
d) internal amino acid sequences provided by SEQ ID NOs 1,2 and 3.

2. A protein according to claim 1 having N-terminal amino acid sequences provided in SEQ ID NO 7.

3. A protein according to claim 1 produced in human fibroblasts.

4. A method of producing a protein according to claim 1, 2 or 3 by the following process:
cultivating human fibroblasts; and purifying the protein by a combination of ion-exchange column, affinity column and reverse phase-column chromatography.

5. A method according to claim 4 by cultivating human fibroblasts on alumina ceramic pieces.

6. OCIF protein with amino acid sequence as provided in SEQ ID NO 4.

7. OCIF cDNA encoding amino acid sequence as provided in SEQ ID NO 4.

8. OCIF cDNA encoding amino acid sequence as provided in SEQ ID No.5

9. OCIF cDNA which is inserted in a vector as 1.6 kb fragment carried by the transformed Escherichia coli pBK/01F10 (FERM BP-5267).

10. OCIF cDNA with nucleotide sequence as provided in SEQ ID NO 6.

11. OCIF cDNA that hybridise to cDNA as provided in SEQ ID NO 6 under stringent conditions.

12. An OCIF protein expressed from cDNA encoding amino acid sequence as provided in SEQ ID NO 4.

13. An OCIF protein with a biological activity to inhibit osteoclast differentiation and/or maturation, that is obtained as amino acid expressed cDNA sharing at least 80% sequence identity with the amino acid as provided in SEQ ID NO 4.

14. A method for the production of a protein according to claim 1 by genetic engineering using cDNA of any of claims 7 to 11.

15. The protein according to claim 1, said protein is obtainable by the method of claim 14.

16. A method of producing the protein according to claim 12 by gene engineering using mammalian cells as host cells.

17. A method of producing the protein according to claim 16 by gene engineering using 293/EBNA cells or CHO cells as mammalian host cells.

18. The protein according to claim 6 or 12, said protein is obtainable by the method of claim 16 or 17.

19. An OCIF variant, mutant or truncated cDNA selected from:
a cDNA with nucleotide sequence as provided in SEQ ID NO 8;
a cDNA with nucleotide sequence as provided in SEQ ID NO 10;
a cDNA with nucleotide sequence as provided in SEQ ID NO 12;
a cDNA with nucleotide sequence as provided in SEQ ID NO 14;
a cDNA with nucleotide sequence as provided in SEQ ID NO 83;
a cDNA with nucleotide sequence as provided in SEQ ID NO 84;
a cDNA with nucleotide sequence as provided in SEQ ID NO 85;
a cDNA with nucleotide sequence as provided in SEQ ID NO 86;
a cDNA with nucleotide sequence as provided in SEQ ID NO 87;
a cDNA with nucleotide sequence as provided in SEQ ID NO 88;
a cDNA with nucleotide sequence as provided in SEQ ID NO 89;
a cDNA with nucleotide sequence as provided in SEQ ID NO 90;
a cDNA with nucleotide sequence as provided in SEQ ID NO 91;
a cDNA with nucleotide sequence as provided in SEQ ID NO 92;
a cDNA with nucleotide sequence as provided in SEQ ID NO 93;
a cDNA with nucleotide sequence as provided in SEQ ID NO 94;
a cDNA with nucleotide sequence as provided in SEQ ID NO 95;
a cDNA with nucleotide sequence as provided in SEQ ID NO 96;
a cDNA with nucleotide sequence as provided in SEQ ID NO 97;
a cDNA with nucleotide sequence as provided in SEQ ID NO 98;
a cDNA with nucleotide sequence as provided in SEQ ID NO 99;
a cDNA with nucleotide sequence as provided in SEQ ID NO 100;
a cDNA with nucleotide sequence as provided in SEQ ID NO 101;
a cDNA with nucleotide sequence as provided in SEQ ID NO 102; and
a cDNA with nucleotide sequence as provided in SEQ ID NO 103.

20. A protein encoded by a cDNA selected from those of claim 19.

21. An OCIF variant, mutant or truncated cDNA selected from:
cDNA encoding amino acid sequence as provided in SEQ ID NO 9;
cDNA encoding amino acid sequence as provided in SEQ ID NO 11;
cDNA encoding amino acid sequence as provided in SEQ ID NO 13;
cDNA encoding amino acid sequence as provided in SEQ ID NO 15;
cDNA encoding amino acid sequence as provided in SEQ ID NO 62;
cDNA encoding amino acid sequence as provided in SEQ ID NO 63;
cDNA encoding amino acid sequence as provided in SEQ ID NO 64;
cDNA encoding amino acid sequence as provided in SEQ ID NO 65;
cDNA encoding amino acid sequence as provided in SEQ ID NO 66;
cDNA encoding amino acid sequence as provided in SEQ ID NO 67;
cDNA encoding amino acid sequence as provided in SEQ ID NO 68;
cDNA encoding amino acid sequence as provided in SEQ ID NO 69;
cDNA encoding amino acid sequence as provided in SEQ ID NO 70;
cDNA encoding amino acid sequence as provided in SEQ ID NO 71;
cDNA encoding amino acid sequence as provided in SEQ ID NO 72;
cDNA encoding amino acid sequence as provided in SEQ ID NO 73;
cDNA encoding amino acid sequence as provided in SEQ ID NO 74;
cDNA encoding amino acid sequence as provided in SEQ ID NO 75;
cDNA encoding amino acid sequence as provided in SEQ ID NO 76;
cDNA encoding amino acid sequence as provided in SEQ ID NO 77;
cDNA encoding amino acid sequence as provided in SEQ ID NO 78;
cDNA encoding amino acid sequence as provided in SEQ ID NO 79;
cDNA encoding amino acid sequence as provided in SEQ ID NO 80;
cDNA encoding amino acid sequence as provided in SEQ ID NO 81; and
cDNA encoding amino acid sequence as provided in SEQ ID NO 82.

22. Genomic OCIF DNA encoding amino acid sequence as provided in SEQ ID NO 5.

23. Genomic DNAs according to claim 22 with the nucleotide sequence as provided in SEQ ID NO 104 or 105.

24. A OCIF variant, mutant or truncated protein selected from:
a protein which has an amino acid sequence of amino acid numbers 1 to 373 of SEQ ID NO. 9;
a protein which has an amino acid sequence of amino acid numbers 1 to 341 of SEQ ID NO. 11;
a protein which has an amino acid sequence of amino acid numbers 1 to 133 of SEQ ID NO. 13;
a protein which has an amino acid sequence of amino acid numbers 1 to 124 of SEQ ID NO. 15;
a protein which has an amino acid sequence of amino acid numbers 1 to 380 of SEQ ID NO. 62;
a protein which has an amino acid sequence of amino acid numbers 1 to 380 of SEQ ID NO. 63;
a protein which has an amino acid sequence of amino acid numbers 1 to 380 of SEQ ID NO. 64;
a protein which has an amino acid sequence of amino acid numbers 1 to 380 of SEQ ID NO. 65;
a protein which has an amino acid sequence of amino acid numbers 1 to 380 of SEQ ID NO. 66;
a protein which has an amino acid sequence of amino acid numbers 1 to 339 of SEQ ID NO. 67;
a protein which has an amino acid sequence of amino acid numbers 1 to 338 of SEQ ID NO. 68;
a protein which has an amino acid sequence of amino acid numbers 1 to 342 of SEQ ID NO. 69;
a protein which has an amino acid sequence of amino acid numbers 1 to 338 of SEQ ID NO. 70;
a protein which has an amino acid sequence of amino acid numbers 1 to 305 of SEQ ID NO. 71;
a protein which has an amino acid sequence of amino acid numbers 1 to 306 of SEQ ID NO. 72;
a protein which has an amino acid sequence of amino acid numbers 1 to 378 of SEQ ID NO. 73;
a protein which has an amino acid sequence of amino acid numbers 1 to 330 of SEQ ID NO. 74;
a protein which has an amino acid sequence of amino acid numbers 1 to 251 of SEQ ID NO. 75;
a protein which has an amino acid sequence of amino acid numbers 1 to 176 of SEQ ID NO. 76;
a protein which has an amino acid sequence of amino acid numbers 1 to 122 of SEQ ID NO. 77;
a protein which has an amino acid sequence of amino acid numbers 1 to 85 of SEQ ID NO. 78;
a protein which has an amino acid sequence of amino acid numbers 1 to 372 of SEQ ID NO. 79;
a protein which has an amino acid sequence of amino acid numbers 1 to 300 of SEQ ID NO. 80;
a protein which has an amino acid sequence of amino acid numbers 1 to 166 of SEQ ID NO. 81; and
a protein which has an amino acid sequence of amino acid numbers 1 to 63 of SEQ ID NO. 82.

25. OCIF cDNA encoding a protein according to claim 24.

26. A pharmaceutical composition comprising a protein according to any of claims 1-3, 6, 12, 13, 15, 18, 20 and 24.

27. An antibody having specific activity to a protein according to any of claims 1-3, 6, 12-13, 15, 18, 20 and 24.

28. An antibody according to claim 27 that is a polyclonal antibody.

29. A antibody according to claim 27 that is a monoclonal antibody.

30. A monoclonal antibody according to claim 29 **characterized by** the following properties:
molecular weight of about 150,000 and of subclass IgG₁, IgG₂ₐ or IgG_{2b}·

31. A method of determining the concentration of the protein of the OCIF using the antibodies of any of claims 27 to 30.

32. Use of a protein according to any of claims 1-3, 6, 12, 13, 15, 18, 20 and 24 in the preparation of medicament for the treatment or prevention of osteoporosis.

33. Use of a protein according to any of claims 1-3, 6, 12, 13, 15, 18, 20 and 24 in the preparation of medicament for the treatment or prevention of decreased bone mass.

34. Use of a protein according to any of claims 1-3, 6, 12, 13, 15, 18, 20 and 24 in the preparation of medicament for the treatment or prevention of an abnormal bone metabolism.

35. A vector in which any of the following cDNA (a) to (d) is inserted:
(a) OCIF cDNA encoding amino acid sequence as provided in SEQ ID No. 4;
(b) OCIF cDNA with nucleotide sequence as provided in SEQ ID No. 6;
(c) OCIF cDNA encoding amino acid sequence as provided in SEQ ID No. 5;
(d) OCIF cDNA which is inserted in a vector as 1-6 Kb fragment carried by the transformed Escherichia coli pBK/01F10 (PERM BP-5267) and encodes a protein having a biological activity to inhibit osteoclast differentiation and/or maturation.

36. The vector of claim 35, said vector is carried by the transformed Escherichia coli pBK/01F10 (FERM BP-5267).

37. A host cell in which the vector according to claims 35 or 36 is introduced.

38. A method for the production of a protein according to claim 1, said method comprises steps (I) and (II);
(I) culturing the host cell according to claim 37;
(II) recovering said protein from the obtained culture.

39. A protein according to claim 1, said protein is obtainable by the method of claim 38.

40. The transformed Escherichia coli pBK/01F10 (PERM BP-5267) .

## Patentansprüche

1. OCIF-Protein, **gekennzeichnet durch** die folgenden Eigenschaften:
a) eine relative Molekülmasse nach SDS-Polyacrylamid-Gelelektrophorese (SDS-PAGE):
von etwa 60 kD unter reduzierenden Bedingungen;
von etwa 60 kD und 120 kD unter nichtreduzierenden Bedingungen;
b) eine hohe Affinität zur Kationenaustauschsäule und Heparinsäule;
c) eine biologische Aktivität zur Hemmung einer Osteoklastendifferenzierung und/oder -maturation,
wobei die Aktivität **durch** 10-minütiges Erwärmen auf 70°C oder 30-minütiges Erwärmen auf 56°C verringert wird;
die Aktivität bei einer 10-minütigen Erwärmung auf 90°C verloren geht;
d) interne Aminosäuresequenzen der SEQ ID Nr. 1, 2 und 3.

2. Protein nach Anspruch 1 mit N-terminalen Aminosäuresequenzen aus SEQ ID Nr. 7.

3. In humanen Fibroblasten produziertes Protein nach Anspruch 1.

4. Verfahren zur Herstellung eines Proteins nach Anspruch 1, 2 oder 3, umfassend den folgenden Prozess:
Kultivieren humaner Fibroblasten; und Reinigen des Proteins durch eine Kombination aus Ionenaustauschsäulen-, Affinitätssäulen- und Umkehrphasensäulenchromatographie.

5. Verfahren nach Anspruch 4, umfassend das Kultivieren humaner Fibroblasten auf Tonerde-Keramik-Stücken.

6. OCIF-Protein mit einer Aminosäuresequenz aus SEQ ID Nr. 4.

7. OCIF cDNA, die eine Aminsäuresequenz aus SEQ ID Nr. 4 kodiert.

8. OCIF cDNA, die eine Aminosäuresequenz aus SEQ ID Nr. 5 kodiert.

9. OCIF cDNA, die in einen Vektor als 1,6 Kb Fragment eingesetzt ist, das von transformiertem Escherichia coli pBK/01F10 (FERMBP-5267) getragen wird.

10. OCIF cDNA mit einer Nucleotidsequenz aus SEQ ID Nr. 6.

11. OCIF cDNA, die mit cDNA der SEQ ID Nr. 6 unter stringenten Bedingungen hybridisiert.

12. OCIF-Protein, exprimiert von cDNA, die eine Aminosäuresequenz der SEQ ID Nr. 4 kodiert.

13. OCIF-Protein mit einer biologischen Aktivität zur Hemmung einer Osteoklastendifferenzierung und/oder - maturation, das als aminosäureexprimierte cDNA erhalten wird, die zu wenigstens 80 % identisch mit der Aminosäuresequenz der SEQ ID Nr. 4 ist.

14. Gentechnisches Verfahren zur Herstellung eines Proteins nach Anspruch 1 unter Verwendung von cDNA nach einem der Ansprüche 7 bis 11.

15. Protein nach Anspruch 1, wobei das genannte Protein mit dem Verfahren aus Anspruch 14 erhältlich ist.

16. Gentechnisches Verfahren zur Herstellung des Proteins nach Anspruch 12 unter Verwendung von Säugetierzellen als Wirtszellen.

17. Gentechnisches Verfahren zur Herstellung des Proteins nach Anspruch 16 unter Verwendung von 293/EBNA-Zellen oder CHO-Zellen als Säugetierwirtszellen.

18. Protein nach Anspruch 6 oder 12, wobei das genannte Protein mit dem Verfahren aus Anspruch 16 oder 17 erhältlich ist.

19. OCIF-Variante, Mutante oder verkürzte cDNA, ausgewählt aus:
einer cDNA mit einer Nucleotidsequenz aus SEQ ID Nr. 8;
einer cDNA mit einer Nucleotidsequenz aus SEQ ID Nr. 10;
einer cDNA mit einer Nucleotidsequenz aus SEQ ID Nr. 12;
einer cDNA mit einer Nucleotidsequenz aus SEQ ID Nr. 14;
einer cDNA mit einer Nucleotidsequenz aus SEQ ID Nr. 83;
einer cDNA mit einer Nucleotidsequenz aus SEQ ID Nr. 84;
einer cDNA mit einer Nucleotidsequenz aus SEQ ID Nr. 85;
einer cDNA mit einer Nucleotidsequenz aus SEQ ID Nr. 86;
einer cDNA mit einer Nucleotidsequenz aus SEQ ID Nr. 87;
einer cDNA mit einer Nucleotidsequenz aus SEQ ID Nr. 88;
einer cDNA mit einer Nucleotidsequenz aus SEQ ID Nr. 89;
einer cDNA mit einer Nucleotidsequenz aus SEQ ID Nr. 90;
einer cDNA mit einer Nucleotidsequenz aus SEQ ID Nr. 91;
einer cDNA mit einer Nucleotidsequenz aus SEQ ID Nr. 92;
einer cDNA mit einer Nucleotidsequenz aus SEQ ID Nr. 93;
einer cDNA mit einer Nucleotidsequenz aus SEQ ID Nr. 94;
einer cDNA mit einer Nucleotidsequenz aus SEQ ID Nr. 95;
einer cDNA mit einer Nucleotidsequenz aus SEQ ID Nr. 96;
einer cDNA mit einer Nucleotidsequenz aus SEQ ID Nr. 97;
einer cDNA mit einer Nucleotidsequenz aus SEQ ID Nr. 98;
einer cDNA mit einer Nucleotidsequenz aus SEQ ID Nr. 99;
einer cDNA mit einer Nucleotidsequenz aus SEQ ID Nr. 100;
einer cDNA mit einer Nucleotidsequenz aus SEQ ID Nr. 101;
einer cDNA mit einer Nucleotidsequenz aus SEQ ID Nr. 102; und
einer cDNA mit einer Nucleotidsequenz aus SEQ ID Nr. 103.

20. Protein, das durch eine cDNA kodiert ist, die aus den in Anspruch 19 genannten ausgewählt ist.

21. OCIF-Variante, Mutante oder verkürzte cDNA, ausgewählt aus:
cDNA, die eine Aminosäuresequenz aus SEQ ID Nr. 9 kodiert;
cDNA, die eine Aminosäuresequenz aus SEQ ID Nr. 11 kodiert;
cDNA, die eine Aminosäuresequenz aus SEQ ID Nr. 13 kodiert;
cDNA, die eine Aminosäuresequenz aus SEQ ID Nr. 15 kodiert;
cDNA, die eine Aminosäuresequenz aus SEQ ID Nr. 62 kodiert;
cDNA, die eine Aminosäuresequenz aus SEQ ID Nr. 63 kodiert;
cDNA, die eine Aminosäuresequenz aus SEQ ID Nr. 64 kodiert;
cDNA, die eine Aminosäuresequenz aus SEQ ID Nr. 65 kodiert;
cDNA, die eine Aminosäuresequenz aus SEQ ID Nr. 66 kodiert;
cDNA, die eine Aminosäuresequenz aus SEQ ID Nr. 67 kodiert;
cDNA, die eine Aminosäuresequenz aus SEQ ID Nr. 68 kodiert;
cDNA, die eine Aminosäuresequenz aus SEQ ID Nr. 69 kodiert;
cDNA, die eine Aminosäuresequenz aus SEQ ID Nr. 70 kodiert;
cDNA, die eine Aminosäuresequenz aus SEQ ID Nr. 71 kodiert;
cDNA, die eine Aminosäuresequenz aus SEQ ID Nr. 72 kodiert;
cDNA, die eine Aminosäuresequenz aus SEQ ID Nr. 73 kodiert;
cDNA, die eine Aminosäuresequenz aus SEQ ID Nr. 74 kodiert;
cDNA, die eine Aminosäuresequenz aus SEQ ID Nr. 75 kodiert;
cDNA, die eine Aminosäuresequenz aus SEQ ID Nr. 76 kodiert;
cDNA, die eine Aminosäuresequenz aus SEQ ID Nr. 77 kodiert;
cDNA, die eine Aminosäuresequenz aus SEQ ID Nr. 78 kodiert;
cDNA, die eine Aminosäuresequenz aus SEQ ID Nr. 79 kodiert;
cDNA, die eine Aminosäuresequenz aus SEQ ID Nr. 80 kodiert;
cDNA, die eine Aminosäuresequenz aus SEQ ID Nr. 81 kodiert; und
cDNA, die eine Aminosäuresequenz aus SEQ ID Nr. 82 kodiert.

22. Genomische OCIF DNA, die eine Aminosäuresequenz aus SEQ ID Nr. 5 kodiert.

23. Genomische DNAs nach Anspruch 22 mit der Nucleotidsequenz aus SEQ ID Nr. 104 oder 105.

24. OCIF-Variante, Mutante oder verkürztes Protein, ausgewählt aus:
einem Protein mit einer Aminosäuresequenz der Aminosäurezahlen 1 bis 373 der SEQ ID Nr. 9;
einem Protein mit einer Aminosäuresequenz der Aminosäurezahlen 1 bis 341 der SEQ ID Nr. 11;
einem Protein mit einer Aminosäuresequenz der Aminosäurezahlen 1 bis 133 der SEQ ID Nr. 13;
einem Protein mit einer Aminosäuresequenz der Aminosäurezahlen 1 bis 124 der SEQ ID Nr. 15;
einem Protein mit einer Aminosäuresequenz der Aminosäurezahlen 1 bis 380 der SEQ ID Nr. 62;
einem Protein mit einer Aminosäuresequenz der Aminosäurezahlen 1 bis 380 der SEQ ID Nr. 63;
einem Protein mit einer Aminosäuresequenz der Aminosäurezahlen 1 bis 380 der SEQ ID Nr. 64;
einem Protein mit einer Aminosäuresequenz der Aminosäurezahlen 1 bis 380 der SEQ ID Nr. 65;
einem Protein mit einer Aminosäuresequenz der Aminosäurezahlen 1 bis 380 der SEQ ID Nr. 66;
einem Protein mit einer Aminosäuresequenz der Aminosäurezahlen 1 bis 339 der SEQ ID Nr. 67;
einem Protein mit einer Aminosäuresequenz der Aminosäurezahlen 1 bis 338 der SEQ ID Nr. 68;
einem Protein mit einer Aminosäuresequenz der Aminosäurezahlen 1 bis 342 der SEQ ID Nr. 69;
einem Protein mit einer Aminosäuresequenz der Aminosäurezahlen 1 bis 338 der SEQ ID Nr. 70;
einem Protein mit einer Aminosäuresequenz der Aminosäurezahlen 1 bis 305 der SEQ ID Nr. 71;
einem Protein mit einer Aminosäuresequenz der Aminosäurezahlen 1 bis 306 der SEQ ID Nr. 72;
einem Protein mit einer Aminosäuresequenz der Aminosäurezahlen 1 bis 378 der SEQ ID Nr. 73;
einem Protein mit einer Aminosäuresequenz der Aminosäurezahlen 1 bis 330 der SEQ ID Nr. 74;
einem Protein mit einer Aminosäuresequenz der Aminosäurezahlen 1 bis 251 der SEQ ID Nr. 75;
einem Protein mit einer Aminosäuresequenz der Aminosäurezahlen 1 bis 176 der SEQ ID Nr. 76;
einem Protein mit einer Aminosäuresequenz der Aminosäurezahlen 1 bis 122 der SEQ ID Nr. 77;
einem Protein mit einer Aminosäuresequenz der Aminosäurezahlen 1 bis 85 der SEQ ID Nr. 78;
einem Protein mit einer Aminosäuresequenz der Aminosäurezahlen 1 bis 372 der SEQ ID Nr. 79;
einem Protein mit einer Aminosäuresequenz der Aminosäurezahlen 1 bis 300 der SEQ ID Nr. 80;
einem Protein mit einer Aminosäuresequenz der Aminosäurezahlen 1 bis 166 der SEQ ID Nr. 81; und
einem Protein mit einer Aminosäuresequenz der Aminosäurezahlen 1 bis 63 der SEQ ID Nr. 82.

25. OCIF DNA, die ein Protein nach Anspruch 24 kodiert.

26. Pharmazeutische Zusammensetzung, umfassend ein Protein nach einem der Ansprüche 1-3, 6, 12, 13, 15, 18, 20 und 24.

27. Antikörper mit spezifischer Aktivität für ein Protein nach einem der Ansprüche 1-3, 6, 12-13, 15, 18, 20 und 24.

28. Antikörper nach Anspruch 27, der ein polyklonaler Antikörper ist.

29. Antikörper nach Anspruch 27, der ein monoklonaler Antikörper ist.

30. Monoklonaler Antikörper nach Anspruch 29, **gekennzeichnet durch** die folgenden Eigenschaften:
relative Molekülmasse von etwa 150.000 und der Unterklasse IgG₁, IgG₂ₐ oder IgG_{2b}.

31. Verfahren zur Bestimmung der Konzentration des OCIF-Proteins unter Verwendung der Antikörper nach einem der Ansprüche 27 bis 30.

32. Verwendung eines Proteins nach einem der Ansprüche 1-3, 6, 12, 13, 15, 18, 20 und 24 zur Herstellung eines Medikamentes zur Behandlung oder Prävention von Osteoporose.

33. Verwendung eines Proteins nach einem der Ansprüche 1-3, 6, 12, 13, 15, 18, 20 und 24 zur Herstellung eines Medikamentes zur Behandlung oder Prävention von Knochenschwund.

34. Verwendung eines Proteins nach einem der Ansprüche 1-3, 6, 12, 13, 15, 18, 20 und 24 zur Herstellung eines Medikamentes zur Behandlung oder Prävention von abnormem Knochenmetabolismus.

35. Vektor, in den eine der folgenden cDNA (a) bis (d) eingesetzt ist:
(a) OCIF cDNA, die eine Aminosäuresequenz aus SEQ ID Nr. 4 kodiert;
(b) OCIF cDNA mit einer Nucleotidsequenz aus SEQ ID Nr. 6;
(c) OCIF cDNA, die eine Aminosäuresequenz aus SEQ ID Nr. 5 kodiert;
(d) OCIF cDNA, die in einen Vektor als 1,6 Kb Fragment eingesetzt ist, das vom transformierten Escherichia coli pBK/01F10 (FERM BP-5267) getragen wird und ein Protein mit einer biologischen Aktivität zur Hemmung von Osteoklastendifferenzierung und/oder Maturation kodiert.

36. Vektor nach Anspruch 35, wobei der genannte Vektor vom transformierten Escherichia coli pBK/01F10 (FERM BP-5267) getragen wird.

37. Wirtszelle, in die der vektor nach Anspruch 35 oder 36 eingeführt ist.

38. Verfahren zur Herstellung eines Proteins nach Anspruch 1, wobei das genannte Verfahren die Schritte (I) bis (II) umfasst:
(I) Kultivieren der Wirtszelle nach Anspruch 37;
(II) Wiedergewinnen des genannten Proteins aus der erhaltenen Kultur.

39. Protein nach Anspruch 1, wobei das genannte Protein mit dem Verfahren aus Anspruch 38 erhältlich ist.

40. Transformiertes Escherichia coli pBK/O1F10 (FERM BP-5267).

## Revendications

1. Protéine OCIF **caractérisée par** les propriétés suivantes :
a) un poids moléculaire à l'électrophorèse sur gel de polyacrylamide-SDS (SDS-PAGE) :
d'approximativement 60 kD dans des conditions réductrices;
d'approximativement 60 kD et 120 kD dans des conditions non réductrices;
b) une haute affinité pour une colonne échangeuse de cations et une colonne d'héparine;
c) une activité biologique d'inhibition de la différenciation et/ou de la maturation des ostéoclastes
son activité étant réduite en chauffant à 70°C pendant 10 minutes ou à 56°C pendant 30 minutes;
son activité étant perdue en chauffant à 90°C pendant 10 minutes;
d) des séquences internes d'acides aminés fournies par les SEQ ID NO 1, 2 et 3.

2. Protéine selon la revendication 1, ayant des séquences d'acides aminés N-terminales présentées dans la SEQ ID NO 7.

3. Protéine selon la revendication 1, produite dans des fibroblastes humains.

4. Méthode de production d'une protéine selon la revendication 1, 2 ou 3, par le procédé suivant :
cultiver des fibroblastes humains; et purifier la protéine par une combinaison de chromatographie sur colonne échangeuse d'ions, sur colonne d'affinité et sur colonne en phase inversée.

5. Méthode selon la revendication 4, en cultivant des fibroblastes humains sur des morceaux de céramique d'alumine.

6. Protéine OCIF avec une séquence d'acides aminés telle que présentée dans la SEQ ID NO 4.

7. ADNc de OCIF codant la séquence d'acides aminés telle que présentée dans la SEQ ID NO 4.

8. ADNc de OCIF codant la séquence d'acides aminés telle que présentée dans la SEQ ID NO 5.

9. ADNc de OCIF qui est inséré dans un vecteur comme un fragment de 1,6 kb porté par le pBK/01F10 (FERM BP-5267) transformé de Escherichia coli.

10. ADNc de OCIF avec une séquence de nucléotides telle que présentée dans la SEQ ID NO 6.

11. ADNc de OCIF qui s'hybride à l'ADNc tel que présenté dans la SEQ NO 6 dans des conditions stringentes.

12. Protéine OCIF exprimée de l'ADNc codant la séquence d'acides aminés telle que présentée dans la SEQ ID NO 4.

13. Protéine OCIF avec une activité biologique d'inhibition de la différenciation et/ou de la maturation des ostéoclastes, qui est obtenue comme un ADNc exprimé par un acide aminé se partageant 80% d'identité de séquence avec l'acide aminé tel que présenté dans la SEQ ID NO 4.

14. Méthode de production d'une protéine selon la revendication 1, par génie génétique en utilisant l'ADNc de l'une quelconque des revendications 7 à 11.

15. La protéine selon la revendication 1, ladite protéine pouvant être obtenue par la méthode de la revendication 14.

16. Méthode de production de la protéine selon la revendication 12, par génie génétique en utilisant des cellules mammaliennes en tant que cellules hôtes.

17. Méthode de production de la protéine selon la revendication 16, par génie génétique en utilisant des cellules 293/EBNA ou des cellules CHO en tant que cellules hôtes mammaliennes.

18. La protéine selon la revendication 6 ou 12, ladite protéine pouvant être obtenue par la méthode de la revendication 16 ou 17.

19. ADNc variant, mutant ou tronqué de OCIF, sélectionné parmi :
un ADNc avec une séquence de nucléotides telle que présentée dans la SEQ ID NO 8;
un ADNc avec une séquence de nucléotides telle que présentée dans la SEQ ID NO 10;
un ADNc avec une séquence de nucléotides telle que présentée dans la SEQ ID NO 12;
un ADNc avec une séquence de nucléotides telle que présentée dans la SEQ ID NO 14;
un ADNc avec une séquence de nucléotides telle que présentée dans la SEQ ID NO 83;
un ADNc avec une séquence de nucléotides telle que présentée dans la SEQ ID NO 84;
un ADNc avec une séquence de nucléotides telle que présentée dans la SEQ ID NO 85;
un ADNc avec une séquence de nucléotides telle que présentée dans la SEQ ID NO 86;
un ADNc avec une séquence de nucléotides telle que présentée dans la SEQ ID NO 87;
un ADNc avec une séquence de nucléotides telle que présentée dans la SEQ ID NO 88;
un ADNc avec une séquence de nucléotides telle que présentée dans la SEQ ID NO 89;
un ADNc avec une séquence de nucléotides telle que présentée dans la SEQ ID NO 90;
un ADNc avec une séquence de nucléotides telle que présentée dans la SEQ ID NO 91;
un ADNc avec une séquence de nucléotides telle que présentée dans la SEQ ID NO 92;
un ADNc avec une séquence de nucléotides telle que présentée dans la SEQ ID NO 93;
un ADNc avec une séquence de nucléotides telle que présentée dans la SEQ ID NO 94;
un ADNc avec une séquence de nucléotides telle que présentée dans la SEQ ID NO 95;
un ADNc avec une séquence de nucléotides telle que présentée dans la SEQ ID NO 96;
un ADNc avec une séquence de nucléotides telle que présentée dans la SEQ ID NO 97;
un ADNc avec une séquence de nucléotides telle que présentée dans la SEQ ID NO 98;
un ADNc avec une séquence de nucléotides telle que présentée dans la SEQ ID NO 99;
un ADNc avec une séquence de nucléotides telle que présentée dans la SEQ ID NO 100;
un ADNc avec une séquence de nucléotides telle que présentée dans la SEQ ID NO 101;
un ADNc avec une séquence de nucléotides telle que présentée dans la SEQ ID NO 102; et
un ADNc avec une séquence de nucléotides telle que présentée dans la SEQ ID NO 103.

20. Protéine codée par un ADNc sélectionné parmi ceux de la revendication 19.

21. ADNc variant, mutant ou tronqué de OCIF, sélectionné parmi :
un ADNc codant une séquence d'acides aminés telle que présentée dans la SEQ ID NO 9;
un ADNc codant une séquence d'acides aminés telle que présentée dans la SEQ ID NO 11;
un ADNc codant une séquence d'acides aminés telle que présentée dans la SEQ ID NO 13;
un ADNc codant une séquence d'acides aminés telle que présentée dans la SEQ ID NO 15;
un ADNc codant une séquence d'acides aminés telle que présentée dans la SEQ ID NO 62;
un ADNc codant une séquence d'acides aminés telle que présentée dans la SEQ ID NO 63;
un ADNc codant une séquence d'acides aminés telle que présentée dans la SEQ ID NO 64;
un ADNc codant une séquence d'acides aminés telle que présentée dans la SEQ ID NO 65;
un ADNc codant une séquence d'acides aminés telle que présentée dans la SEQ ID NO 66;
un ADNc codant une séquence d'acides aminés telle que présentée dans la SEQ ID NO 67;
un ADNc codant une séquence d'acides aminés telle que présentée dans la SEQ ID NO 68;
un ADNc codant une séquence d'acides aminés telle que présentée dans la SEQ ID NO 69;
un ADNc codant une séquence d'acides aminés telle que présentée dans la SEQ ID NO 70;
un ADNc codant une séquence d'acides aminés telle que présentée dans la SEQ ID NO 71;
un ADNc codant une séquence d'acides aminés telle que présentée dans la SEQ ID NO 72;
un ADNc codant une séquence d'acides aminés telle que présentée dans la SEQ ID NO 73;
un ADNc codant une séquence d'acides aminés telle que présentée dans la SEQ ID NO 74;
un ADNc codant une séquence d'acides aminés telle que présentée dans la SEQ ID NO 75;
un ADNc codant une séquence d'acides aminés telle que présentée dans la SEQ ID NO 76;
un ADNc codant une séquence d'acides aminés telle que présentée dans la SEQ ID NO 77;
un ADNc codant une séquence d'acides aminés telle que présentée dans la SEQ ID NO 78;
un ADNc codant une séquence d'acides aminés telle que présentée dans la SEQ ID NO 79;
un ADNc codant une séquence d'acides aminés telle que présentée dans la SEQ ID NO 80;
un ADNc codant une séquence d'acides aminés telle que présentée dans la SEQ ID NO 81; et
un ADNc codant une séquence d'acides aminés telle que présentée dans la SEQ ID NO 82.

22. ADN génomique de OCIF codant une séquence d'acides aminés telle que présentée dans la SEQ ID NO 5.

23. ADN génomiques selon la revendication 22 avec la séquence de nucléotides telle que présentée dans la SEQ ID NO 104 ou 105.

24. Protéine OCIF variante, mutante ou tronquée sélectionnée parmi :
une protéine qui a une séquence d'acides aminés des acides aminés numéros 1 à 373 de la SEQ ID NO. 9;
une protéine qui a une séquence d'acides aminés des acides aminés numéros 1 à 341 de la SEQ ID NO. 11;
une protéine qui a une séquence d'acides aminés des acides aminés numéros 1 à 133 de la SEQ ID NO. 13;
une protéine qui a une séquence d'acides aminés des acides aminés numéros 1 à 124 de la SEQ ID NO. 15;
une protéine qui a une séquence d'acides aminés des acides aminés numéros 1 à 380 de la SEQ ID NO. 62;
une protéine qui a une séquence d'acides aminés des acides aminés numéros 1 à 380 de la SEQ ID NO. 63;
une protéine qui a une séquence d'acides aminés des acides aminés numéros 1 à 380 de la SEQ ID NO. 64;
une protéine qui a une séquence d'acides aminés des acides aminés numéros 1 à 380 de la SEQ ID NO. 65;
une protéine qui a une séquence d'acides aminés des acides aminés numéros 1 à 380 de la SEQ ID NO. 66;
une protéine qui a une séquence d'acides aminés des acides aminés numéros 1 à 339 de la SEQ ID NO. 67;
une protéine qui a une séquence d'acides aminés des acides aminés numéros 1 à 338 de la SEQ ID NO. 68;
une protéine qui a une séquence d'acides aminés des acides aminés numéros 1 à 342 de la SEQ ID NO. 69;
une protéine qui a une séquence d'acides aminés des acides aminés numéros 1 à 338 de la SEQ ID NO. 70;
une protéine qui a une séquence d'acides aminés des acides aminés numéros 1 à 305 de la SEQ ID NO. 71;
une protéine qui a une séquence d'acides aminés des acides aminés numéros 1 à 306 de la SEQ ID NO. 72;
une protéine qui a une séquence d'acides aminés des acides aminés numéros 1 à 378 de la SEQ ID NO. 73;
une protéine qui a une séquence d'acides aminés des acides aminés numéros 1 à 330 de la SEQ ID NO. 74;
une protéine qui a une séquence d'acides aminés des acides aminés numéros 1 à 251 de la SEQ ID NO. 75;
une protéine qui a une séquence d'acides aminés des acides aminés numéros 1 à 176 de la SEQ ID NO. 76;
une protéine qui a une séquence d'acides aminés des acides aminés numéros 1 à 122 de la SEQ ID NO. 77;
une protéine qui a une séquence d'acides aminés des acides aminés numéros 1 à 85 de la SEQ ID NO. 78;
une protéine qui a une séquence d'acides aminés des acides aminés numéros 1 à 372 de la SEQ ID NO. 79;
une protéine qui a une séquence d'acides aminés des acides aminés numéros 1 à 300 de la SEQ ID NO. 80;
une protéine qui a une séquence d'acides aminés des acides aminés numéros 1 à 166 de la SEQ ID NO. 81; et
une protéine qui a une séquence d'acides aminés des acides aminés numéros 1 à 63 de la SEQ ID NO. 82.

25. ADNc de OCIF codant une protéine selon la revendication 24.

26. Composition pharmaceutique comprenant une protéine selon l'une quelconque des revendications 1-3, 6, 12, 13, 15, 18, 20 et 24.

27. Anticorps ayant une activité spécifique contre une protéine selon l'une quelconque des revendications 1-3, 6, 12-13, 15, 18, 20 et 24.

28. Anticorps selon la revendication 27 qui est un anticorps polyclonal.

29. Anticorps selon la revendication 27 qui est un anticorps monoclonal.

30. Anticorps monoclonal selon la revendication 29, **caractérisé par** les propriétés suivantes :
poids moléculaire d'environ 150.000 et de sous-classe IgG₁, IgG₂ₐ ou IgG_{2b}.

31. Méthode de détermination de la concentration de la protéine du OCIF en utilisant les anticorps de l'une quelconque des revendications 27 à 30.

32. Utilisation d'une protéine selon l'une quelconque des revendications 1-3, 6, 12, 13, 15, 18, 20 et 24, dans la préparation d'un médicament pour le traitement ou la prévention de l'ostéoporose.

33. Utilisation d'une protéine selon l'une quelconque des revendications 1-3, 6, 12, 13, 15, 18, 20 et 24 dans la préparation d'un médicament pour le traitement ou la prévention d'une diminution de masse osseuse.

34. Utilisation d'une protéine selon l'une quelconque des revendications 1-3, 6, 12, 13, 15, 18, 20 et 24 dans la préparation d'un médicament pour le traitement ou la prévention d'un métabolisme osseux anormal.

35. Un vecteur dans lequel l'un quelconque des ADNc (a) à (d) suivants, est inséré :
(a) ADNc de OCIF codant une séquence d'acides aminés telle que présentée dans la SEQ ID NO.4;
(b) ADNc de OCIF codant une séquence d'acides aminés telle que présentée dans la SEQ ID NO.6;
(c) ADNc de OCIF codant une séquence d'acides aminés telle que présentée dans la SEQ ID NO.5;
(d) ADNc de OCIF qui est inséré dans un vecteur comme un fragment de 1,6 kb porté par le pBK/01F10 (FERM BP-5267) transformé de Escherichia coli, et code une protéine ayant une activité biologique d'inhibition de la différenciation et/ou de la maturation des ostéoclastes.

36. Le vecteur de la revendication 35, ledit vecteur est porté par le pBK/01F10 (FERM BP-5267) transformé de Escherichia coli.

37. Cellule hôte dans laquelle le vecteur selon les revendications 35 ou 36, est introduit.

38. Méthode de production d'une protéine selon la revendication 1, ladite méthode comprenant les étapes (I) et (II);
(I) cultiver la cellule hôte selon la revendication 37;
(II) récupérer ladite protéine de la culture obtenue.

39. Protéine selon la revendication 1, ladite protéine pouvant être obtenue par la méthode de la revendication 38.

40. Le pBK/01F10 (FERM BP-5267) transformé de Escherichia coli.
